# EUROPEAN PATENT APPLICATION

(11) **EP 4 527 851 A1**
(43) Date of publication of application: **26.03.2025**
(21) Application number: 23199018.5
(22) Date of filing: 22.09.2023
(51) Int. Cl.: C07K 16/28, C07K 16/46, A61K 39/395, A61P 35/00

(54) **BISPECIFIC ANTIBODIES BINDING LTBR AND LRRC15**

(71) Applicant: Bayer Aktiengesellschaft, 51373 Leverkusen (DE); Deutsches Krebsforschungszentrum, 69120 Heidelberg (DE)
(72) Inventor: Filarsky, Katharina, 82439 Großweil (DE); Weber, Ernst, 40764 Langefeld (DE); Kirchhoff, Dennis, 13086 Berlin (DE); Gritzan, Uwe, 50823 Köln (DE); Froehling, Christina, 57577 Hamm/Sieg (DE); McAleese Eser, Fionnuala Mary, 40764 Langenfeld (DE); Ellinger, Philipp, 42697 Solingen (DE); Heikenwälder, Mathias, 69120 Heidelberg (DE); Willuda, Jörg, 16548 Glienicke/Nordbahn (DE); Wengner, Antje Margret, 13086 Berlin (DE); Camps, Jordi, 13187 Berlin (DE)
(74) Representative: BIP Patents

(57) **Abstract**

What is described herein relates to an isolated tetravalent bispecific antibody comprising four antigen binding domains wherein a first and second antigen binding domain are binding to human LTβR and wherein a third antigen binding domain and a fourth antigen binding are binding to the same antigen other than LTβR wherein each of the first and second antigen binding domains are specific scFV fragments.

## Description

### Field of the disclosure

The present disclosure relates to bispecific antibodies (BsAB) binding human LTβR and human LRRC15. Also provided are BsABs which are agonists of LTPR signaling in the presence of LRRC15. Furthermore, described are pharmaceutical uses of BsABs which are agonists of LTβR signaling in the presence of LRRC15.

### Background

The hallmark of so called non-inflamed or "cold" tumours is a lack of immune response towards the tumor and especially a lack of immune cell infiltration, which is associated with a lower benefit from anti-cancer immunotherapies (Chen and Mellman, Nature 541:321-30 (2017)). One possible target for anti-cancer immunotherapy is the lymphotoxin beta receptor (LTBR/TNFRSF3), a receptor of the TNF superfamily. LTβR is activated by two different trimeric ligands, LIGHT (TNFSF14) and lymphotoxin α1b2 (LTa1β2). While LTa1β2 is specific for LTβF, LIGHT also binds to and activates HVEM (TNFRSF14), a receptor expressed on and implicated in the regulation of immune cells. Interaction of LTβR with LIGHT or LTα1β2 induces an ordered aggregation or "clustering"/"hyper-clustering" of neighbouring LTβR receptors initiating signal transduction pathways (Piao et al. Cells 2021, 10, 747 as well as Kucka and Wajant H (2021) Cell Dev. Biol. 8:615141 doi: 10.3389/fcell.2020.615141). It has been demonstrated that activation of LTβR by its ligands leads to ectopic formation of tertiary lymphoid structures (TLS) (Schrama et al., Immunity 14: 111-121 (2001)). Presence of TLS in the tumour microenvironment typically correlates with immune infiltration and is also associated with better prognosis, suggesting that TLS are involved in antitumor immune responses. Therefore, activation of LTPR has the potential to promote TLS formation in the tumour microenvironment and induce anti-tumour immune responses and improve current cancer immunotherapies. Due to the broad expression of LTPR in the organism, an agonistic LTβR targeting drug capable of inducing TLS and creating an activating immune environment bears a substantial risk of causing systemic immune-related adverse events. Johansson-Percival et al. reported weight loss in mice after systemic administration of an LTβR activating compound, VTP-LIGHT (Johansson-Percival et al., Nat. Immunol. 18:1207-17 (2017)). Moreover Gurney et al. reported *in vitro* and *in vivo* studies with a bispecific fusion construct consisting of a heterotrimeric single-chain LTa1β2 moiety fused to a B7-H4 specific tumor targeting antibody (WO 2018/119118). Hence in contrast to the construct of Johansson-Percival the bispecific antibody of WO 2018/119118 was a specific agonist of LTPR and did not activate HVEM. However, the models used by Gurney et al. were artificial models consisting of engineered cell lines overexpressing B7-H4. Activity in models with non-engineered B7-H4 expression levels that are more representative for B7-H4 levels in human tumors remained unclear. No observations on tolerability in mice were reported. Building on these findings Lorenzi et al. (WO 2021/116337) provided a bispecific antibody consisting of the LTβR mAb1 of WO2004002431 fused to an anti-fibronectin extra domain B (EDB) moiety. Lorenzi et al. (WO 2021/116337) reported that targeting of LTβR and a tumour-associated antigen (TAA) that is co-expressed with LTβR on tumour cells with a bispecific antibody that binds to both LTBR and such TAA- Mesothelin was used in the example - was not capable of activating LTβR in a tumour-specific manner. On the other hand, Lorenzi et al. (WO 2021/116337) published that bispecific antibodies that bind with one antigen binding domain to LTβR and with another antigen binding domain to EDB of Fibronectin (a TAA that is present in the extracellular matrix), were capable of activating LTβR in a tumorspecific manner. They reported particularly good results when such bispecific antibodies contained three antigen binding domains, e.g. two antigen binding domains targeting the EDB and one antigen binding domain targeting LTβR. Hence, the conclusion of Lorenzi et al. (WO 2021/116337) was that if a bispecific antibody is to be used to activate LTβR in a tumorspecific manner the tumour associated antigen has to be present as protein in the extracellular matrix of the tumor and not expressed on a cell. Furthermore WO 2023/117834 A1 discloses bispecific bivalent or trivalent antibodies specific for LTβR and fibroblast Activation Protein (FAP) and WO 2022/157094 discloses agonistic antibodies specific for LRRC15. For agonist antibodies Fc region are known in the art which elicit less signalling events that stimulate the receptor-bearing cell to perform effector functions upon binding to an Fc domain of an antibody or which binds Fc receptors (FcyR, FcR) with less affinity, thereby reducing antibody directed cytotoxicity (ADCC) effector function. Examples of these "silent IgG1 Fc domains" are Fc domains comprising the following mutations LALA (L234A and L235A and N297A) (Strohl, W, 2009, Curr. Opin. Biotechnol. Vol. 20 (6): 685- 691). E233P/L234V/L235A/DG236/D265G/A327Q/A330S (cf. EP2794658 A1), "aglycosylation" ( Bolt et al., Eur J Immunol. 1993 Feb;23(2):403-11) and "PG-LALA" (Schlothauer et al., Protein Eng Des Sel 2016 Oct;29(10):457-466).

### Summary

According to a first aspect, what is described herein relates to an isolated tetravalent bispecific antibody (BsAB) comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain is capable of binding to. BsABs according to what is described herein are characterized by their ability to stimulate the LTβR signaling pathway if used as targeted LTβR antibody, while not inducing relevant stimulation of the LTβR signaling pathway if used as non-targeted LTβR antibody. In some preferred embodiments of the first aspect the third and fourth antigen binding domains of the isolated tetravalent bispecific antibody described herein are capable of binding LRRC15. Preferably the LTβR is human LTβR or a fragment thereof, and/or the LRRC15 is human LRRC15 or a fragment thereof.

In a second aspect what is described herein relates to specific first antigen binding domains capable of binding to LTβR and specific second antigen binding domains capable of binding to LTβR. Further provided are tetravalent BsABs for use as a medicament as well as tetravalent BsABs for use in the treatment of cancer. Further provided are pharmaceutical compositions comprising both, a tetravalent BsAB as described herein, and a pharmaceutically acceptable vehicle.

### Brief description of the drawing

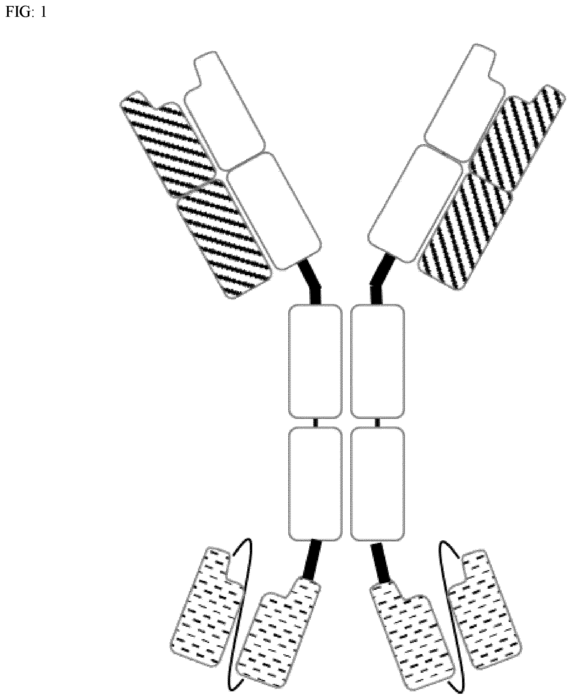

**Fig. 1** is a schematic representation of a preferred embodiment of an (isolated) tetravalent bispecific antibody described herein. In this example the (isolated) tetravalent bispecific antibody consists of an scFv part comprising a first and a second antigen binding domain *(dotted*) capable of binding to human LTβR and an IgG1 part comprising a third and fourth antigen binding domain capable of binding to an antigen other than LTβR, here human LRRC15 (white part plus hatched part). The two heavy chains of this exemplary (isolated) tetravalent bispecific antibody are represented by the white parts plus the dotted parts and the two light chain are represented by the hatched parts i.e. the two scFV fragments of this exemplary (isolated) tetravalent bispecific antibody were expressed as part of each heavy chain. This exemplary (isolated) tetravalent bispecific antibody selectively stimulated the LTβR signaling pathway in cell types expressing the third and a fourth antigen binding domain e.g. LLRC15. In other cell types not expressing the third and a fourth antigen binding domain the (isolated) tetravalent bispecific antibody was only a low level agonist of LTβR.

### Description of the sequence IDs

The Sequence Listing associated with this application is filed in electronic format via EFS-Web and hereby incorporated by reference into the specification in its entirety. The name of the text file containing the Sequence Listing is BHC221027_ST26.xml. The size of the text file is 163.921 bytes and the text file was created on September 15, 2023.

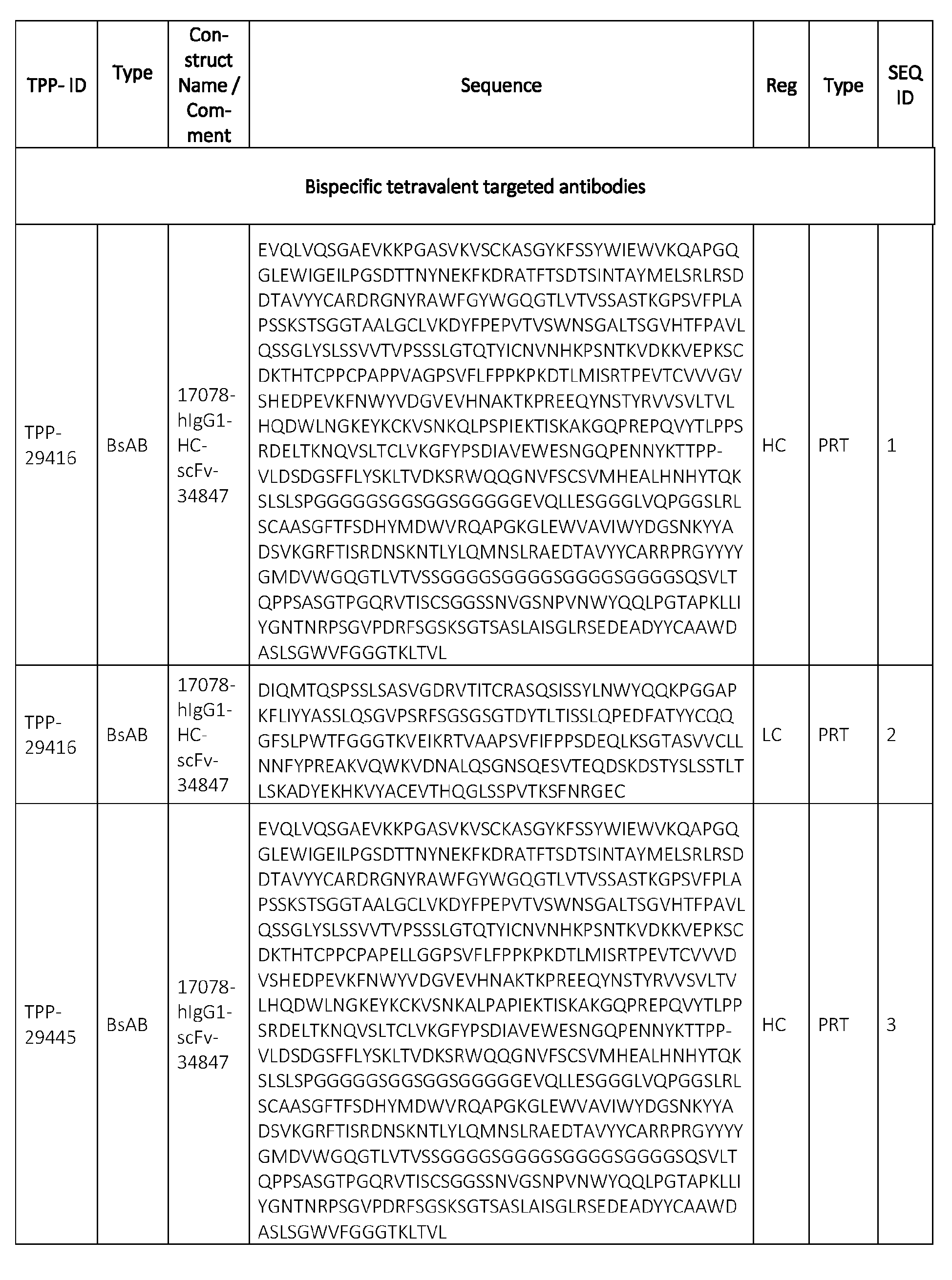

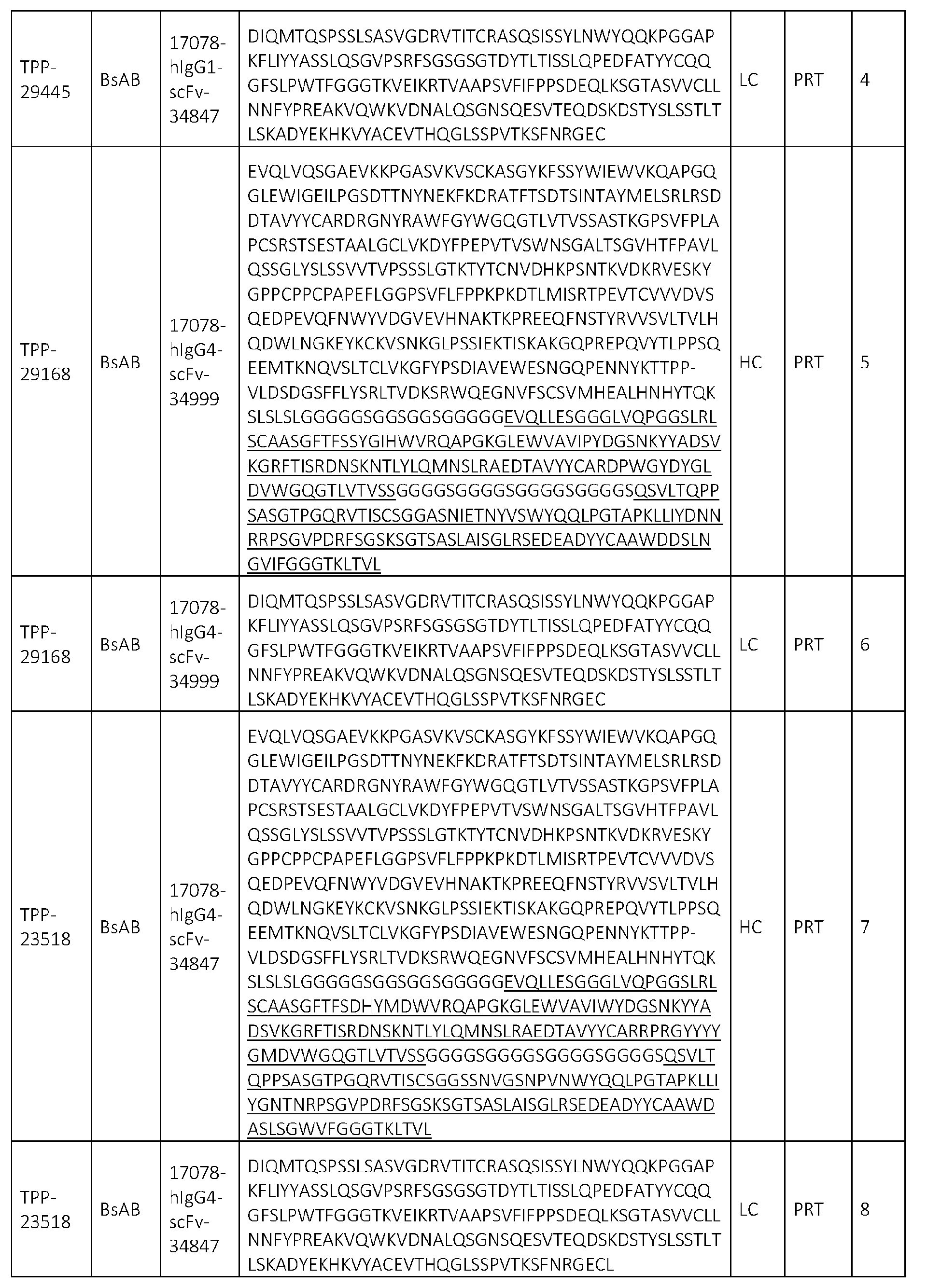

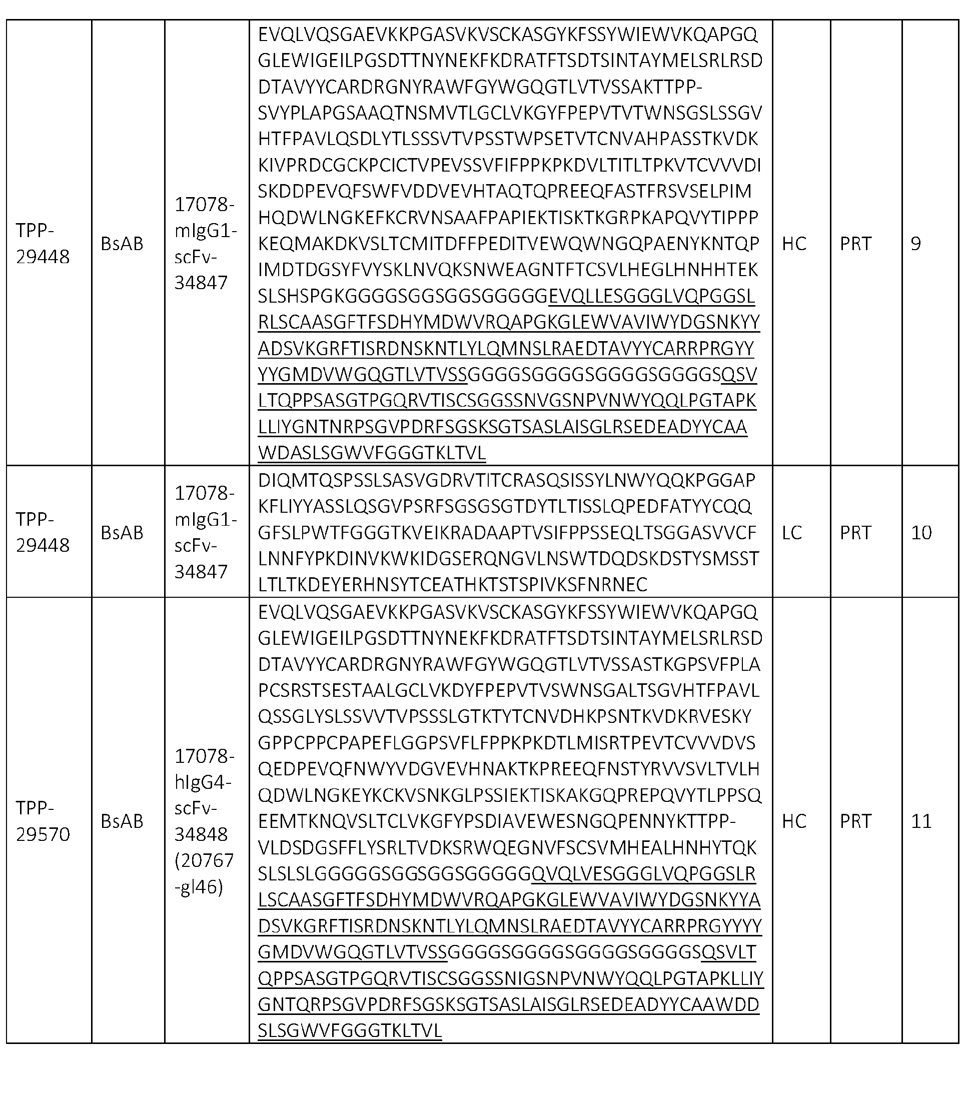

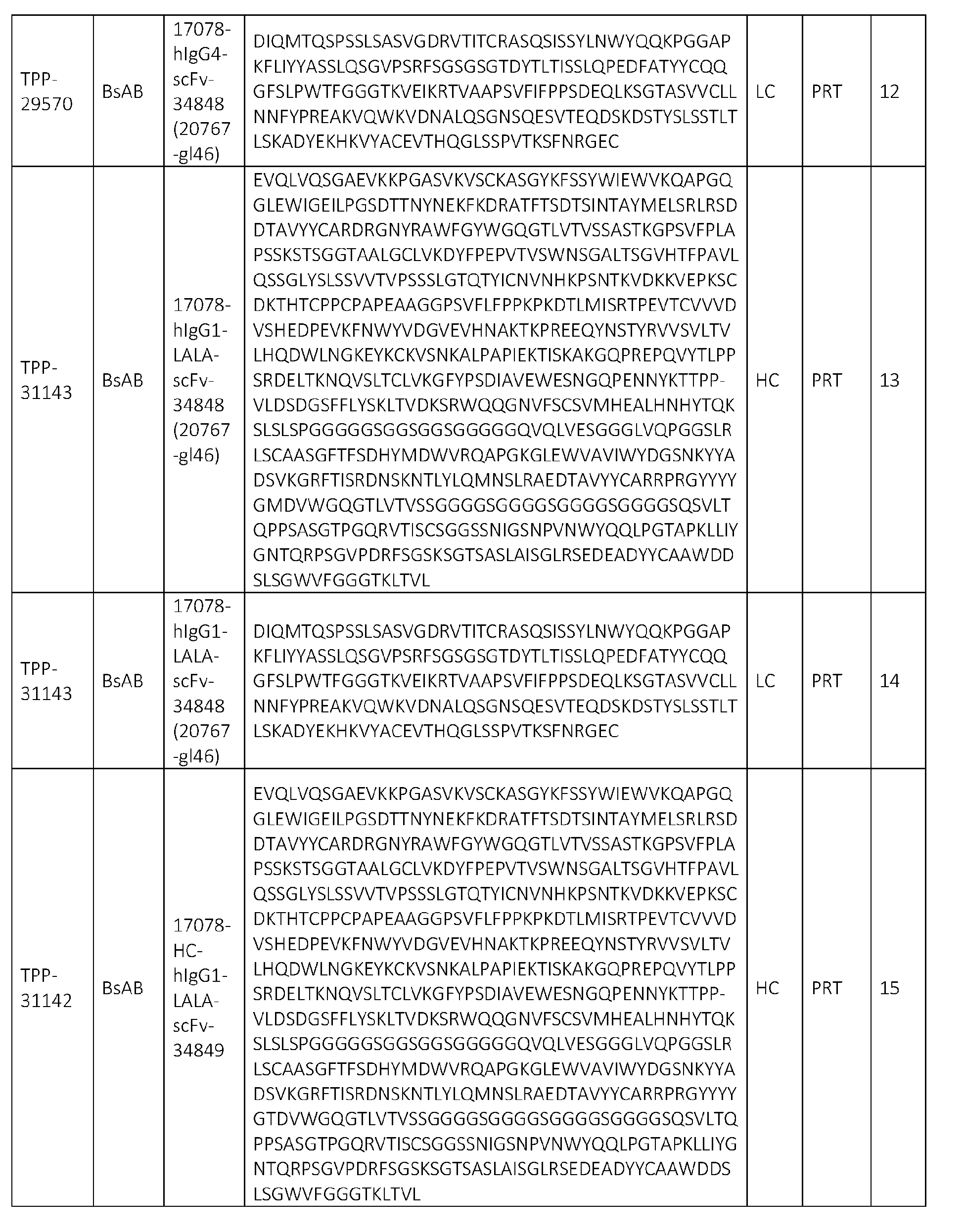

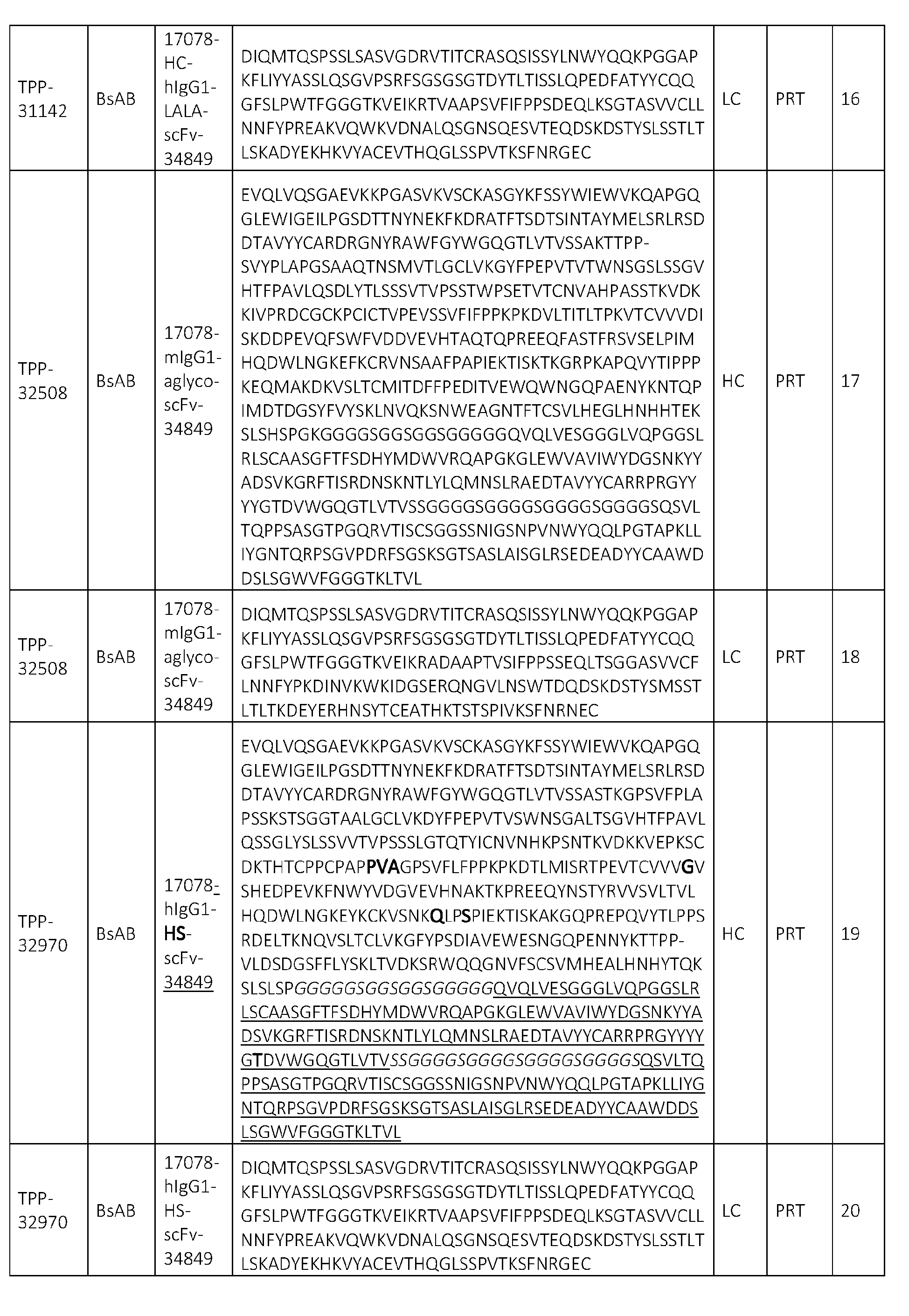

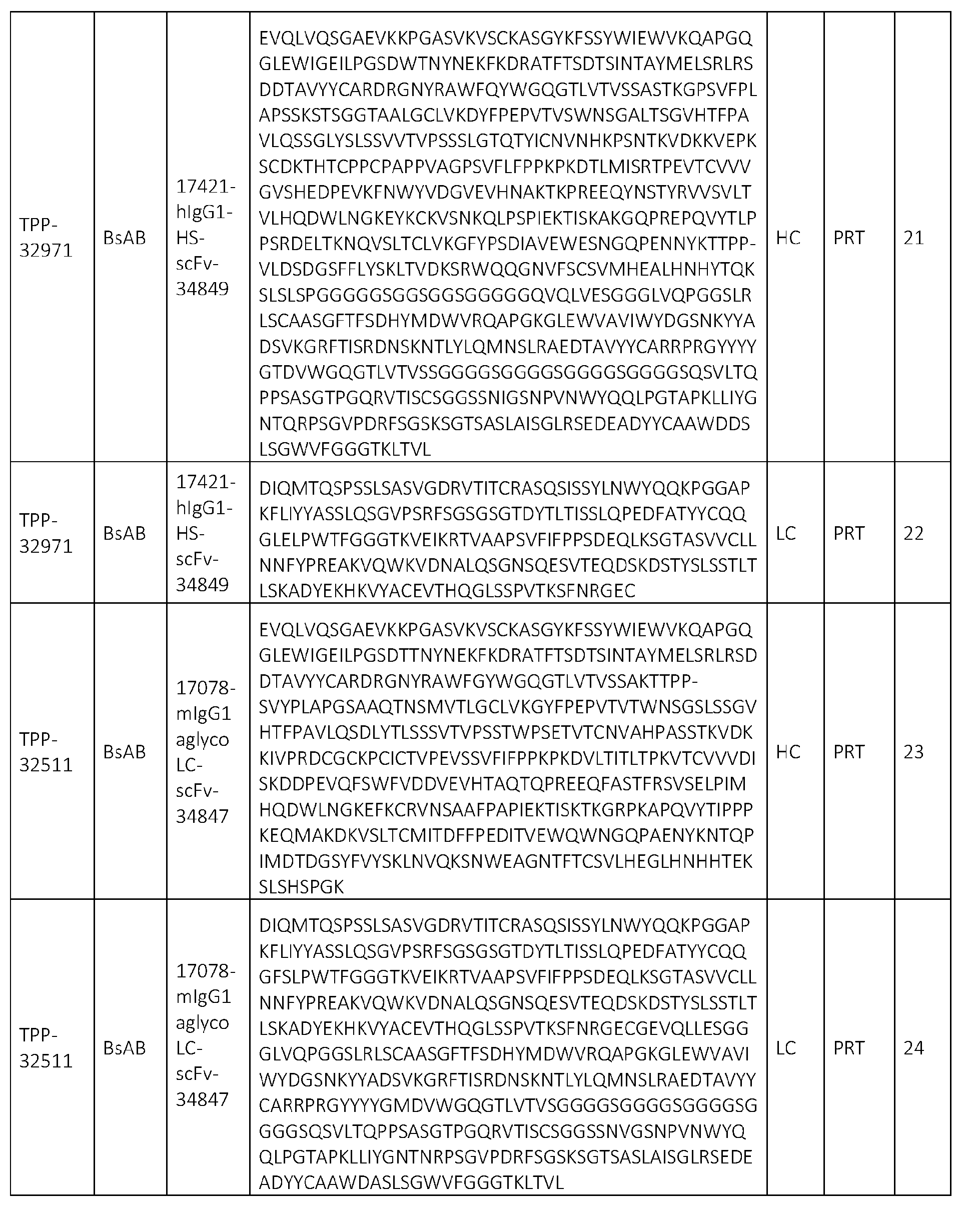

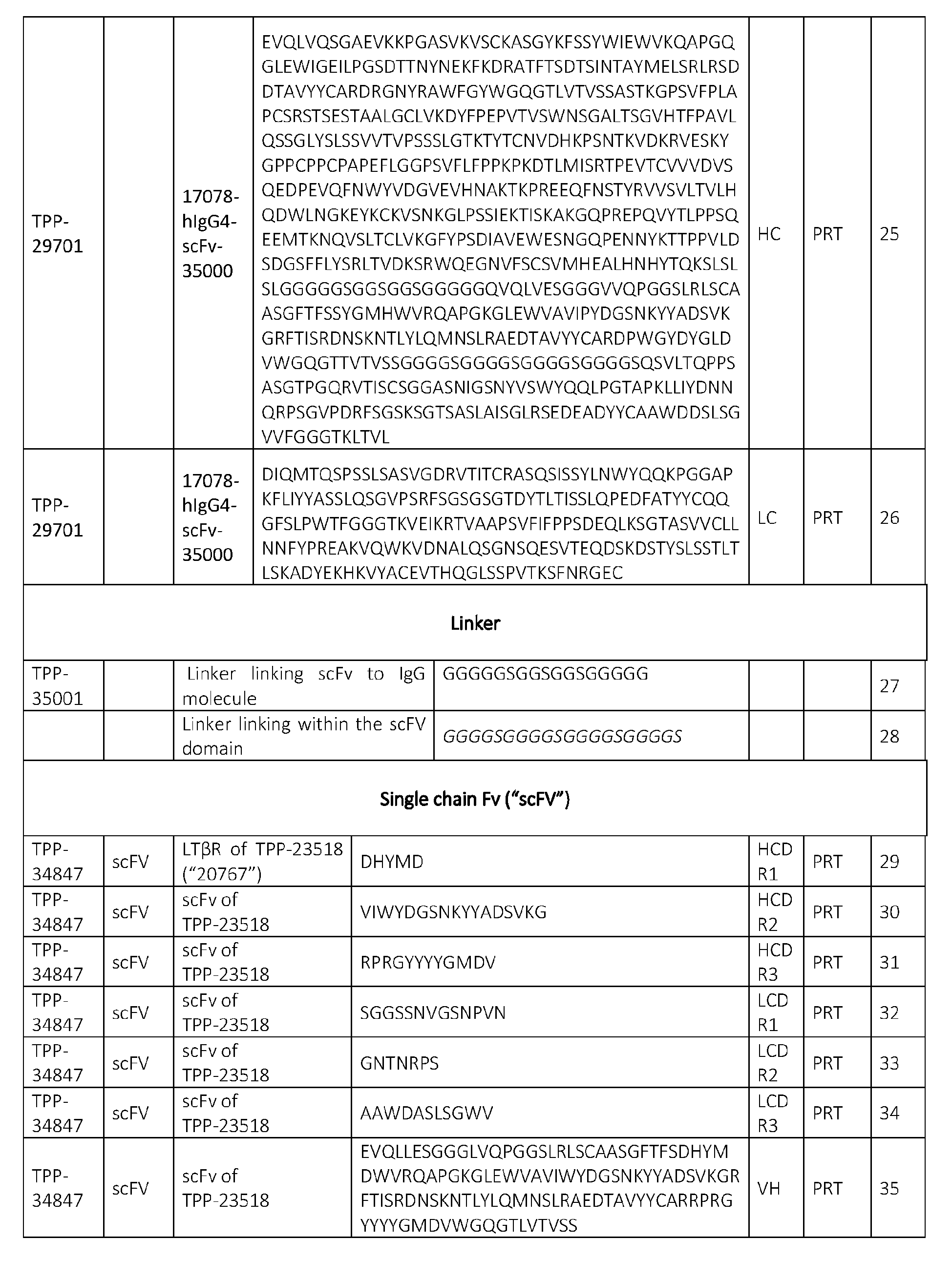

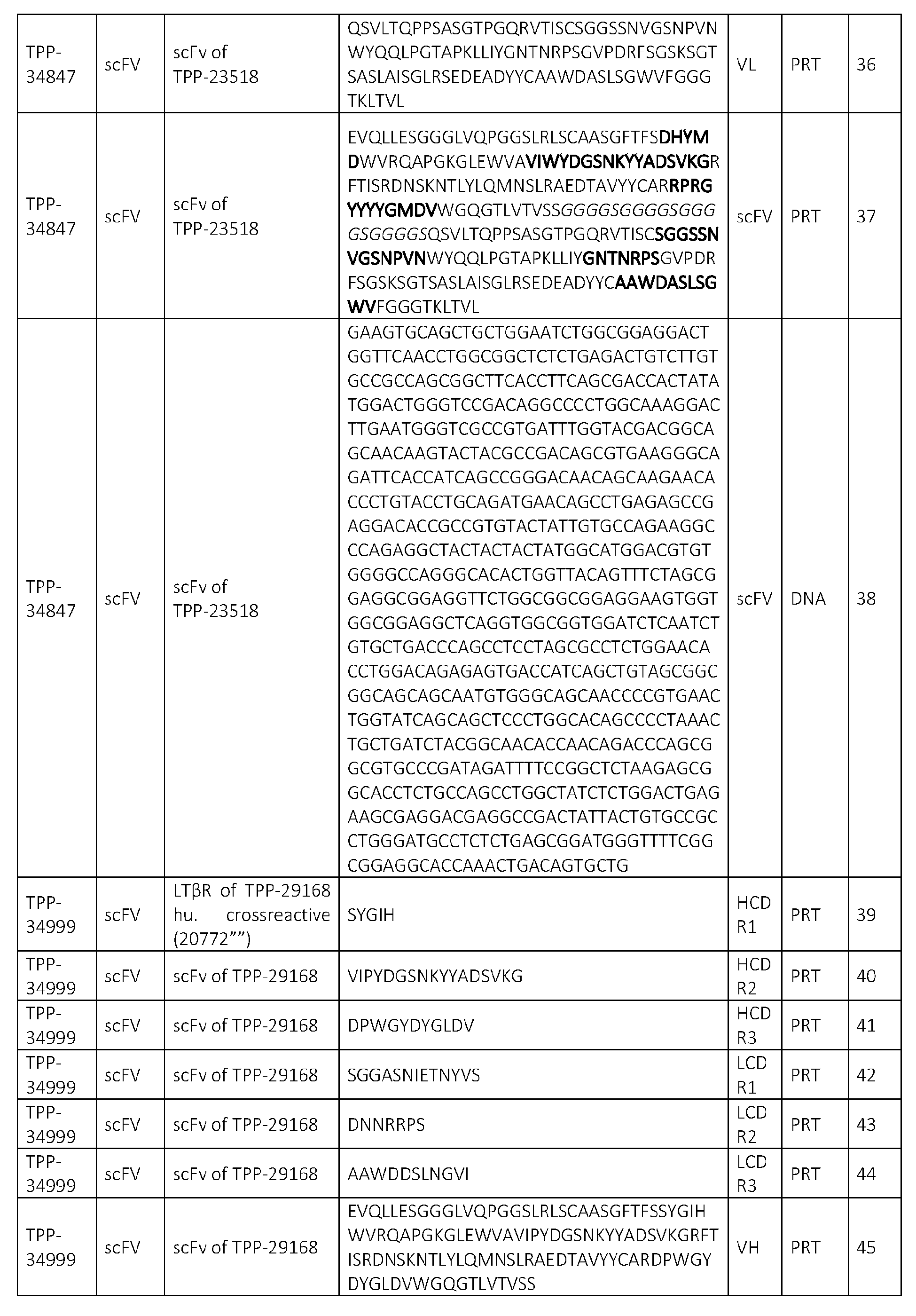

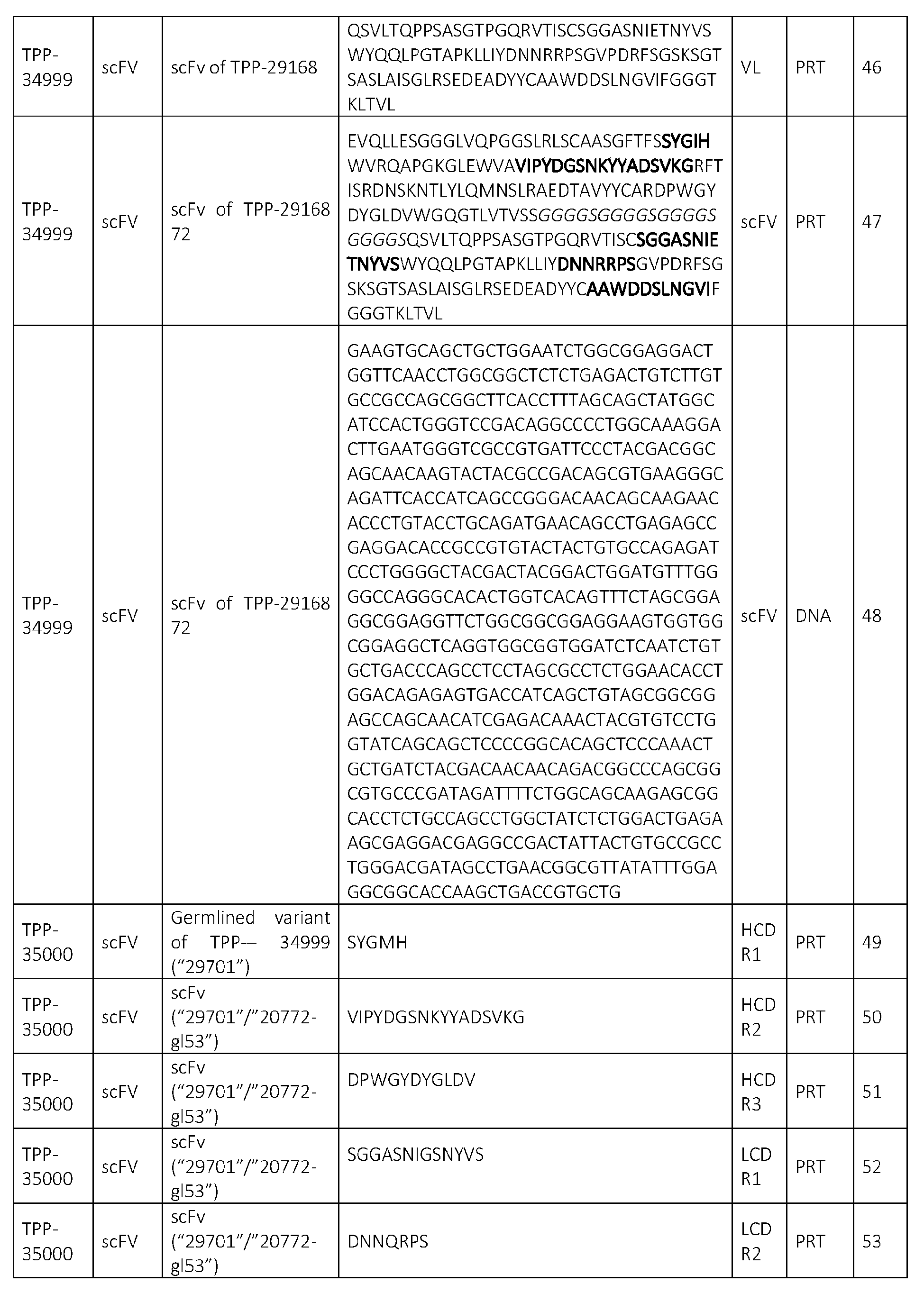

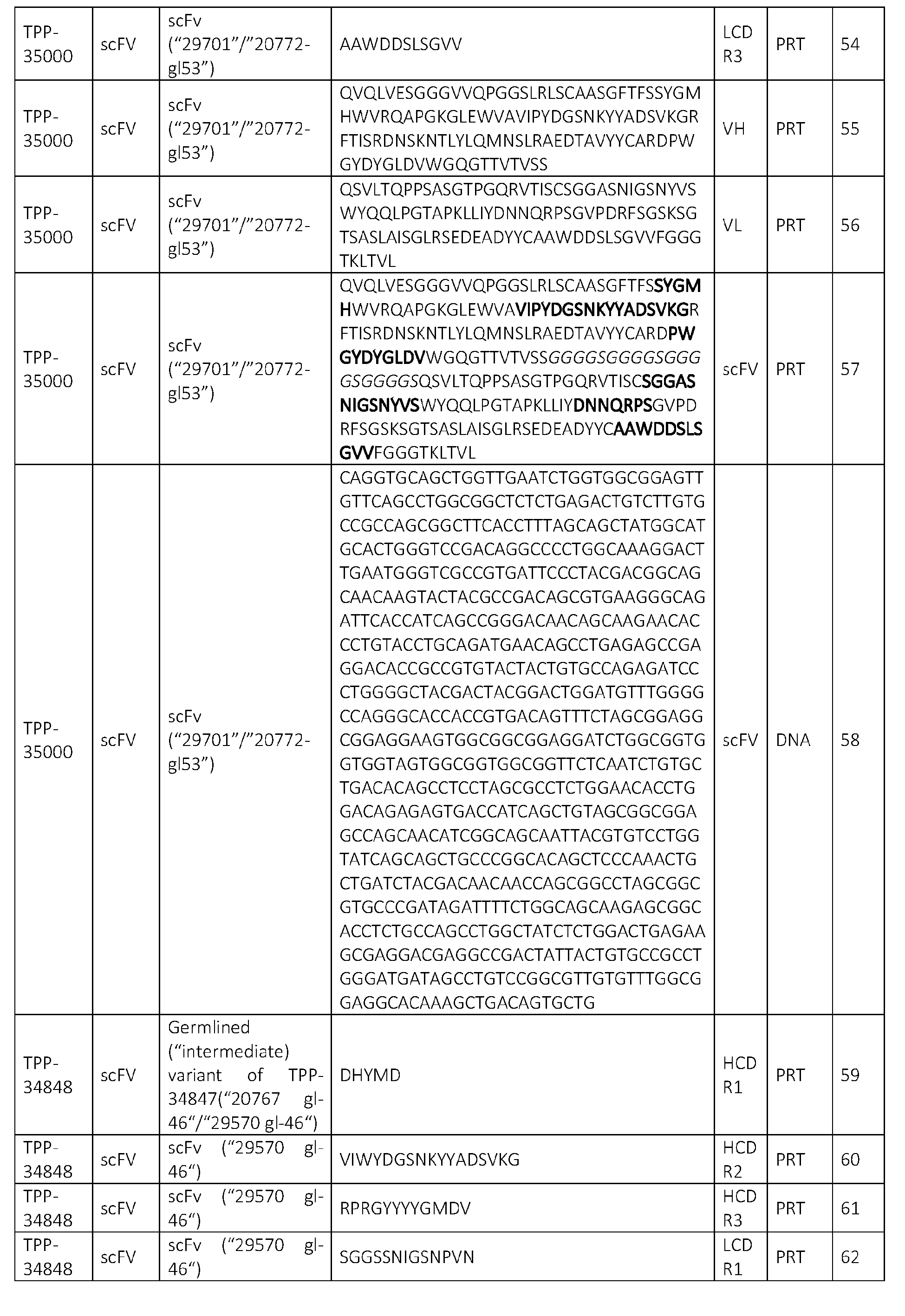

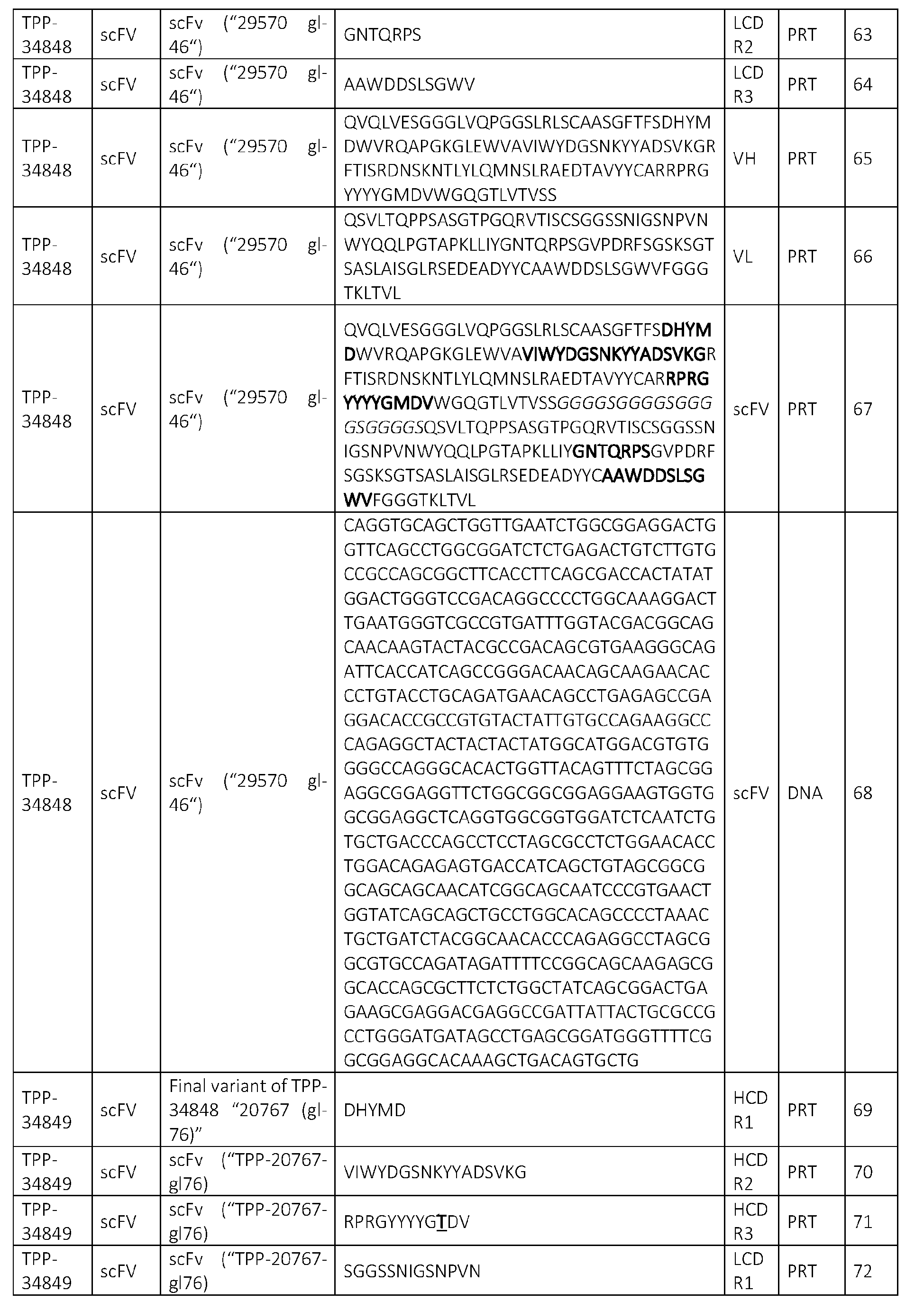

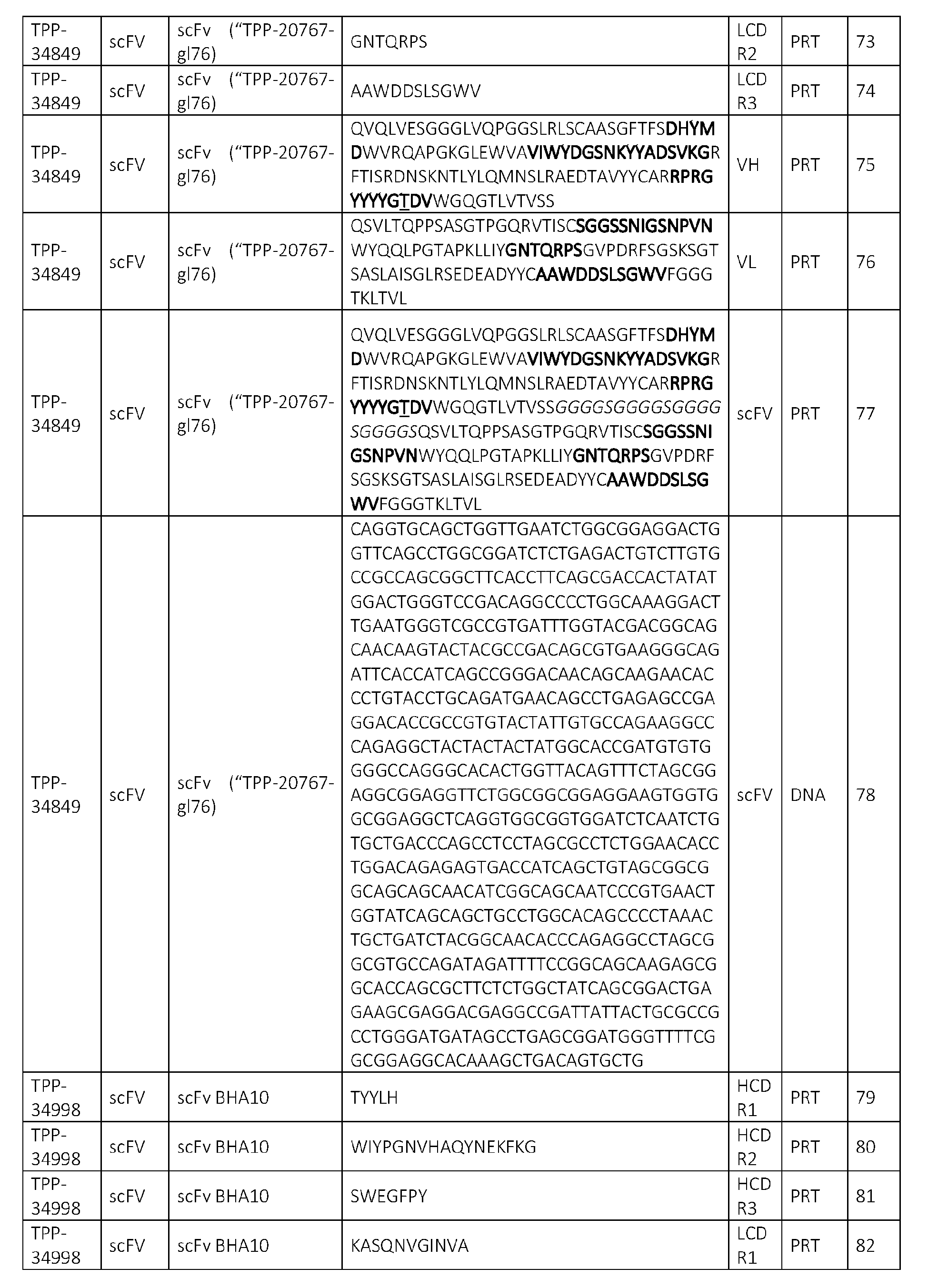

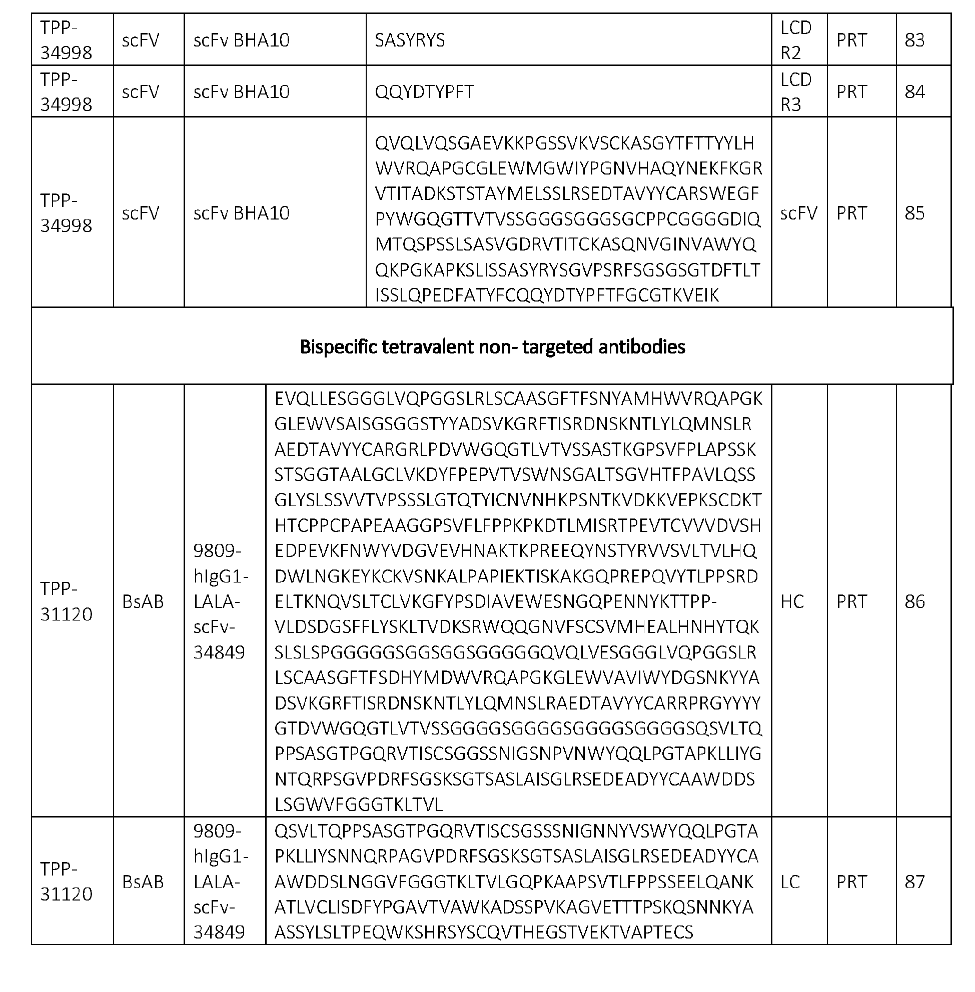

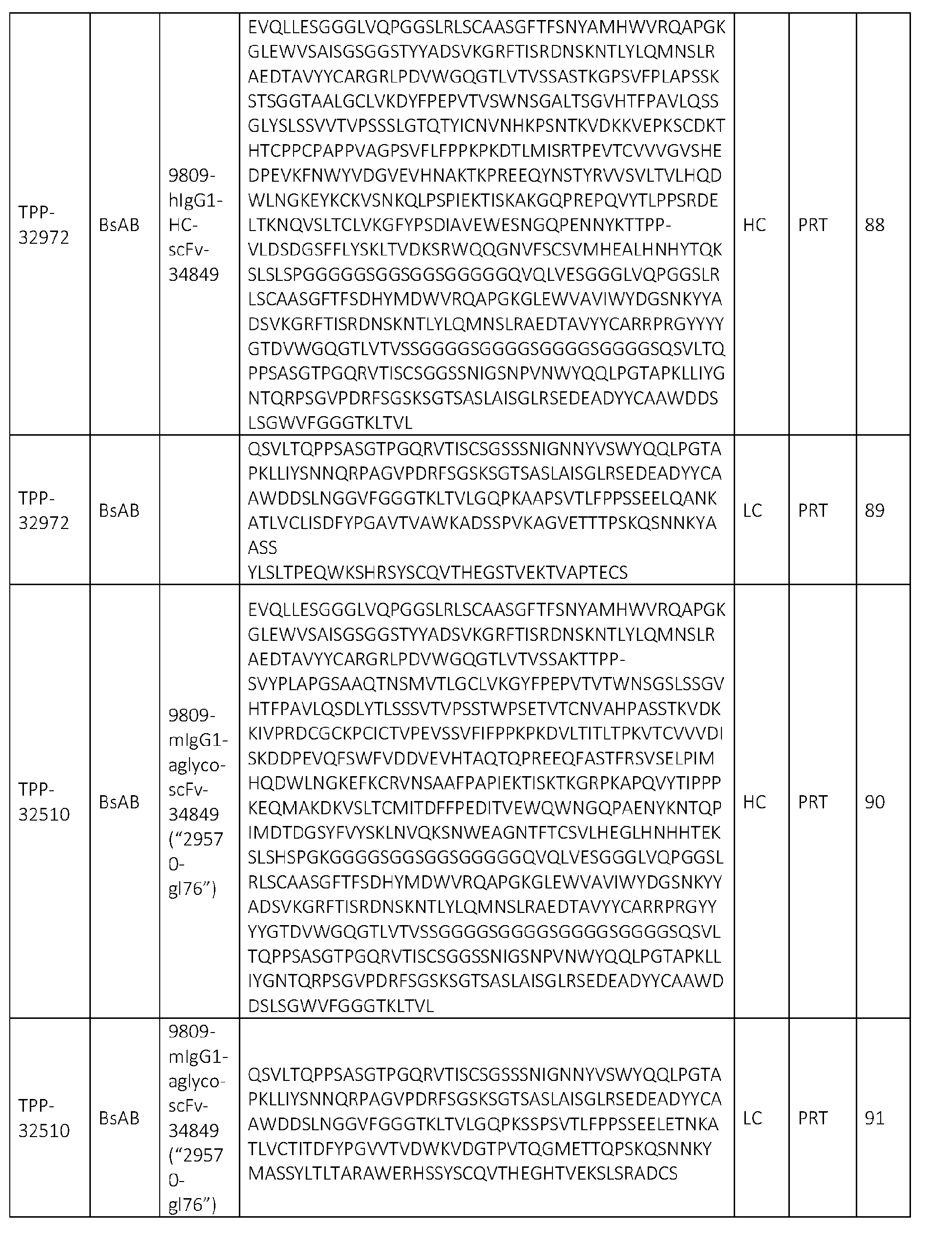

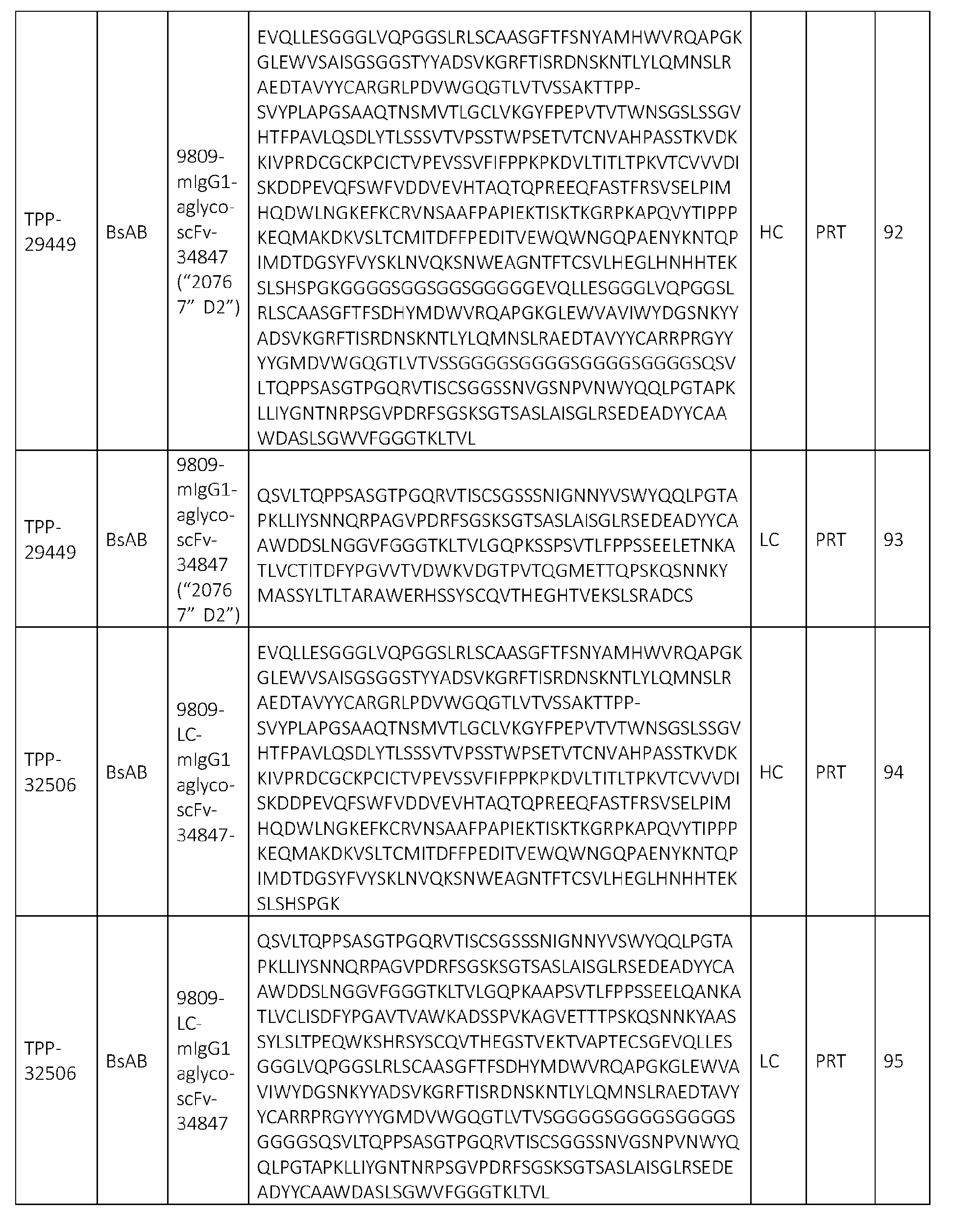

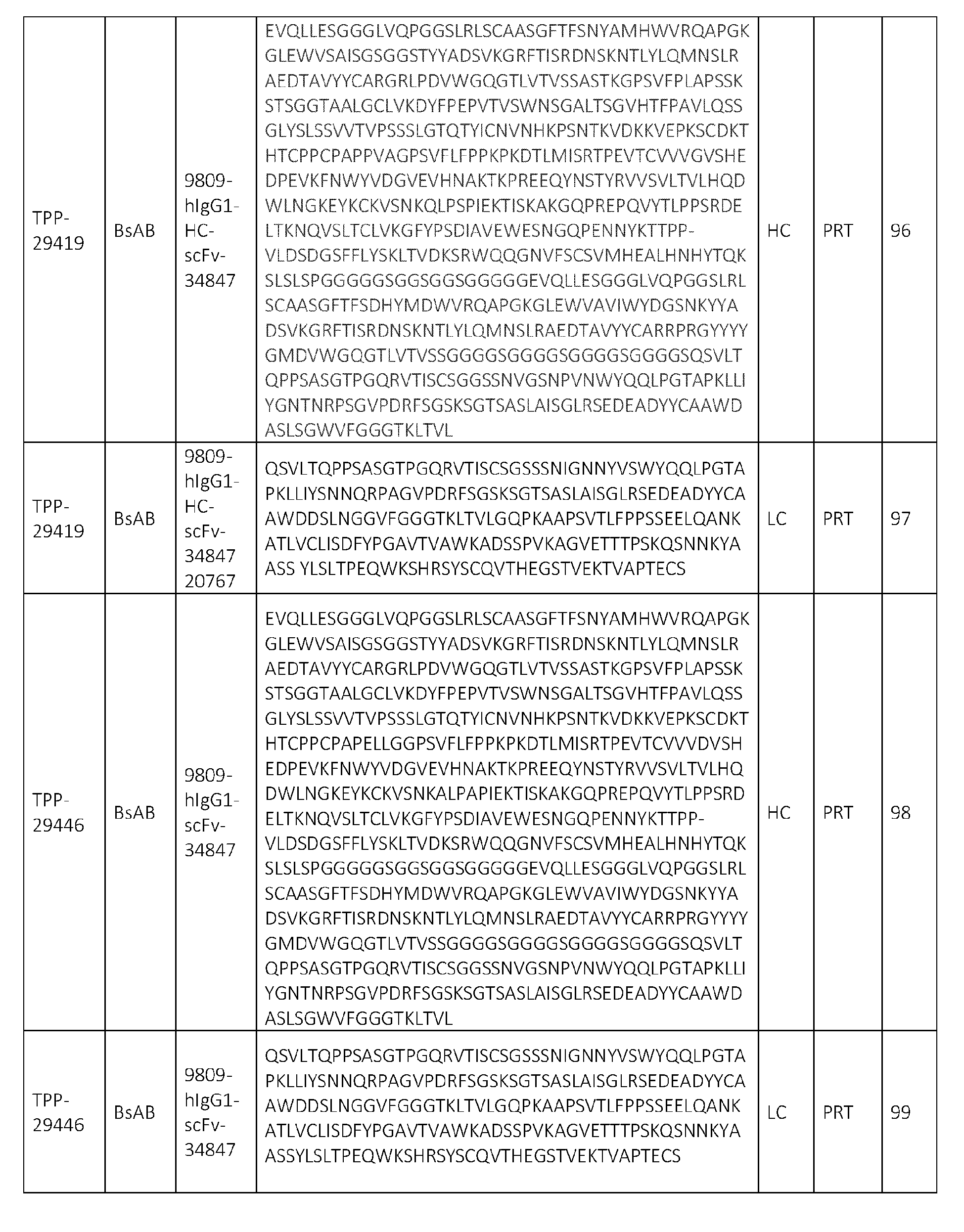

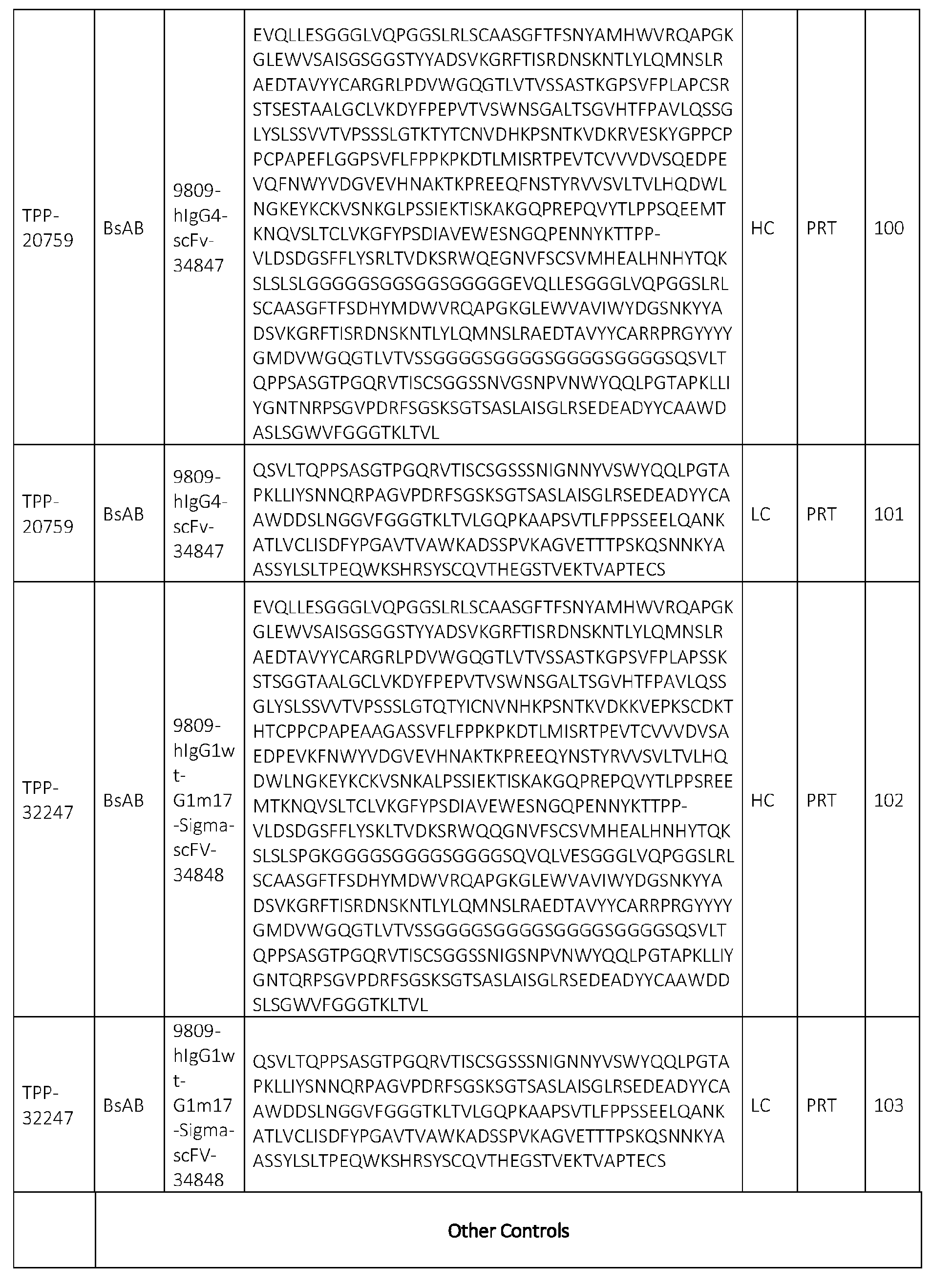

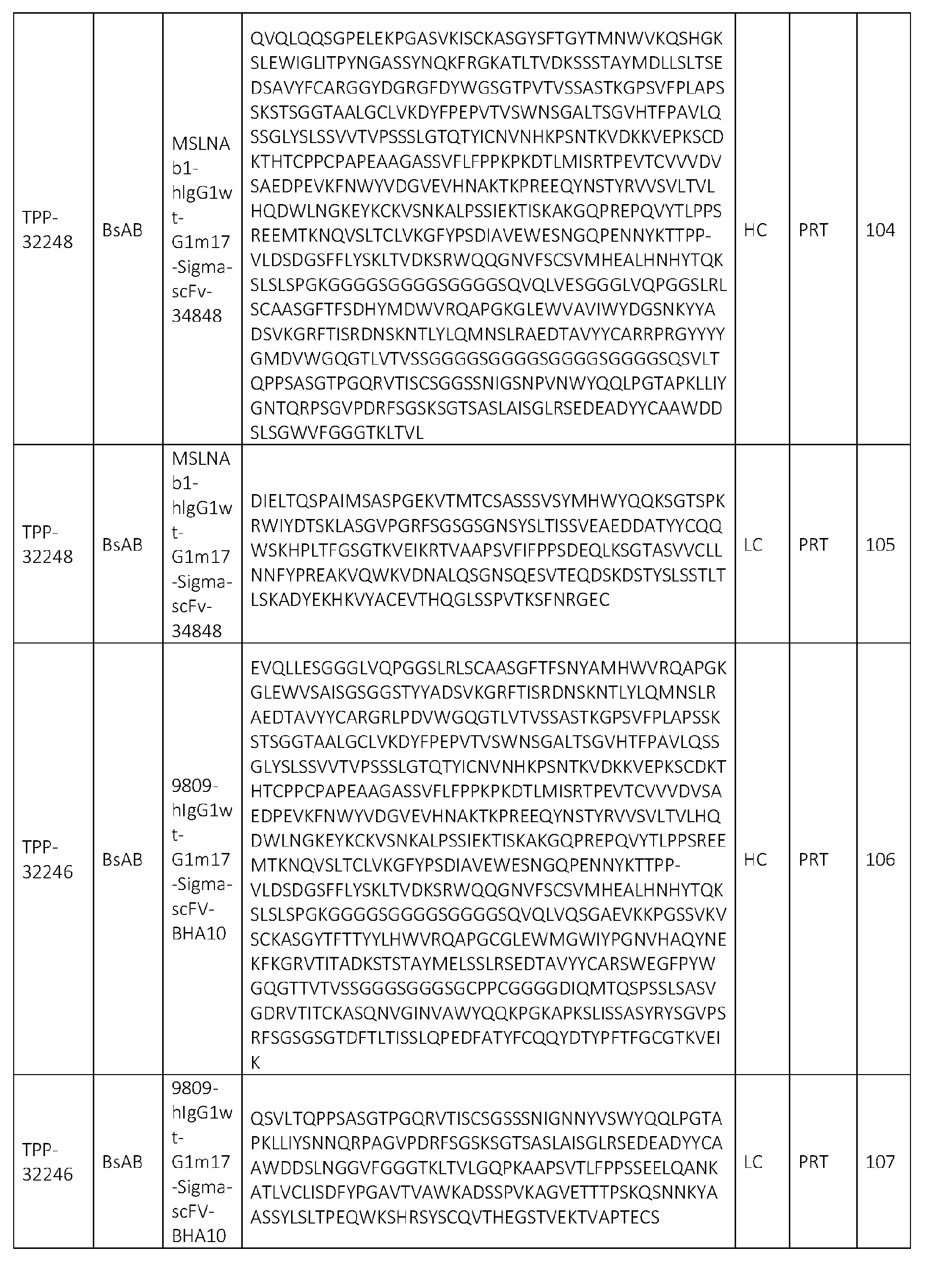

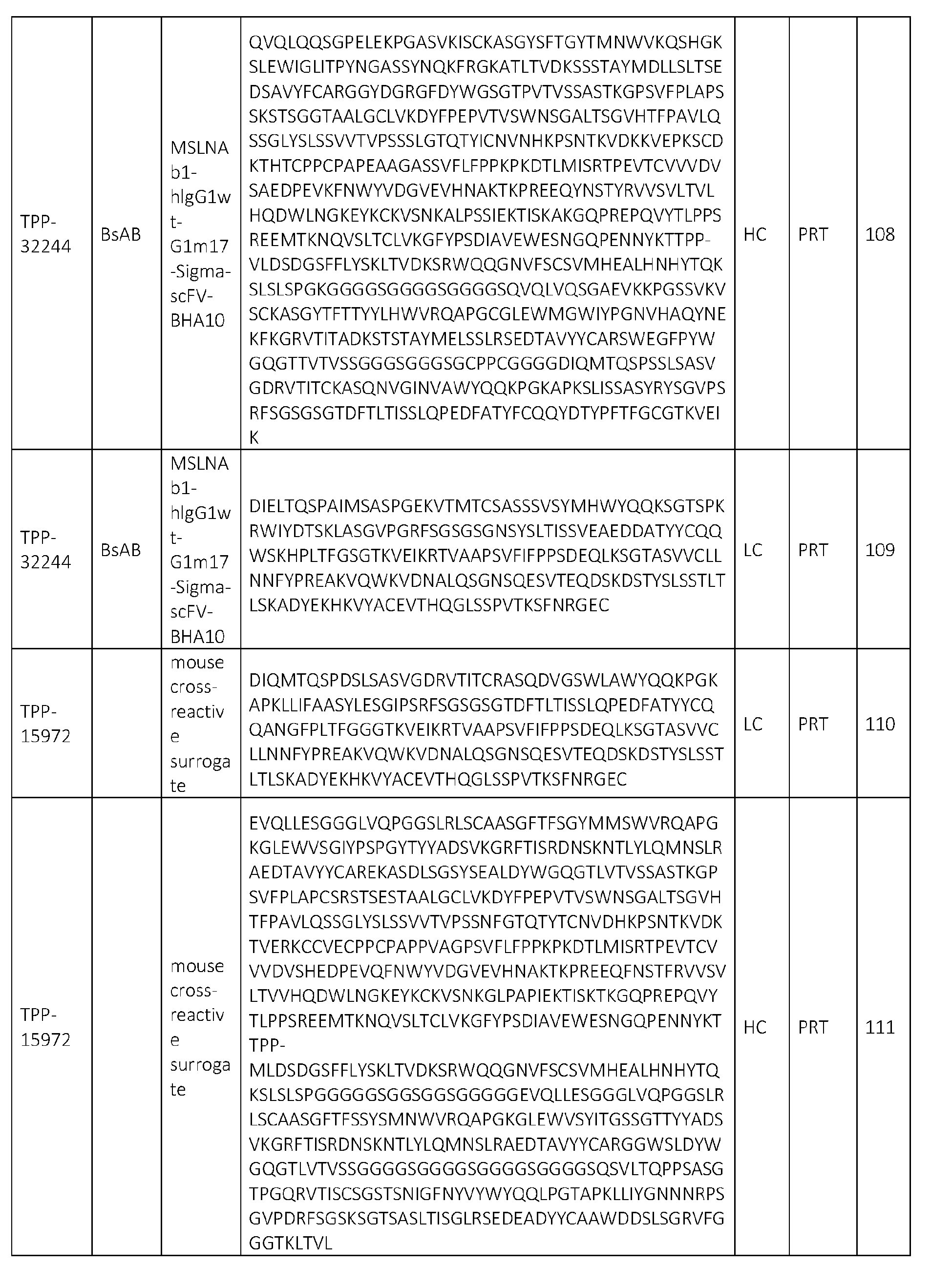

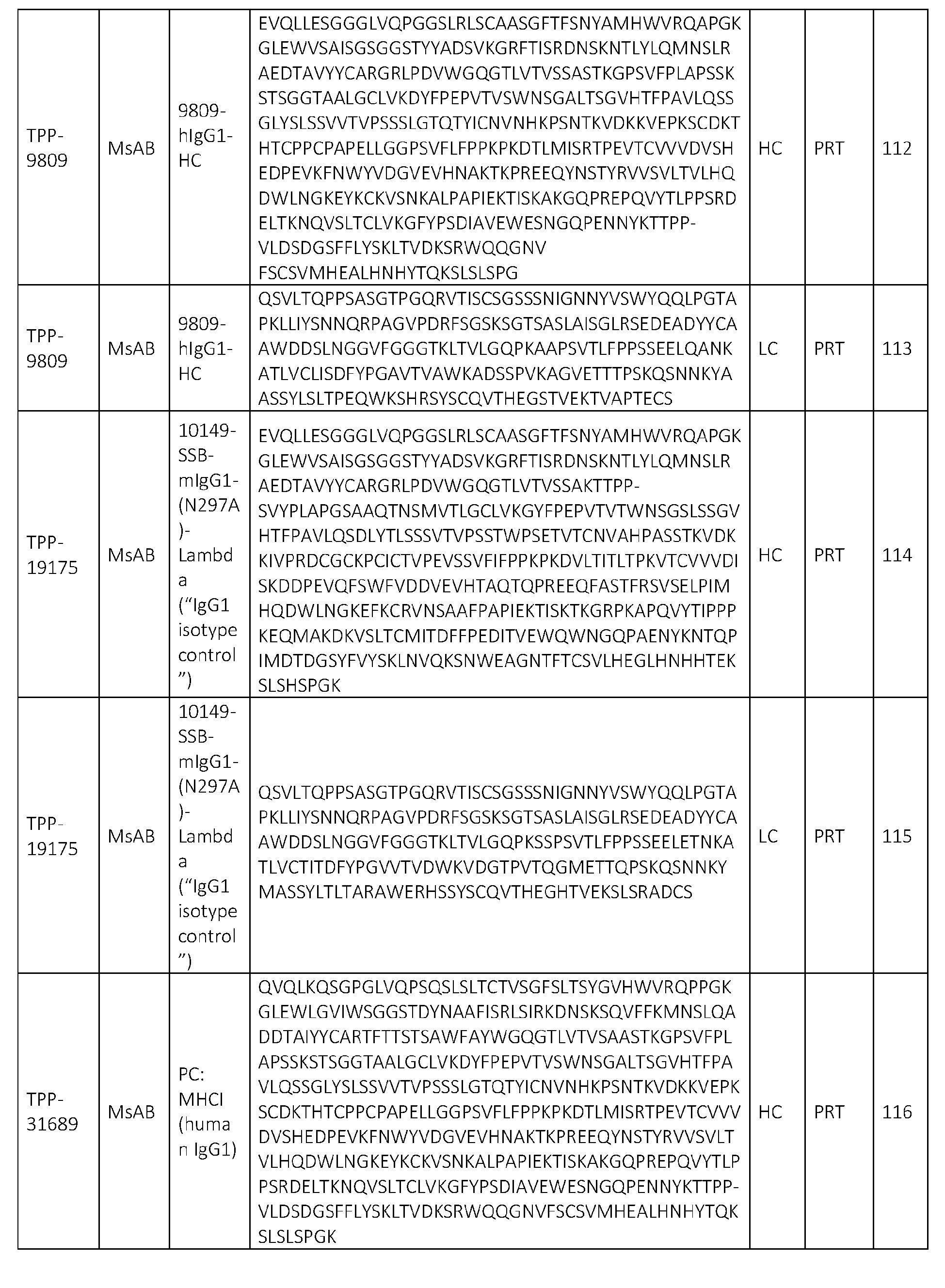

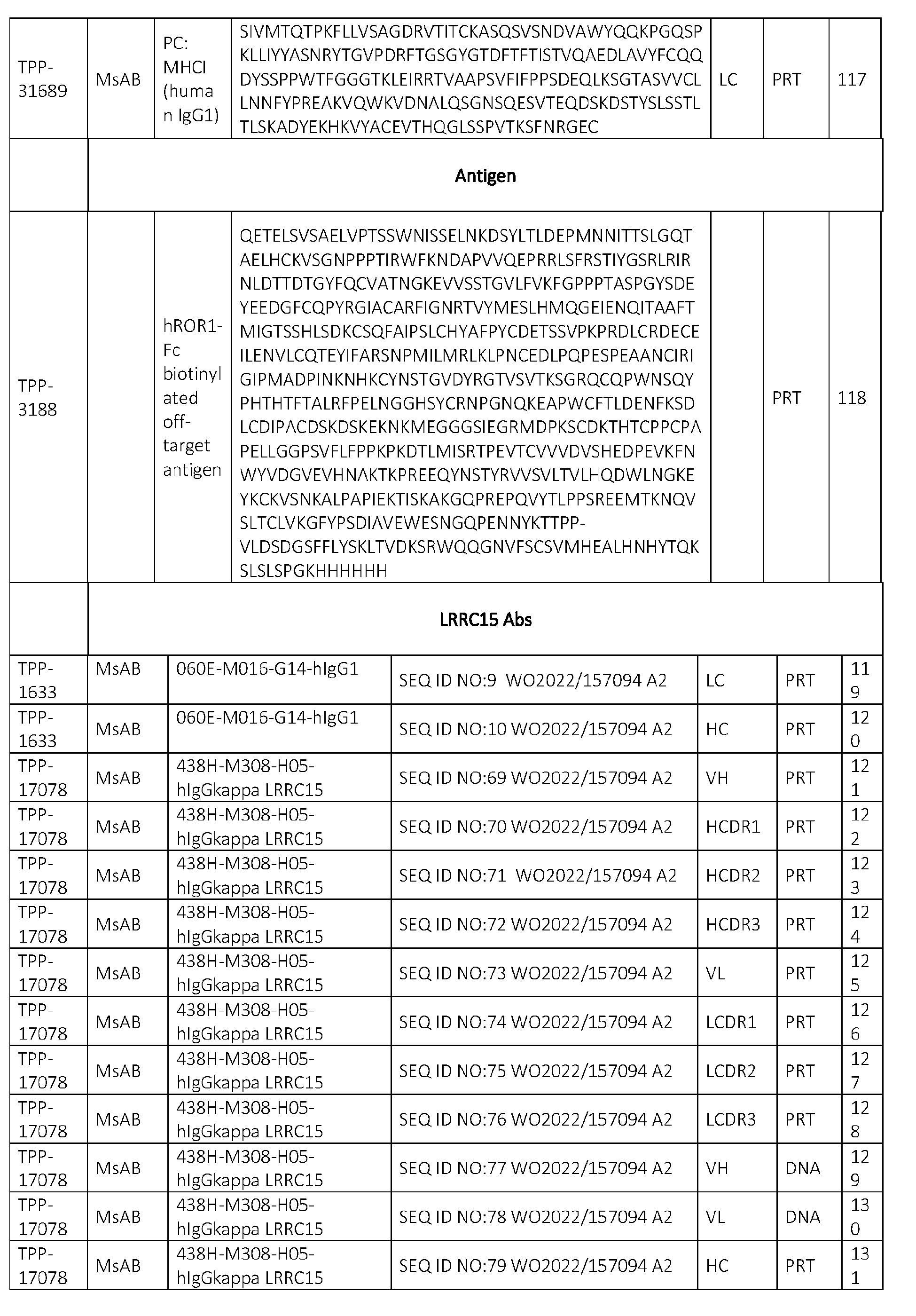

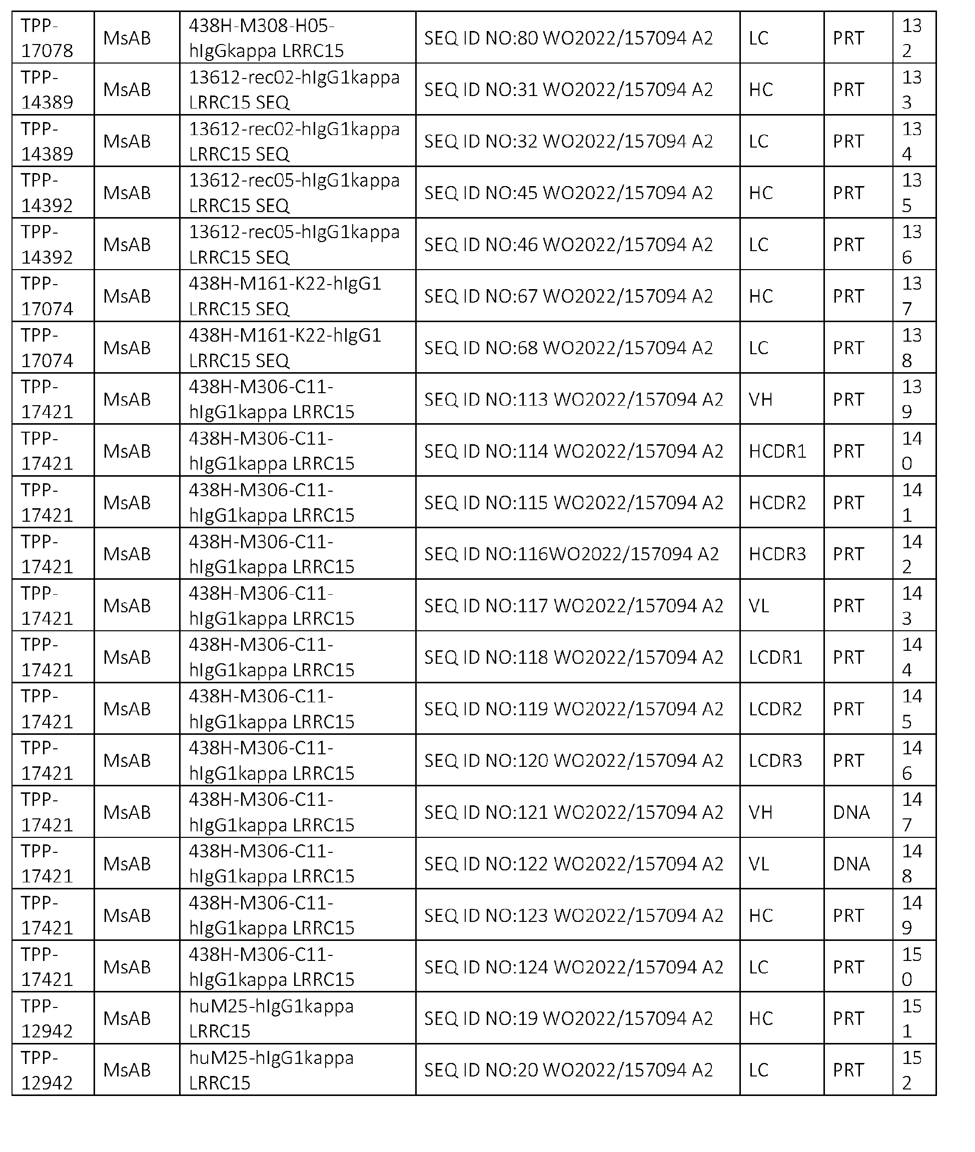

### Definitions

### General definitions

Unless otherwise defined, all scientific and technical terms used in the description, figures and claims have their ordinary meaning as commonly understood by one of ordinary skill in the art. All publications, patent applications, patents, and other references mentioned herein are incorporated by reference in their entirety. In case of conflict, the present specification, including definitions, will prevail. If two or more documents incorporated by reference include conflicting and/or inconsistent disclosure with respect to each other, then the document having the later effective date shall control. The materials, methods, and examples are illustrative only and not intended to be limiting. Unless stated otherwise, the following terms used in this document, including the description and claims, have the definitions given below.

Singular forms such as "**a**", "**an**" or "**the**" include plural references unless the context clearly indicates otherwise. Thus, for example, reference to a "BsAB" includes a single BsAB as well as a plurality of BsAB, either the same or different. Likewise reference to "cell" includes a single cell as well as a plurality of cells.

The word "**about**" as used herein refers to a value being within an acceptable error range for the particular value as determined by one of ordinary skill in the art, which will depend in part on how the value is measured or determined, i. e., on the limitations of the measurement system. For example, "about" can mean within 1 or more than 1 standard deviation per the practice in the art. The term "about" is also used to indicate that the amount or value in question may be the value designated or some other value that is approximately the same. The phrase is intended to convey that similar values promote equivalent results or effects as described herein. In this context "about" may refer to a range above and/or below of up to 20% or 10 %. Wherever the term "about" is specified for a certain assay or embodiment, that definition prevails for the particular context.

It is furthermore understood that slight variations above and below a stated range can be used to achieve substantially the same results as a value within the range. Also, unless indicated otherwise, the disclosure of ranges is intended as a continuous range including every value between the minimum and maximum values.

The terms "**comprising**", "**including**", "**containing**", "**having**" etc. shall be read expansively or openended and without limitation.

Unless otherwise indicated, the term "**at least**" preceding a series of elements is to be understood to refer to every element in the series. The terms **"at least one"** and **"at least one of"** include for example, one, two, three, four, or five or more elements.

### Detailed description

The term "**isolated**" when applied to a defined biological subject matter such as a nucleic acid, gene, polypeptide, protein or antibody, denotes that the biological subject matter is essentially free of other cellular components with which it is associated in the natural state. In particular, an isolated gene is separated from open reading frames that flank the gene and encode a protein other than the gene of interest. The isolated subject matter is preferably in a homogenous state and may be without limitation in a dry state, or in an aqueous solution.

A nucleic acid, polypeptide, protein, antibody or cell that is the predominant species present in a preparation is called "**substantially purified**". Preferably, this means that the nucleic acid, polypeptide, protein, antibody or cell is at least 85% pure, more preferably at least 95% pure, and most preferably at least 99% pure. Purity and homogeneity are typically determined using analytical chemistry techniques such as polyacrylamide gel electrophoresis or high performance liquid chromatography, or fluorescence activated cell sorting for cells.

A "**detectable moiety**" is a moiety arranged for detection using a matching imaging technology. Examples of detectable moieties include various enzymes or enzymatic labels, prosthetic groups, fluorescent groups or materials, luminescent groups or materials, bioluminescent groups or materials, radioactive isotopes or materials, positron emitting metals, nonradioactive paramagnetic metal ions, and reactive moieties. The detectable substance can be coupled or conjugated either directly to the conjugate, antibody or fragment thereof or indirectly, e.g. without limitation through a linker known in the art or through another moiety, using techniques known in the art. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, β-galactosidase, acetylcholinesterase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Examples of suitable prosthetic groups include streptavidin/biotin and avidin/biotin. Examples of suitable fluorescent groups or materials include umbelliferone, fluorescein, fluorescein isothiocyanate, rhodamine, dichlorotriazinylamine fluorescein, dansyl chloride or phycoerythrin. An example of a luminescent group or material includes luminol. Examples of bioluminescent groups or materials include luciferase, luciferin, and aequorin. Examples of suitable radioactive isotopes or materials include ¹²⁵I, ¹³¹I, ¹¹¹In or ^{99m}Tc.

### Targets

The term "**LRRC15**" refers to the protein Leucine-rich repeat-containing protein 15. The human LRRC15 protein is encoded by the gene LRRC15 (NCBI gene ID 131578). A synonym for LRRC15 is LIB. The LRRC15 protein comprises human, murine, cynomolgus and further mammalian and non-mamalian homologues. Sequence(s) for human LRRC15 are accessible via UniProt Identifier Q8TF66

(LRC15_HUMAN), for instance human isoform Q8TF66-1 or Q8TF66-2 (Entry version 159, June 17, 2020). Sequence(s) for murine LRRC15 are accessible via UniProt Identifier Q80X72 (LRRC15_MOUSE). Sequence(s) for cynomolgus (Macaca fascicularis) LRRC15 are accessible via UniProt Identifier G7NYR2 (G7NYR2_MACFA). Different isoforms and variants may exist for the different species and are all comprised by the term LRRC15. Also comprised are LRRC15 molecules before and after maturation, i.e., independent of cleavage of one or more pro-domains. In addition, synthetic variants of the LRRC15 protein may be generated and are comprised by the term LRRC15. The protein LRRC15 may furthermore be subject to various modifications, e.g, synthetic or naturally occurring modifications. WO2022/157094 A2 discloses antibodies binding to LRRC15 and demonstrates especially in figures 8-11 and Table 14 (cf. reproduction below) via immunohistochemistry (IHC) analysis and via RNAseq data, available via the "the cancer genome atlas (TCGA) database" that LRRC15 is expressed in different tumor types, including breast cancer > head and neck squamous cell cancer > squamous lung cancer > pancreatic cancer > diffused large B-cell carcinoma > lung adenocarcinoma > colorectal cancer > gastric cancer, as well as Sarcoma.

**Table 1 (cf. Table 14 of WO2022/157094 A2) Summary of LRRC15-stained xenograft and murine syngeneic models, listed by respective tissue type. LRRC15 staining intensities were scored from 1+ to 3+ upon visual inspection.**

| Tissue | Model | IHC Score | | |
|---|---|---|---|---|
| | | 1+ | 2+ | 3+ |
| Sarcoma | Sarc 4183 | | | 3+ |
| | Sarc 9503 | | | 3+ |
| | Sarc 10751 | | | 3+ |
| Breast Cancer | KPL-4 | 1+ | | |
| | T47D | | | 3+ |
| | MX-1 | | 2+ | |
| | MDA-MB-231 | | 2+ | |
| | BT20 | 1+ | | |
| | BT-474 | 1+ | | |
| | MCF-7 | | | 3+ |
| | MFM-223 | 1+ | | |
| NSCLC | NCI-H292 | | | 3+ |
| | NCI-H1975 | 1+ | | |
| | NCI-H460 | 1+ | | |
| | A549 | | 2+ | |
| | NCI-H322 | | | 3+ |
| | NCI-H522 | 1+ | | |
| | NCI-H1993 | 1+ | | |
| | NCI-H441 | | 2+ | |
| | NCI-H228 | | 2+ | |
| | NCI-H82 | | 2+ | |
| | NCI-H226 | | 2+ | |
| Murine Breast Cancer | 4T1 | | 2+ | |
| Murine Lung Cancer | Lewis Lung | | 2+ | |
| Murine Colorectal Cancer | MC38 | | | |
| | CT26 | 1+ | | |
| Murine Testicular Teratoma | F9 | 1+ | | |
| Murine Melanoma | B16F10 | 1+ | | |

The term "**LTβR**" refers to the protein Lymphotoxin Beta Receptor. The human LTβR is encoded by the gene LTβR (NCBI gene ID: 4055). Synonyms for LTβR are CD18, D12S370, LT-BETA_R, TNF_R_III, TNFCR, TNFR_RP, TNFR2_RP, TNFR3, TNFRSF3, TNR3. The LTβR protein comprises human, murine, cynomolgus and further mammalian and non-mamalian homologues. Sequence(s) for human LTβR are accessible via UniProt Identifier P36941 (TNR3_HUMAN)), for instance human isoform P36941-1 (referred to as canonical sequence) or P3694-2 (Entry version 196, September 29, 2021). Sequence(s) for murine LTβR are accessible via UniProt Identifier P50284 (LTβR_MOUSE).). Different isoforms and variants may exist for the different species and are all comprised by the term LTβR. Also comprised are LTβR molecules before and after maturation, i.e., independent of cleavage of one or more pro-domains. In addition, synthetic variants of the LTβR protein may be generated and are comprised by the term LTβR. The protein LTβR may furthermore be subject to various modifications, e.g, synthetic or naturally occurring modifications. Recombinant human LTβR (rh LTβR) is commercially available or can be manufactured as known in the art.

### Biological subject-matter

The terms "**peptide**", "**polypeptide**", and "**protein**" are used interchangeably herein, and refer to a compound which comprises at least two amino acid residues covalently linked by at least one peptide bond. No limitation is placed on the maximum number of amino acids that can comprise a peptide's sequence. A "peptide" may comprise without limitation modified amino acids, non naturally-occuring amino acids and/or D amino acids. Unless otherwise indicated, a particular peptide sequence also encompasses variants wherein at least one amino acid has been replaced by an amino acid which is characterized by similar structural properties. A "peptide" may be a natural peptide, a recombinant peptide, a synthetic peptide, or a combination thereof. A "peptide" may be, for example, a biologically active fragment, an oligopeptide, a homodimer, a heterodimer, a peptide variant, a modified peptide, a peptide derivative, a peptide analog, a fusion protein, among others.

The term "**amino acid**" or "**amino acid residue**" ("aa") as used herein typically refers to a naturally-occurring amino acid but may also refer to a non naturally-occuring amino acid. The term typically refers to an L-amino acid but may also encompass a D-amino acid. An amino acid may or may not be modified as described elsewhere herein. The one letter code is used herein to refer to the respective amino acid. The term "**nucleic acid**" refers to deoxyribonucleotides or ribonucleotides and polymers thereof composed of monomers (nucleotides) containing a sugar, phosphate and a base that is either a purine or pyrimidine. For example and without limitation nucleic acids may occur in single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., (1991); Ohtsuka et al., (1985); Rossolini et al., (1994)).

A "**nucleic acid fragment**" is a portion of a given nucleic acid molecule. Deoxyribonucleic acid (DNA) in the majority of organisms is the genetic material while ribonucleic acid (RNA) is involved in the transfer of information contained within DNA into peptides or proteins.

The term "**nucleotide sequence**" refers to a polymer of DNA or RNA which can be single- or double-stranded, optionally containing synthetic, non-natural or altered nucleotide bases capable of incorporation into DNA or RNA polymers.

The terms "**nucleic acid**", "**nucleic acid molecule**", "**nucleic acid fragment**", "**nucleic acid sequence or segment**", or "**polynucleotide**" are used interchangeably and may also be used interchangeably with gene, cDNA, DNA and RNA encoded by a gene.

The term "**gene**" is used broadly to refer to any segment of nucleic acid associated with a biological function. Thus, genes include coding sequences and/or the regulatory sequences required for their expression. For example, "gene" refers to a nucleic acid fragment that expresses mRNA, functional RNA, or specific protein, including regulatory sequences. "Genes" also include nonexpressed DNA segments that, for example, form recognition sequences for other proteins. "Genes" can be obtained from a variety of sources, including cloning from a source of interest or synthesizing from known or predicted sequence information, and may include sequences designed to have desired parameters. An "allele" is one of several alternative forms of a gene occupying a given locus on a chromosome.

"**Sequence identity**", "**percent identity**" or "**percent (%) sequence identity**" is a number that describes how similar a query sequence is to a target sequence, more precisely how many characters in each sequence are identical after alignment. The most popular tool to calculate sequence identity is BLAST (basic local alignment search tool, NCBI), which performs comparisons between pairs of sequences, searching for regions of local similarity. Suitable alignment methods are known in the art, e.g. Needleman-Wunsch algorithm for global-global alignment, using BLOSUM62 matrix, with gap opening penalty of 11 and a gap extension penalty of 1. Afterwards, the pairs of aligned identical residues can be counted and then divided by the total length of the alignment (including gaps, internal as well as external) to arrive at the percent identity value. For "percent similarity" values, the same approach as for percent identity values can be used, except that what is counted, instead of pairs of identical residues, would be the aligned residue pairs with BLOSUM62 values that are not negative (i.e., ≥0). "Sequence homology" indicates the percentage of amino acids that are identical or that represent conservative amino acid substitutions.

A "**host cell**" is a cell that is used in to receive, maintain, reproduce and amplify a vector. A host cell also can be used to express the polypeptide encoded by the vector. The nucleic acid contained in the vector is replicated when the host cell divides, thereby amplifying the nucleic acids.

The term "**vector**", as used herein, refers to a nucleic acid molecule capable of propagating a nucleic acid molecule to which it is linked. The term further comprises plasmids (non-viral) and viral vectors. Certain vectors are capable of directing the expression of nucleic acids or polynucleotides to which they are operatively linked. Such vectors are referred to herein as "expression vectors". Expression vectors for eukaryotic use can be constructed by inserting a polynucleotide sequence encoding at least one protein of interest (POI) into a suitable vector backbone. The vector backbone can comprise the necessary elements to ensure maintenance of the vector and, if desirable, to provide amplification within the host. For viral vectors, e.g. lentiviral or retroviral vectors, further virus specific elements such as structural elements or other elements can be required and are well known in the art. These elements can be for instance provided in cis (on the same plasmid) or in trans (on a separate plasmid). Viral vectors may require helper viruses or packaging lines for large-scale transfection. Vectors may contain further elements such as e.g. enhancer elements (e.g. viral, eukaryotic), introns, and viral origins of plasmid replication for replication in mammalian cells. According to what is described herein, expression vectors typically have a promoter sequence that drives expression of the POI. Expression of the POI and/or selective marker protein may be constitutive or regulated (e.g. inducible by addition or removal of small molecule inductors). Preferred regulatory sequences for mammalian host cell expression include viral elements that direct high levels of expression of a POI in mammalian cells, such as regulatory elements, promoters and/or enhancers derived from cytomegalovirus (CMV), Simian Virus 40 (SV40), adenovirus, (e.g., the adenovirus major late promoter Ad LP) or polyoma. For further description of viral regulatory elements, and sequences thereof, see e.g., U.S. 5.168.062 by Stinski, U.S. 4,510,245 by Bell et al. and U.S. 4,968,615 by Schaffner et al.

### Antibodies

The term "**antibody**" includes, but is not limited to, an immunoglobulin molecule (e.g. without limitation human IgG1, IgG2, IgG3, IgG4, IgM, IgD, IgE, IgA1, IgA2, mouse IgG1, IgG2a, IgG2b, IgG2c, IgG3, IgA, IgD, IgE or IgM, rat IgG1, IgG2a, IgG2b, IgG2c, IgA, IgD, IgE or IgM, rabbit IgA1, IgA2, IgA3, IgE, IgG, IgM, goat IgA, IgE, IgG1, IgG2, IgE, IgM or chicken IgY) that binds to a particular antigen. The term also comprises bispecific antibodies as described elsewhere herein. Depending on the context, the term antibody may also refer to a functional fragment of a full length antibody such as a single-chain Fv (scFv fragment). The term furthermore includes any proteinaceous binding molecule with immunoglobulin-like function. According to what is described herein, the antibodies or antigen binding domains thereof are preferably characterized by an affinity for their target corresponding to a KD of less than 10⁻⁷ M, more preferably of less than 10⁻⁸ M, even more preferably in the range from 10⁻¹¹ M to 10⁻⁹ M.An antibody may be composed of two identical pairs of polypeptide chains. In particular embodiments, antibodies may comprise four polypeptide chains, e.g. two "**heavy chains**" (**H**) (about 50-70 kDa) and two "**light chains**" **(L)** (about 25 kDa), which may be connected by disulfide bonds.

The amino-terminal portion of a polypeptide chain of an antibody usually comprises a "**variable region**", e.g. of about 100 to 110 or more amino acids, which is primarily responsible for antigen recognition. The heavy chain variable region is abbreviated herein as "**VH**", the light chain variable region is abbreviated herein as "**VL**". The VH and VL regions can be further subdivided into regions of hypervariability, termed CDRs, interspersed with regions that are more conserved, termed "**framework regions**" or "**FR**". Each VH and VL is typically composed of three CDRs and up to four FRs, arranged from amino-terminus to carboxy-terminus e.g., in the following order: FR1, CDR1, FR2, CDR2, FR3, CDR3, FR4.

The term "**CDR**" refers to the complementary determining region of an antibody. CDRs are crucial to the diversity of antigen specificities. A set of CDRs constitutes a paratope. There are usually three CDRs (CDR1, CDR2 and CDR3), arranged non-consecutively on the amino acid sequence of a variable domain of an antigen receptor. The CDRs are typically held together in close proximity by the FR regions and - e.g. with the CDRs from the other chain - contribute to the formation of the antigen binding site of antibodies. The CDRs of the light chain are termed LCDR1, LCDR2 and LCDR3. The CDRs of the heavy chain are termed HCDR1, HCDR2 and HCDR3. HCDR3 is the most variable CDR. As known in the art, the amino acid position/boundary delineating a hypervariable region of an antibody can vary, depending on the context and the various definitions known in the art. As used herein, numbering of antibody amino acid residues is done according to the immunoglobulin amino acid residue numbering system of Kabat. The carboxy-terminal portion of each polypeptide chain of an antibody usually comprises a "**constant region**", a portion of the antibody molecule that confers effector functions. The heavy chain constant region can comprise e.g. three domains CH1, CH2 and CH3. The light chain constant region is comprised of one domain (CL). The heavy chain constant region can for example be selected from any of the five isotypes: alpha (α), delta (δ), epsilon (ε), gamma (g), or mu (µ).

The term "**fragment crystallizable region**", also "**Fc domain**", "**Fc region**" or "**Fc part**" as used herein refers to a C-terminal region of an antibody heavy chain that contains at least a portion of the constant region. The Fc region may interact with Fc receptors and some proteins of the complement system, e.g. on immune effector cells. The Fc region defines the class of an antibody or isotype. For an IgG antibody, the Fc region consists of the C2H and C3H domains. The term includes native sequence Fc regions and variant Fc regions. For example, a human IgG heavy chain Fc region may extend from Cys226, or from Pro230, to the carboxyl-terminus of the heavy chain.

The term "**bispecific antibody** (BsAB)" according to what is described herein refers to antibodies especially monoclonal antibodies that have binding specificities i.e. antigen binding domains for at least two different epitopes i.e. antigen binding sites. The at least two different epitopes of the BsAB constructs described herein are associated with at least two different targets. BsAB constructs according to what is described herein thus comprise specificities for at least two different antigens or targets. Bispecific antibodies usually do not occur naturally and are usually artificial hybrid antibodies or immunoglobulins. A variety of formats for bispecific antibodies and methods to produce them are well known in the art (Songsivilai & Lachmann, Clin. Exp. Immunol. 79:315-321 (1990)). These include but are not limited to bi- or multispecific full length antibodies or antibody fragments, single chain antibodies, diabodies and triabodies, Fc fusions and antibodies having the constant domain structure of full length antibodies to which further antigen-binding sites are linked via one or more linker or peptide-linker for example N-terminal and/or C-terminal. Possible further antigen-binding sites can be comprised for example in single chain Fv (scFv), VH domain and/or VL domain, Fab, (Fab)2, VHH nanobodies (Hamers-Casterman C et al., (1993) Nature 363(6428), 446-448), single domain antibodies, scFabs, or fragments of any of these. Techniques for making bi- or multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and chemical conjugation of two different monoclonal antibodies (see Staerz et al. (1985) Nature 314(6012): 628-31). Multispecific antibodies may also be made by cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol, 148(5): 1547-1553 (1992)) and using single- chain Fv (sFv) dimers (see,e.g. Gruber et al., J. Immunol, 152:5368 (1994)); and preparing trispecific antibodies as described, e.g., in Tutt et al. J. Immunol. 147: 60 (1991) and by controlled Fab arm exchange (cFAE) (Labrijn AF et al. Proc Natl Acad Sci USA 2013; 110:5145-50). Hence bispecific antibody formats comprise IgG-like and non-IgG-like antibodies (Fan et al (2015) Journal of Hematology & Oncology. 8: 130). Examples of IgG-like bispecific antibodies are compounds with monoclonal antibody (mAb) structure of two Fab arms and one Fc region, wherein the two Fab sites bind different antigens e.g. because each heavy and light chain pair may be from a unique mAb i.e. these bispecific antibodies are bivalent. Bispecific antibody constructs according to what is described herein are an example of multi-specific antibody constructs binding to different epitopes. Hence a bispecific antibody refers to antibodies binding at least two different epitopes and a trispecific antibody construct refers to an antibody binding at least three different epitopes.

The term "**valent**" according to what is described herein denotes the presence of a specified number of antigen binding domains in an antibody molecule. As such, the terms bivalent, trivalent, tetravalent denote the presence of two, three or four binding sites, respectively, in an antibody construct. The bispecific antibodies according to what is described herein are at least bivalent and may be multivalent, for example bivalent, trivalent, tetravalent or hexavalent.

Antibodies e.g. bispecific antibodies or antigen binding domains thereof can be full length from a single species. Antibodies e.g. bispecific antibodies or antigen binding domains thereof can be chimeric. Antibodies e.g. bispecific antibodies or antigen binding domains thereof can be fully or partially humanized, as known in the art.

Antibodies e.g. bispecific antibodies or antigen binding domains thereof according to what is described herein may have been modified to alter at least one Fc region-mediated biological effector function, e.g. by reduced or improved binding to the Fc receptor (e.g. FcyR). FcyR binding may be reduced, e.g. by mutating the immunoglobulin constant region / Fc region of the antibody (See, e.g., Canfield and Morrison, 1991, J. Exp. Med. 173: 1483-1491; and Lund et al., 1991, J. Immunol. 147:2657-2662), or may be enhanced, e.g. by afucosylation. Reducing or enhancing Fc(y)R binding may also reduce or enhance other effector functions which rely on Fc(y)R interactions, such as opsonization, phagocytosis, ADCP or ADCC.

Antibodies e.g. bispecific antibodies or antigen binding domains thereof can be produced synthetically or recombinantly. A number of technologies are available to produce antibodies. For example, phage-antibody technology can be used to generate antibodies (Knappik et al., J. Mol. Biol. 296:57-86, 2000). Another approach for obtaining antibodies is to screen a DNA library from B cells as described in WO 91/17271 and WO 92/01047. In these methods, libraries of phages are produced in which members display different antibodies on their outer surfaces. Antibodies are usually displayed as Fv or Fab fragments. Phage displaying antibodies are selected by affinity enrichment for binding to a selected protein. Antibodies can also be produced using trioma methodology (e.g., Oestberg et al., Hybridoma 2:361-367, 1983; U.S. Patent 4,634,664; U.S. Patent 4,634,666).

Antibodies e.g. bispecific antibodies or antigen binding domains thereof can also be purified from any cell that expresses the antibodies, including host cells that have been transfected with antibody-encoding expression constructs. The host cells can be cultured under conditions whereby the antibodies are expressed. Purified antibody can be separated from other cellular components that can associate with the antibody in the cell, such as certain proteins, carbohydrates, or lipids, using methods well known in the art. Such methods include, but are not limited to, size exclusion chromatography, ammonium sulfate fractionation, ion exchange chromatography, affinity chromatography, and preparative gel electrophoresis. Purity of the preparations can be assessed by any means known in the art, such as SDS-polyacrylamide gel electrophoresis. A preparation of purified antibodies can contain more than one type of antibody.

Alternatively, antibodies e.g. bispecific antibodies or antigen binding domains thereof according to what is described herein can be produced using chemical methods to synthesize its amino acid sequence, such as by direct peptide synthesis using solid-phase techniques (e.g., Merrifield, J. Am. Chem. Soc. 85:2149-2154, 1963; Roberge et al., Science 269:202-204, 1995). Protein synthesis can be performed using manual techniques or by automation. Optionally, fragments of antibodies can be separately synthesized and combined using chemical methods to produce a full-length molecule.

The term "**binding domain**" or "**antigen binding domain**" as used herein refers to any portion of an antibody e.g. a bispecific antibody which binds to a specific target or antigen. An antigen binding domain is an antigen binding site i.e. it binds an epitope. Such an antigen binding domain may or may not have a tertiary structure which is independent from the rest of the antibody e.g. a bispecific antibody and may or may not bind its target as an individual entity.

A "**targeting moiety**" as used herein is any chemical structure binding to a biological target expressed on tumor cells or located in a tumor microenvironment. Upon binding of the targeting moiety to its biological target LTβR signaling pathway is induced, stimulated or upregulated in the cell expressing the biological target or a neighboring cell(s). The targeting moiety localizes itself as part of a bigger structure e.g., the isolated tetravalent bispecific antibody described herein at a specific site where the presence is required to exert the intended effect of LTβR signaling pathway induction and/or stimulation. Thus, a targeting group or moiety serves to provide greater localization of a molecule to at least one desired site in the body of a subject in comparison with the concentration of an equivalent complex not comprising the targeting moiety. According to what is described herein, the targeting moiety can be for example selected without limitation from the group consisting of nucleotides, DNA and RNA fragments, aptamers, peptides, proteins, antibodies or fragments thereof, nanoparticles, or small molecules, or any combination thereof. Thus an antigen binding domain is always also a targeting moiety. Hence also each antigen binding domain other than an antigen binding domain capable of binding to LTβR is a targeting moiety in the sense used herein.

A "**targeting moiety binding to (human) LRRC15**" is a chemical structure binding to (human) protein LRRC15. According to highly preferred embodiments, the targeting moiety can be an antibody or antigen binding domain thereof, as described in WO2022/157094 A2.

A "**targeted LTβR antibody**" refers to the isolated tetravalent bispecific antibody of the first aspect wherein the antibody comprises two targeting moieties for a site where the presence of the isolated bispecific antibody of the first aspect is required to exert the intended effect of LTβR signaling pathway induction and/or stimulation. In contrast a "**non-targeted LTβR antibody**" refers to an isolated tetravalent bispecific antibody comprising at least one, preferably two targeting moieties specific for a target which is not present or present in insufficient quantities in a given experimental setting or *in vivo* environment.

The term "**linker**" as used herein refers to any molecule enabling a direct topological connection of different portions of a BsAB or antibody construct. Examples for linkers establishing a covalent connection between the different antibody portions include peptide linker and non-proteinaceous polymers, including but not limited to polyethylene glycol (PEG), polypropylene glycol, polyoxyalkylenes, or copolymers of polyethylene glycol, polypropylene glycol.

The term "**peptide linker**" according to what is described herein refers to a sequence of amino acids, wherein said sequence links the amino acid sequence of a first portion of an antibody construct to a second portion of an antibody construct. For example, a peptide linker can link a first (variable and/or binding) domain to a second variable and/or binding) domain of the antibody construct. For example, a peptide linker can also link a portion of the antibody construct to another portion of the antibody construct, such as an antigen binding domain to an Fc domain or a fragment thereof. Suitable peptide linkers are described in US4751180, US 4935233, WO 88/09344 and Holliger et al. (1993) Proc. Natl. Acad. Sci. USA 90:6444-6448; Poljak et al. (1994) Structure 2:1121-1123. Preferably, the peptide linker has a length that is adequate to link two entities in such a way that they maintain their conformation relative to each other, such that the desired activity is not hampered. In particular, where the bispecific antibody construct according to the what is described herein comprises one or more linkers, the one or more linker preferably have a length and sequence which do not disturb the individual binding specificities of the antigen binding domains. The linker peptide may or may not predominantly include the following amino acid residues: Gly, Ser, Ala, or Thr. Useful linkers include glycine-serine polymers, including for example (GS)ₙ, (GSGGS)ₙ, (GGGGS)ₙ, (GGGS)ₙ, and (GGGGS)ₙG, where n is an integer of at least one (and preferable 2, 3, 4, 5, 6, 7, 8, 9, 10). Useful linkers also include glycine-alanine polymers, alanine-serine polymers, and other flexible linkers. Linker sequences may include any sequence of any length of CL/CH1 domain or not all residues of CL/CH1 domain. Linkers can be derived from immunoglobulin light chain, for example Cκ or Cλ. Linkers can be derived from immunoglobulin heavy chains of any isotype, including for example Cγ1, Cy2, Cy3, Cy4, Cα1, Cα2, C8, Cα, and Cµ. Linker sequences may also be derived from other proteins such as Ig-like proteins (e.g. TCR, FcR, KIR), hinge region-derived sequences, other natural sequences from other proteins, or can be charged linkers. Methods for linking the domains to each other are well known in the art and comprise for example genetic engineering. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).

Where an antibody e.g. bispecific antibody or antigen binding domains thereof according to what is described herein is in a certain format, this does not exclude further modifications including but not limited to natural or synthetic attachments or fusion to other elements. In particular, an antibody construct or a BsAB may be PEGylated or (hyperglycosylated.

The terms "**(anti) LRRC15 antibody**", "**antibody binding LRRC15**" and "**antibody that binds to LRRC15**" are used synonymously herein and comprise at least one antigen binding domain capable of binding LRRC15, preferably with sufficient affinity such that the antibody is useful as a diagnostic and/or therapeutic agent in targeting LRRC15. In some embodiments, the extent of binding of an antibody binding LRRC15 to an unrelated protein different from LRRC15 is less than about 10 %, less than about 5%, or preferably less than about 2%, and most preferably less than about 1 % of the binding of the antibody to LRRC15 as measured, e.g. by surface plasmon resonance (SPR) or a further standard method such as ELISA assay. In certain preferred embodiments, an antibody that binds to LRRC15 has a dissociation constant (K_{D}) of ≤ 1 µM, ≤ 500 nM, ≤ 200 nM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an antibody that binds to LRRC15 has a binding activity (EC50) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an anti LRRC15 antibody binds to an antigen - i.e. an epitope thereof - of LRRC15 that is conserved among LRRC15 from different species. LRRC15 is expressed on activated fibroblasts in tumor infiltrating stroma and in sarcoma cells.

The terms "**(anti) LTβR antibody**", "**antibody binding LTβR** " and "**antibody that binds to LTβR**" are used synonymously herein and comprise at least one antigen binding domain capable of binding LTβR preferably with sufficient affinity such that the antibody is useful as a diagnostic and/or therapeutic agent in targeting LTβR. In certain embodiments, an antigen binding domain binding to human LTβR and hence the antibody binding LTβR binds to an antigen - i.e. an epitope thereof - of LTβR that is conserved among LTβR from different species. In some embodiments, the extent of binding of an antigen binding domain binding to human LTβR and hence the antibody binding LTβR to an unrelated protein different from LTβR is less than about 10 %, less than about 5%, or preferably less than about 2%, and most preferably less than about 1 % of the binding of the antibody to LTβR as measured, e.g. by surface plasmon resonance (SPR) or a further standard method such as ELISA assay. In certain preferred embodiments, an antigen binding domain binding to human LTβR and hence the antibody binding LTβR has a dissociation constant (K_{D}) of ≤ 1 µM, ≤ 500 nM, ≤ 200 nM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹M to 10⁻¹¹ M). In certain embodiments, an antigen binding domain binding to human LTβR and hence the antibody binding LTβR has a binding activity (EC50) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In the present disclosure, at least two of the antigen binding domains of the isolated tetravalent bispecific antibody are directed towards LTβR, and at least one other antigen binding domain is directed to e.g., without limitation a cell-surface protein, receptor, receptor subunit, tissue-specific antigen, virally derived protein, virally encoded envelope protein, bacterially derived protein, or bacterial surface protein.

### Antibody fragments

A **"fragment", "functional fragment" or "antigen-binding fragment" or "antigen binding domain"** is required to retain the ability of an antibody to bind the particular antigen. Fragments of an antibody therefore typically comprise a functional portion of a full-length antibody, generally the antigen binding region or variable region thereof. Preferably, a fragment of an antibody as used herein substantially retains the affinity of the full-length antibody. For example, suitable fragments of the anti-LRRC15 antigen binding domain binding to (human) LRRC15 and/or anti-LTβR antigen binding domain binding to (human) LTβR of the bispecific antibody will retain the ability to bind to the target proteins, e.g. to bind to LRRC15 or LTβR, respectively. Examples of antibody fragments include, but are not limited to, Fab, Fab', F(ab')2, Fv fragments, single-chain antibody molecules such as single-chain Fv (scFv), diabodies and domain antibodies, see Holt, L.J., et al., Trends Biotechnol. (2003), 21, 11, 484-490.

A "**Fab fragment**" contains the constant domain of the light chain and the first constant domain (CH2) of the heavy chain. "**Fab' fragments**" differ from Fab fragments by the addition of a few residues at the carboxyl terminus of the heavy chain CH2 domain including one or more cysteines from the antibody hinge region. "**F(ab') fragments**" are produced by cleavage of the disulfide bond at the hinge cysteines of the F(ab')2 pepsin digestion product. Additional chemical couplings of antibody fragments are known to those of ordinary skill in the art. Fab and F(ab')2 fragments lack the Fc fragment of intact antibody, clear more rapidly from the circulation of animals, and may have less non-specific tissue binding than an intact antibody, see, e.g., Wahl et al., 1983, J. Nucl. Med. 24:316.

An "**Fv fragment**" is the minimum fragment of an antibody that contains a complete target recognition and binding site. This region consists of a dimer of one heavy and one light chain variable domain in a tight, non-covalent association (VH-VL dimer). It is in this configuration that the three CDRs of each variable domain interact to define an antigen binding site on the surface of the VH-VL dimer. Often, the six CDRs confer antigen binding specificity upon the antibody. However, in some instances even a single variable domain (or half of an Fv comprising only three CDRs specific for a target) may have the ability to recognize and bind antigen, although at a lower affinity than the entire binding site.

**Single-chain Fv**" or "**single chain Fv**" or "**scFv**" antibody fragments comprise the heavy chain variable region- VH - and light chain variable region -VL- domains of an antibody in a single polypeptide chain. Generally, the Fv polypeptide further comprises a polypeptide linker between the VH and VL domains which enables the scFv to form the desired structure for antigen binding. Hence also an scFvs comprises six CDRS - three of the VL termed LCDR1, LCDR2 and LCDR3 and thee of the VH termed HCDR1, HCDR2 and HCDR3.

"**Single domain antibodies**" are composed of a single VH or VL domains which exhibit sufficient affinity to the target. In a specific embodiment, the single domain antibody is a camelized antibody, see, e.g., Riechmann, 1999, Journal of Immunological Methods 231:25-38.

"**Potency**" denotes the amount of antibody needed to produce a given effect. Efficacy is the maximal effect that an antibody produces irrespective of concentration (dose). Hence "more potent" as used herein refers to a lower EC50 value in a given assay at a given antibody concentration and/or more stimulation of the LTβR signaling pathway e.g. NF-κβ signaling with the setting as described in example 4.2 or stimulation of chemokines such as CXCL10 and CXCL9 with the setting as described in example 4.1.

### Binding

The term "**affinity**" or "**binding affinity**" is a term of the art and describes the strength of non-covalent binding between a single binding site of a molecule and its binding partner. The affinity of an antibody or fragment thereof such as the antigen binding domain of a bispecific antibody for a target can be determined using techniques well known in the art, for example by ELISA, isothermal titration calorimetry (ITC), surface plasmon resonance (SPR), flow cytometry or fluorescent polarization assays. Preferably the affinity is provided as dissociation constant KD, in the alternative the affinity is provided as an EC50 value.

The "**dissociation constant**" (KD) has molar units (M) and corresponds to the concentration of the binder (e.g. antibody or antigen binding domain thereof) at which half of the target proteins or binding partners are occupied at equilibrium. The smaller the dissociation constant is, the higher is the affinity between the binder and its target. The KD values can be preferably measured by using surface plasmon resonance assays using suitable devices including but not limited to Biacore instruments like Biacore T100, Biacore T200, Biacore 2000, Biacore 4000, a Biacore 3000 (GE Healthcare Biacore, Inc., cf. e.g., Sjolander & Urbaniczky; Anal. Chem. 63:2338-2345, 1991; Szabo, et al., Curr. Opin. Struct. Biol. 5:699-705, 1995), or a ProteOn XPR36 instrument (Bio-Rad Laboratories, Inc.). In the alternative the affinity can be determined using any method known in the art including, for example immunoassays such as enzyme-linked immununospecific assay (ELISA) and fluorescence-activated cell sorting (FACS) for quantification of antibody binding to cells that express an antigen. Where assay conditions were found to influence the determined KD, the assay setup with the least standard deviation shall be used.

"**Half maximal effective concentration**" (EC50) refers to the concentration of a drug, modulator, antibody, fragment, conjugate or molecule which induces a response halfway between the baseline and maximum after a specified incubation time. In the context of affinity, the EC50 thus reflects the concentration of binder (e.g. antibody or antigen binding domain thereof) that is needed for half-maximal binding. An EC50 can be determined if an inflection point can be determined by mathematical modeling (e.g., non-linear regression) of the dose-response curve describing the relationship between applied drug, antibody, fragment, conjugate or molecule concentration and signal. For example, if the dose-response curve follows a sigmoidal curve, an EC50 can be determined. Where the response is an inhibition, the EC50 is termed **"half maximal inhibitory concentration"** (IC50).

As used herein, an antigen binding domain e.g. comprised in a bispecific antibody that **"binds specifically to",** is **"specific to/for"** or **"specifically recognizes"** an antigen of interest, e.g. LRRC15 or LTβR, is one that binds the antigen with sufficient affinity such that it is useful as a therapeutic agent in targeting a cell or tissue expressing the antigen, and does not significantly cross-react with proteins other than orthologs and variants (e.g. mutant forms, splice variants, or proteolytically truncated forms) of the aforementioned antigen target. The term "specifically recognizes" as used herein can be exhibited, for example, by an antigen binding domain e.g. comprised in a bispecific antibody having a monovalent KD for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less.

In its most general form, **"specific binding"** is referring to the ability of an antigen binding domain e.g. comprised in a bispecific antibody to discriminate between the antigen of interest and an unrelated antigen, as determined, for example, by surface plasmon resonance (SPR), Western blot, ELISA-, RIA-, ECL-, IRMA-test or peptide scans. For example, a standard ELISA assay can be carried out. The scoring may be carried out by standard color development (e.g. secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

**"Polyspecificity",** also **"polyreactivity"** or **"unspecific binding"** refers to the antibodies' e.g. the bispecific antibodies ability to bind a defined set of unrelated antigens, i.e. where the antibody e.g. the bispecific antibody binds specifically to a target and binds unspecifically to at least one further unrelated antigen. Polyspecificity is substantial if the (therapeutic) applicability of the antibody e.g. the bispecific antibody is compromised. Binding of non-protein structures including without limitation target negative cell lines or tissues, baculo virus particle (BVP), insulin or DNA, may be evaluated as known in the art and as described herein. In a first example, binding to target negative human cell lines can be determined e.g. by FACS analysis using mock transfected CHO or HEK cells. In a second example, unspecific binding to different tissues or cell populations can be analyzed by FACS analysis of a cell line or panel of cell lines derived from the respective tissue, or by FACS analysis of a sorted cell population. In a third example, unspecific binding to BVP, insulin or DNA can be analyzed using ELISA, e.g. as described in Hötzel, Isidro, et al. "A strategy for risk mitigation of antibodies with fast clearance." MAbs. Vol. 4. No. 6. Taylor & Francis, 2012, Avery, Lindsay B., et al. "Establishing in vitro in vivo correlations to screen monoclonal antibodies for physicochemical properties related to favorable human pharmacokinetics." MAbs. Vol. 10. No. 2. Taylor & Francis, 2018, and Jain, Tushar, et al. "Biophysical properties of the clinical-stage antibody landscape." Proceedings of the National Academy of Sciences 114.5 (2017): 944-949, incorporated herein in their entirety and in particular with regards to the technical details necessary to analyze and quantify unspecific binding. The degree of unspecific binding of an antibody according to what is described herein at least with regard to BVP, insulin and DNA, is preferably lower than the degree of unspecific binding of reference antibody Gantenerumab (Roche) and most preferably lower than the degree of unspecific binding of reference antibody Remicade (Janssen Biotech).

An antibody e.g. a bispecific antibody or antigen binding domain thereof is termed "cross-reactive" or "cross reactive" if the antibody or antigen binding domain thereof binds an antigen from a first species (e.g. human LRRC15 or LTβR) and a related antigen from at least one further species (e.g. cynomolgus LRRC15 ), e.g. without limitation both with a KD value of less than 10-7 M, more preferably of less than 10-8 M, even more preferably in the range from 10-9 M to 10-11 M.

As used herein, the term **"epitope"** refers to a structure that is specifically bound by an antigen binding domain of an antibody e.g. a bispecific antibody or of an antibody fragment. Epitopes may be characterized by specific three dimensional structures or charge patterns. For example, these three dimensional structures or charge patterns may be defined by amino acids or sugar residues. According to preferred embodiments an epitope may be a defined amino acid sequence, which may or may not be modified.

### FC functions

An **"activating Fc domain"** refers to an Fc region of a given antibody that elicits signaling events that stimulate the receptor-bearing cell to perform effector functions upon binding to an Fc domain of an antibody. Human activating Fc receptors include without limitation FcyRIIIa (CD16a), FcγRI (CD64), FcyRIIa (CD32), and FcαRI (CD89).

A "**silent Fc domain**" refers to an Fc region of a given antibody which elicits less signaling events that stimulate the receptor-bearing cell to perform effector functions upon binding to an Fc domain of an antibody or which binds Fc receptors (FcyR, FcR) with less affinity, thereby reducing antibody directed cytotoxicity (ADCC) effector function as well as clustering of LTβR receptors. Examples of silent IgG1 Fc domains are Fc domains comprising the following mutations LALA (L234A and L235A) and N297A (Strohl, W., 2009, Curr. Opin. Biotechnol. Vol. 20 (6): 685- 691). Another Fc-silencing variant contains the mutations E233P/L234V/L235A/DG236/D265G/ A327Q/A330S it is termed "HS-HC" or "HS" herein (cf. EP2794658). Another early approach intended to reduce effector function was to mutate the glycosylation site at N297 with mutations such as N297A, N297Q, and N297G ("aglycosylation"; Bolt et al., Eur J Immunol. 1993 Feb;23(2):403-11). Other examples include P329G/L234A/L235A in human IgG1 ("PG-LALA"; Schlothauer et al., Protein Eng Des Sel 2016 Oct;29(10):457-466).

The term "**effector functions**" refers to those biological activities attributable to the Fc region of an antibody e.g. a bispecific antibody, which vary with the antibody isotype. Examples of antibody effector functions include without limitation: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation. Due to variations in the relevant Fc region of a given antibody or intended alterations in the relevant Fc region of a given antibody different antibodies can elicit different effector functions and/or the same effector function to a different extend.

"**Afucosylated**" antibodies are antibodies e.g. BsAB engineered such that the oligosaccharides in the Fc region of the antibody do not have any fucose sugar units. Glycosylation of an antibody can alter its function. For example, if glycosylation at N297 in the CH2 domain of an IgG is completely eliminated, binding to FcyRs is lost. However, modulation of the specific carbohydrate composition at N297 can have the opposite effect and enhance the ADCC activity of the antibody. In brief, the affinity of an antibody for the activating FcyRs depends on the composition of the N297 N-linked oligosaccharide. There are 32 different possible combinations of oligosaccharides that can occur at this site. Naturally occurring human IgG and those produced by hybridomas or other common expression systems are usually composed of N-acetylglucosamine (GlcNAc) and three mannose residues that form a core carbohydrate. This core is attached to two additional GlcNAc groups to form biantennary branches. The addition of galactose at each branch can occur as well as the terminal addition of sialic acid to these galactose molecules. Fucose is often part of the core GlcNAc. This fucose, through steric hindrance, obstructs the interaction of the antibody with the FcyRIIIA. Thus, elimination of this fucose molecule while maintaining other forms of glycosylation at this site increases the binding of the antibody to the activating FcyRs, enhancing its ability to elicit ADCC and/or ADCP (Almagro 2017, Front Immunol. 2017; 8: 1751). Methods of preparing fucose-less antibodies include growth in rat myeloma YB2/0 cells (ATCC CRL 1662). YB2/0 cells express low levels of FUT8 mRNA, which encodes α-1,6-fucosyltransferase, an enzyme necessary for fucosylation of polypeptides. Afucosylated antibodies are preferred embodiments of what is described herein.

"**C1q binding and complement dependent cytotoxicity**" also "**Complement-dependent cytotoxicity**" ("**CDC**") is an effector function of IgG and IgM (bispecific) antibodies. When they are bound to a surface antigen on a target cell, the classical complement pathway is triggered by bonding protein C1q to these antibodies, resulting in formation of a membrane attack complex (MAC) and target cell lysis. Complement system is efficiently activated by human IgG1, IgG3 and IgM antibodies, weakly by IgG2 antibodies and is not activated by IgG4 antibodies. Several laboratory methods exist for determining the efficacy of CDC and are known in the art.

An antibody "**inducing CDC**" is an antibody which may elicit a substantial amount of formation of a membrane attack complex and lysis of target cells. Preferably, the CDC induction results in the lysis of at least 2 %, 5 %, 10 %, more preferably at least 15 %, 20 %, 25 %, 30 %, 35 %, 40 %, 45 %, most preferably at least 50 %, 55 %, 60 %, 65 %, 70 %, 75 %, 80 %, 85 %, 90 %, 95 % or 99 % of the target cells.

A "**modification promoting the association of the first and the second subunit of the Fc domain**" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable. Thus, (hetero)dimerization occurs between a polypeptide comprising the first Fc domain subunit and a polypeptide comprising the second Fc domain subunit, which might be non-identical, e.g. in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain. According to what is described herein, antibodies comprising an Fc region may or may not comprise a modification promoting the association of the first and the second subunit of the Fc domain.

### Antibody formats

An antibody may be monoclonal or polyclonal. The term "**polyclonal**" refers to antibodies that are heterogenous populations of antibodies, derived for example from the sera of animals immunized with an antigen or an antigenic functional derivative thereof. For the production of polyclonal immunoglobulins, one or more of various host animals may be immunized by injection with the antigen. Various adjuvants may be used to increase the immunological response, depending on the host species.

**"Monoclonal antibodies"** are substantially homogenous populations of antibodies binding a particular antigen. Monoclonal antibodies may be obtained by methods well known to those skilled in the art (see for example, Köhler et al., Nature (1975) 256, 495-497, and U.S. Patent No. 4,376,110). An antibody or fragment with specific binding affinity can be isolated, enriched, or purified from a prokaryotic or eukaryotic organism. The antibodies according to what is described herein are preferably monoclonal.

**"Humanized antibodies"** contain CDR regions derived from a non human species, such as mouse, that have, for example, been engrafted, along with any necessary framework back-mutations, into human sequence-derived V regions. Thus, for the most part, humanized antibodies are human immunoglobulins (recipient antibody) in which residues from a hypervariable region of the recipient are replaced by residues from a hypervariable region of a non-human species (donor antibody) such as mouse, rat, rabbit or non human primate having the desired specificity, affinity, and capacity. See, for example, U.S. Pat. Nos. 5,225,539; 5,585,089; 5,693,761; 5,693,762; 5,859,205, each herein incorporated by reference. In some instances, framework residues of the human immunoglobulin are replaced by corresponding non-human residues (see, for example, U.S. Pat. Nos. 5,585,089; 5,693,761; 5,693,762, each herein incorporated by reference). Furthermore, humanized antibodies may comprise residues that are not found in the recipient antibody or in the donor antibody. These modifications are made to further refine antibody performance (e.g., to obtain the desired affinity). In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. For further details see Jones et al., Nature 331:522-25 (1986); Riechmann et al., Nature 332:323-27 (1988); and Presta, Curr. Opin. Struct. Biol. 2:593-96 (1992), each incorporated herein by reference.

**Fully human antibodies (human antibodies)** comprise human derived CDRs, i.e. CDRs of human origin. Preferably, a fully human antibody according to what is described herein is an antibody having at least 90 %, 91 %, 92 %, 93 %, 94 %, 95 %, 96 %, 97 %, 98 %, 99 %, 99.5 % or 100 % sequence identity with the closest human VH germline gene (e.g. sequence extracted from recommended list and analyzed in IMGT/Domain-gap-align).

As accepted by usual nomenclature systems such as the INN species subsystem in force until 2017, fully human antibodies may comprise a low number of germline deviations compared with the closest human germline reference determined based on the IMGT database (http://www.imgt.org). For example, a fully human antibody according to what is described herein may comprise up to 1, 2, 3, 4 or 5 germline deviations per CDR compared with the closest human germline reference. Fully human antibodies can be developed from human derived B cells by cloning techniques in combination with a cell enrichment or immortalization step. The majority of fully human antibodies in clinical use, however, were isolated either from immunized mice transgenic for the human IgG locus or from sophisticated combinatorial libraries by phage display (Brüggemann M., Osborn M.J., Ma B., Hayre J., Avis S., Lundstrom B. and Buelow R., Human Antibody Production in Transgenic Animals, Arch Immunol Ther Exp (Warsz.) 63 (2015), 101-108; Carter P.J., Potent antibody therapeutics by design, Nat Rev Immunol 6 (2006), 343-357; Frenzel A., Schirrmann T. and Hust M., Phage display-derived human antibodies in clinical development and therapy, MAbs 8 (2016), 1177-1194; Nelson A.L., Dhimolea E. and Reichert J.M., Development trends for human monoclonal antibody therapeutics, Nat Rev Drug Discov 9 (2010), 767-774.)).

Several techniques are available to generate fully human antibodies or to generate antibodies comprising human derived CDRs (cf. WO2008/112640 A3). Cambridge Antibody Technologies (CAT) and Dyax have obtained antibody cDNA sequences from peripheral B cells isolated from immunized humans and devised phage display libraries for the identification of human variable region sequences of a particular specificity. Briefly, the antibody variable region sequences are fused either with the Gene III or Gene VIII structure of the M13 bacteriophage. These antibody variable region sequences are expressed either as Fab or single chain Fv (scFv) structures at the tip of the phage carrying the respective sequences. Through rounds of a panning process using different levels of antigen binding conditions (stringencies), phages expressing Fab or scFv structures that are specific for the antigen of interest can be selected and isolated. The antibody variable region cDNA sequences of selected phages can then be elucidated using standard sequencing procedures. These sequences may then be used for the reconstruction of a full antibody having the desired isotype using established antibody engineering techniques. Antibodies constructed in accordance with this method are considered fully human antibodies (including the CDRs). In order to improve the immunoreactivity (antigen binding affinity and specificity) of the selected antibody, an in vitro maturation process can be performed, including a combinatorial association of different heavy and light chains, deletion/addition/mutation at the CDR3 of the heavy and light chains (to mimic V-J, and V-D-J recombination), and introduction of random mutations (to mimic somatic hypermutation). An example of a "fully human" antibody generated by this method is the anti-tumor necrosis factor α antibody, Humira (adalimumab).

"**Derivatized antibodies**" are typically modified by glycosylation, acetylation, pegylation, phosphorylation, sulfation, amidation, derivatization by known protecting/blocking groups, proteolytic cleavage, linkage to a cellular ligand or other protein. Any of numerous chemical modifications may be carried out by known techniques, including, but not limited to, specific chemical cleavage, acetylation, formylation, metabolic synthesis of tunicamycin, etc. Additionally, the derivative may contain one or more non-natural amino acids, e.g., using ambrx technology. See, e.g., Wolfson, 2006, Chem. Biol. 13(10): 1011-2. Antibodies according to what is described herein may be derivatized, e.g. sulfated.

The term **"maturated antibodies"** or **"maturated antigen-binding fragments"** such as maturated Fab variants or **"optimized"** variants includes without limitation derivatives of an antibody or antibody fragment exhibiting stronger binding - i. e. binding with increased affinity - to a given antigen such as the extracellular domain of a target protein. Maturation is the process of identifying a small number of mutations within the six CDRs of an antibody or antibody fragment leading to this affinity increase. The maturation process is the combination of molecular biology methods for introduction of mutations into the antibody and screening for identifying the improved binders.

The term **"germlining"** refers to replacement of unusual residues in the framework residues of an antibody with those present in the pre-mutated germline genes to reduce the potential for immunogenicity.

### Therapy

"**Treating**" a disease in a subject or "treating" a subject having a disease refers to subjecting the subject to a pharmaceutical treatment, e.g., the administration of a drug, such that at least one symptom of the disease is decreased or prevented from worsening.

The terms "**prevent**", "**preventing**", "**prevention**" and the like refer to reducing the probability of developing a disease, disorder, or condition in a subject, who does not have, but is at risk of or susceptible to developing a disease, disorder, or condition.

The term "**effective amount**" or "**therapeutically effective amount**" are used interchangeably herein and refer to an amount sufficient to achieve a particular biological result or to modulate or ameliorate a symptom in a subject, or the time of onset of a symptom, typically by at least about 10%; usually by at least about 20%, preferably at least about 30%, or more preferably at least about 50%. Efficacy of the use of an antibody in cancer therapy can be assessed based on the change in tumor burden. Both tumor shrinkage (objective response) and time to the development of disease progression are important endpoints in cancer clinical trials. Standardized response criteria, known as RECIST (Response Evaluation Criteria in Solid Tumors), were published in 2000. An update (RECIST 1.1) was released in 2009. RECIST criteria are typically used in clinical trials where objective response is the primary study endpoint, as well as in trials where assessment of stable disease, tumor progression or time to progression analyses are undertaken because these outcome measures are based on an assessment of anatomical tumor burden and its change over the course of the trial. An effective amount for a particular subject may vary depending on factors such as the condition being treated, the overall health of the subject, the method, route, and dose of administration and the severity of side effects. When in combination, an effective amount is in ratio to a combination of components and the effect is not limited to individual components alone.

If not defined otherwise, "**Complete Response**" (CR) is defined as disappearance of all target lesions. Any pathological lymph nodes (whether target or non-target) must have reduction in short axis to <10 mm. For "**Partial Response**" (PR) at least a 30% decrease in the sum of diameters of target lesions has to be reached, taking as reference the baseline sum diameters. For "Progressive Disease" (PD) at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. In "Stable Disease" (SD) neither sufficient shrinkage to qualify for PR nor sufficient increase to qualify for PD is observed, taking as reference the smallest sum diameters while on study.

Typical "**subjects**" according to what is described herein include human and non-human subjects. Subjects can be mammals such as mice, rats, cats, dogs, primates and/or humans.

"**Pharmaceutical compositions**" (also "therapeutic formulations") of the antibody, fragment or conjugate can be prepared by mixing the antibody having the desired degree of purity with optional physiologically acceptable carriers, excipients or stabilizers, e.g. according to Remington's Pharmaceutical Sciences (18th ed.; Mack Pub. Co.: Eaton, Pa., 1990), e.g. in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as Tween^{®}, Pluronic^{®} or polyethylene glycol (PEG).

A "**fixed combination**" in the present description is used as known to persons skilled in the art and is defined as a combination wherein, for example, a first active ingredient according to the present description and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "**kit-of-parts**" in the present description is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

### EMBODIMENTS

### Bispecific Antibody (First Aspect)

### General Tetravalent Bispecific Antibody

As described in examples 4 and 5 the targeted isolated tetravalent bispecific antibody (BsAB) - comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain was binding to - described herein induced stimulation of mouse CXCL9 or human CXCL10 chemokines when used as targeted antibody. Thus the targeted isolated tetravalent bispecific antibody were agonists of LTβR signaling. As defined above a **"targeted LTβR antibody**" refers to the isolated tetravalent bispecific antibody wherein the antibody comprises two targeting moieties e.g. antigen binding domains for a site where the intended effect of LTβR signaling pathway induction and/or stimulation is to be exerted. In examples 4 and 6 the isolated tetravalent bispecific antibodies were targeted to LRRC15 while in example 5 the isolated tetravalent bispecific antibodies were targeted to Mesothelin. In example 4 the targeted isolated tetravalent bispecific antibodies described herein induced more stimulation of mouse CXCL9 or human CXCL10 chemokines than the untargeted control antibodies comprising identical first and seconds antigen binding domain capable of binding to LTβR. The induction of the chemokines by the targeted isolated bispecific antibodies also occurred at a lower EC50 value in example 4 compared to the induction by the non-targeted LTβR antibody. In example 6 the targeted LTβR antibodies showed better anti-tumor efficacy than the non-targeted LTβR or control antibodies. Hence the first and second antigen binding domains capable of binding to LTβR of the isolated tetravalent bispecific antibody of the first aspect stimulate the LTβR signaling pathway more and show better anti-tumor efficacy when comprised in a targeted LTβR antibody. Accordingly in a first aspect, what is described herein relates to an isolated tetravalent bispecific antibody (BsAB) comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain is capable of binding to. In contrast to known bispecific agnostic LTβR antibodies these antibodies of the first aspect are tetravalent and hence have a higher avidity but nevertheless induce stimulation of the LTβR pathway to a lesser extend if used as non-targeted LTβR antibody.

In some preferred embodiments of the first aspect the isolated tetravalent bispecific antibody comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain is capable of binding to, wherein the isolated tetravalent bispecific antibody is a targeted antibody. It is preferred that in this isolated tetravalent bispecific antibody comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain is capable of binding to, wherein the isolated tetravalent bispecific antibody is a targeted antibody the third and fourth antigen binding domains are capable of binding to the same antigen other than LTβR. It is further preferred that in this isolated tetravalent bispecific antibody comprising a first antigen binding domain capable of binding to LTβR and a second antigen binding domain capable of binding to LTβR are identical and that a third antigen binding domain capable of binding to an antigen other than LTβR and a fourth antigen binding domain capable of binding to the same antigen other than LTβR to which the third antigen binding domain is capable of binding to are also identical.

In some preferred embodiments of the first aspect the third and fourth antigen binding domains of the isolated tetravalent bispecific antibody are capable of binding to an antigen associated with a tumor and/or a cancer. In some of these preferred embodiments of first aspect the third and fourth antigen binding domains of the isolated tetravalent bispecific antibody are capable of binding to an antigen associated with a cancer. In some of these preferred embodiments of first aspect the third and fourth antigen binding domains of the isolated tetravalent bispecific antibody are capable of binding to an antigen present on a tumor cell and/or a cancer cell and/or a cell associated with a cancer cell and/or an antigen present in the extracellular tumor or cancer environment. It is especially preferred that the third and fourth antigen binding domains of the isolated tetravalent bispecific antibody are capable of binding to an antigen associated with a cancer cell and that this cancer cell is part of a tumor

Thus, it is highly preferred that the isolated tetravalent bispecific antibody comprising a first antigen binding domain capable of binding to LTβR and a second antigen binding domain capable of binding to LTβR which are identical and a third antigen binding domain capable of binding to an antigen other than LTβR and a fourth antigen binding domain capable of binding to the same antigen other than LTβR to which the third antigen binding domain is capable of binding to which are also identical and that the third and fourth binding domains are capable of binding to an antigen associated with a tumor.

In the most preferred embodiment the isolated tetravalent bispecific antibody comprising a first antigen binding domain capable of binding to LTβR and a second antigen binding domain capable of binding to LTβR which are identical and a third antigen binding domain capable of binding to an antigen other than LTβR and a fourth antigen binding domain capable of binding to the same antigen other than LTβR to which the third antigen binding domain is capable of binding are also identical and that the third and fourth binding domains are capable of binding to LRRC15.

In some preferred embodiments, the tetravalent bispecific antibody according to the first aspect or at least a portion thereof is monoclonal. In some embodiments, the tetravalent bispecific antibody is chimeric. In some preferred embodiments, the tetravalent bispecific antibody is fully or partially humanized. In some embodiments according to the first aspect, the format of the tetravalent bispecific antibody is IgG-like. In some embodiments according to the first aspect, the isolated tetravalent bispecific antibody comprises at least two scFv fragments, which each include a heavy chain variable region and a light chain variable region, linked together by a scFv linker. As discussed, for this format, a number of suitable scFv linkers is available, including traditional peptide bonds, generated by recombinant techniques (Huston et al. (1988) Proc. Natl. Acad. Sci USA 85:5879-5883). In this embodiment the isolated tetravalent bispecific antibody preferably either comprises the two scFv fragments as part of the heavy chain (cf. Fig. 1) or as part of the light chain or one of the at least two scFV fragments can be part of the heavy chain whereas the other of the at least two scFV fragments can be part of the light chain.

It is preferred that each of the two scFv LTβR fragments of the isolated tetravalent bispecific antibody of the first aspect comprises the linker sequence GGGGSGGGGSGGGGSGGGGS (SEQ ID NO. 28). In some embodiments according to the first aspect, the tetravalent bispecific antibody comprises at least one linker, linking the at least two scFV fragments to the IgG like part of the tetravalent bispecific antibody . In some embodiments, the at least one linker linking the at least two scFV fragments to the IgG part comprises at least one peptide linker. In some embodiments, the at least one peptide linker is from 1 to 50 amino acids in length, preferably 1 to 30 amino acids in length, most preferably 4 to 16 amino acids in length. In some embodiments, the at least one peptide linker has a size of 4, 5, 6, 7, 8, 9, 10, 11, 12, 13, 14, 15 or 16 amino acids. In some embodiments, the at least one peptide linker has a size of 4, 3, 2, or one amino acid. In a preferred embodiment, the at least one peptide linker is rich of glycine. In one embodiment, the at least one peptide linker consists of a single glycine. In a preferred embodiment, the one or more linker does not promote secondary structures. In a preferred embodiment, the BsAB comprises a GGGGSnG peptide linker, wherein n is an integer selected from the list consisting of 1, 2, 3, 4, 5, 6, 7, 8, 9 and 10. In a preferred embodiment, the isolated tetravalent bispecific antibody described herein comprises at least one (GGGGS)3G peptide linker, which links an scFv fragment to a heavy chain of an IgG like part. In a highly preferred embodiment the isolated tetravalent bispecific antibody described herein comprises two GGGGGSGGSGGSGGGGG (TPP-35001, SEQ ID NO. 27) peptide linkers, which each link one scFv fragment to a heavy chain of an IgG like part.

In some embodiments the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG1 molecule. It is preferred that in such an embodiment the Fc part of the IgG1 is or comprises an silent Fc domain.

In some embodiments the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG4 molecule. It is preferred that in such an embodiment the Fc part of the IgG4 is or comprises a silent Fc domain.

In preferred embodiments the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG1 molecule, wherein the scFv fragments are each linked to the constant region of the heavy chain via one (GGGGS)3 linker peptide. It is preferred that in such an embodiment the Fc part of the IgG1 is or comprises a silent Fc domain.

In preferred embodiments the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG4 molecule, wherein the scFv fragments are each linked to the constant region of the heavy chain via one (GGGGS)3 linker peptide. It is preferred that in such an embodiment the Fc part of the IgG4 is or comprises a silent Fc domain.

In a highly preferred embodiment the isolated tetravalent bispecific antibody of the first aspect consists a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG1 molecule, wherein the scFv fragments are each linked to the constant region of the light chain via one (GGGGS)3 linker peptide. It is preferred that in such an embodiment the Fc part of the IgG1 is or comprises asilent Fc domain.

In a preferred embodiment the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG4 molecule, wherein the scFv fragments are each linked to the constant region of the light chain via one (GGGGS)3 linker peptide.

In a most preferred embodiments the isolated tetravalent bispecific antibody of the first aspect comprises a first antigen binding domain capable of binding to LTβR and a second antigen binding domain capable of binding to LTβR which are identical as scFv fragments and a third antigen binding domain capable of binding to an antigen other than LTβR and a fourth antigen binding domain capable of binding to the same antigen other than LTβR to which the third antigen binding domain is capable of binding to which are also identical and part of an IgG1 molecule, wherein the scFv fragments are each linked to the constant region of the light chain via one (GGGGS)3 linker peptide wherein the Fc part of the IgG1 is or comprises an silent Fc domain.

In preferred embodiments of the first aspect described herein the Fc part of the molecule comprises a silent Fc domain i.e. silencing mutations in the Fc-part of the antibody. Such silencing mutations in the Fc-part of the antibody are for e.g., but not limited to E233P, L234V, L235A, ΔG236, D265G, A327Q, or A330S (EP2794658). Further Fc engineering examples include human IgG4 variants L235E or F234A/L235A and the human IgG1 variant L234A/L235A ("LALA"; Xu et al., Cell Immunol 2000 Feb 25;200(1):16-26). Another early approach intended to reduce effector function was to mutate the glycosylation site at N297 with mutations such as N297A, N297Q, and N297G ("aglycosylation"; Bolt et al., Eur J Immunol. 1993 Feb;23(2):403-11). Another variation is a cross-subclass approach to reduce effector function as exemplified by the approved anti-C5 therapeutic eculizumab, which carries CH1 and hinger region from IgG2 but carries CH2 and CH3 from IgG4. Other examples include P329G/L234A/L235A in human IgG1 ("PG-LALA"; Schlothauer et al., Protein Eng Des Sel 2016 Oct;29(10):457-466), "IgG1sigma" (L234A/L235A/G237A/P238S/H268A/A330S/P331S, Tam et al., Antibodies (Basel) 2017 Sep 1;6(3):12 i.e. all antibodies carrying the term "sigma" in their name comprise these mutations), "IgG1-NNAS" (S298N/T299A/Y300S, Zhou et al., MAbs Jan-Dec 2020;12(1):1814583) (numbering according to Eu nomenclature; Edelman et al., Proc Natl Acad Sci USA. 1969 May; 63(1): 78-85; Kabat et al., 1991, Sequences of Proteins of Immunological Interest, 5th Edition. U.S. Department of Health and Human Services, Public Health Service, National Institutes of Health, NIH Publication No. 91-3242). Most preferably isolated tetravalent bispecific of the first aspect comprises two identical FC parts wherein each FC part comprises the silencing mutations are E233P, L234V, L235A, AG236, D265G, A327Q, and A330S (EP2794658). For illustration purposes only these mutations are given in the heavy chain sequence of TPP-32970 (SEQ ID NO. 19) as bold amino acids in the LRRC15-IgG1 part (not underlined part) of the molecule. Hence with respect to the numbering of TPP-32970 ( SEQ ID NO. 19) the mutations are E235P, L236V, L237A, AG238, D266G, A328Q, and A331S and it should be noted that "AG238" is an deletion and hence not shown.

### LTβR part of the Tetravalent Bispecific Antibody

As already detailed above what is described herein relates to an isolated tetravalent bispecific antibody (BsAB) comprising a first antigen binding domain capable of binding to LTβR and a second antigen binding domain capable of binding to LTβR and a third antigen binding domain capable of binding to an antigen other than LTβR and a fourth antigen binding domain capable of binding to the same antigen other than LTβR to which the third antigen binding domain is capable of binding to. In highly preferred embodiments the first antigen binding domain capable of binding LTβR and the second antigen binding domain capable of binding LTβR of the isolated tetravalent bispecific antibody described herein are identical.

In some preferred embodiments A of the first aspect the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of
a) SEQ ID NO. 69, SEQ ID NO. 70, SEQ ID NO. 71, SEQ ID NO. 72:, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 44 (TPP-34999), or
e) SEQ ID NO. 49, SEQ ID NO. 50, SEQ ID NO. 51, SEQ ID NO. 52, SEQ ID NO. 53 and SEQ ID NO. 54 (TPP-35000).

**In some preferred embodiments A of the first aspect** the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of SEQ ID NO. 69, SEQ ID NO. 70, SEQ ID NO. 71, SEQ ID NO. 72, SEQ ID NO. 73 and SEQ ID NO. 74 (TPP-34849). **In some preferred embodiments A of the first aspect** the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of SEQ ID NO. 59, SEQ ID NO. 60, SEQ ID NO. 61, SEQ ID NO. 62, SEQ ID NO. 63, and SEQ ID NO. 64 (TPP-34848). **In some preferred embodiments A of the first aspect** the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of SEQ ID NO. 29, SEQ ID NO. 30, SEQ ID NO. 31, SEQ ID NO. 32:, SEQ ID NO. 33 and SEQ ID NO 34 (TPP-34847). **In some preferred embodiments A of the first aspect** the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of SEQ ID NO. 39, SEQ ID NO. 40, SEQ ID NO. 41, SEQ ID NO. 42, SEQ ID NO. 43 and SEQ ID NO. 44 (TPP-34999). **In some preferred embodiments A of the first aspect** the first and second antigen binding domains of the isolated tetravalent bispecific antibody capable of binding LTβR comprise at least one, two, three, four, five and preferably six CDR sequences having at least 90 %, 95 %, 98 %, 99 % or 100 % sequence identity with at least one of SEQ ID NO. 49, SEQ ID NO. 50, SEQ ID NO. 51, SEQ ID NO. 52, SEQ ID NO. 53 and SEQ ID NO. 54 (TPP-35000).

**In some preferred embodiments A of the first aspect** the first and second antigen binding domains of the isolated tetravalent bispecific antibody capable of binding LTβR comprise six CDR sequences wherein at least one, two, three, four, five or six CDR sequences have one or two single amino acid exchange as compared to
a) SEQ ID NO. 69, SEQ ID NO. 70, SEQ ID NO. 71, SEQ ID NO. 72:, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32:, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 44 (TPP-34999), or
e) SEQ ID NO. 49, SEQ ID NO. 50, SEQ ID NO. 51, SEQ ID NO. 52, SEQ ID NO. 53 and SEQ ID NO. 54 (TPP-35000).

**In some preferred embodiments A of the first aspect** the first and second antigen binding domains of the isolated tetravalent bispecific antibody capable of binding LTβR are identical antigen-binding scFv fragments comprising at least one, two, three, four, five and preferably six CDR sequences having at least 90 %, 95 %, 98 %, 99 % or 100 % sequence identity with at least one of
a) SEQ ID NO. 69, SEQ ID NO. 70, SEQ ID NO. 71, SEQ ID NO. 72:, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32:, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 44 (TPP-34999), or
e) SEQ ID NO. 49, SEQ ID NO. 50, SEQ ID NO. 51, SEQ ID NO. 52, SEQ ID NO. 53 and SEQ ID NO. 54 (TPP-35000).

**In some highly preferred embodiments A of the first aspect** the first and second antigen binding domains of the isolated tetravalent bispecific antibody capable of binding/binding LTβR comprise
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848), or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000).

**In some highly preferred embodiments A of the first aspect** the first and second antigen binding domains of the isolated tetravalent bispecific antibody capable of binding/binding LTβR comprise a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849). **In some highly preferred embodiments A of the first aspect** the first and second antigen binding domains of the isolated tetravalent bispecific antibody capable of binding/binding LTβR comprise a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848). **In some highly preferred embodiments A of the first aspect** the first and second antigen binding domains of the isolated tetravalent bispecific antibody capable of binding/binding LTβR comprise a heavy chain variable region comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847). **In some highly preferred embodiments A of the first aspect** the first and second antigen binding domains of the isolated tetravalent bispecific antibody capable of binding/binding LTβR comprise a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999). **In some highly preferred embodiments A of the first aspect** the first and second antigen binding domains of the isolated tetravalent bispecific antibody capable of binding/binding LTβR comprise a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

**In some highly preferred embodiments A of the first aspect** the first and second antigen binding domains of the isolated tetravalent bispecific antibody capable of binding/ binding LTβR comprise
a) a heavy chain variable region having at least 90 %, 95 %, 98 %, 99 % or 100 % sequence identity with SEQ ID NO. 75 and in a light chain variable region having at least 90 %, 95 %, 98 %, 99 % or 100 % sequence identity with SEQ ID NO. 76 (TPP-34849) or
b) a heavy chain variable region having at least 90 %, 95 %, 98 %, 99 % or 100 % sequence identity with SEQ ID NO. 65 and in a light chain variable region having at least 90 %, 95 %, 98 %, 99 % or 100 % sequence identity with SEQ ID NO. 66 (TPP-34848) or
c) a heavy chain variable region having at least 90 %, 95 %, 98 %, 99 % or 100 % sequence identity with SEQ ID NO. 35 and in a light chain variable region having at least 90 %, 95 %, 98 %, 99 % or 100 % sequence identity with SEQ ID NO. 36 (TPP-34847) or
d) a heavy chain variable region having at least 90 %, 95 %, 98 %, 99 % or 100 % sequence identity with SEQ ID NO. 45 and in a light chain variable region having at least 90 %, 95 %, 98 %, 99 % or 100 % sequence identity with SEQ ID NO. 46 (TPP-34999) or
e) a heavy chain variable region having at least 90 %, 95 %, 98 %, 99 % or 100 % sequence identity with SEQ ID NO. 55 and in a light chain variable region having at least 90 %, 95 %, 98 %, 99 % or 100 % sequence identity with SEQ ID NO. 56 (TPP-35000).

**In some highly preferred embodiments A of the first aspect** the first and second antigen binding domains of the isolated tetravalent bispecific antibody capable of binding/ binding LTβR comprise a heavy chain variable region having at least 90 %, 95 %, 98 %, 99 % or 100 % sequence identity with SEQ ID NO. 75 and in a light chain variable region having at least 90 %, 95 %, 98 %, 99 % or 100 % sequence identity with SEQ ID NO. 76 (TPP-34849). **In some highly preferred embodiments A of the first aspect** the first and second antigen binding domains of the isolated tetravalent bispecific antibody capable of binding/ binding LTβR comprise a heavy chain variable region having at least 90 %, 95 %, 98 %, 99 % or 100 % sequence identity with SEQ ID NO. 65 and in a light chain variable region having at least 90 %, 95 %, 98 %, 99 % or 100 % sequence identity with SEQ ID NO. 66 (TPP-34848). **In some highly preferred embodiments A of the first aspect** the first and second antigen binding domains of the isolated tetravalent bispecific antibody capable of binding/ binding LTβR comprise a heavy chain variable region having at least 90 %, 95 %, 98 %, 99 % or 100 % sequence identity with SEQ ID NO. 35 and in a light chain variable region having at least 90 %, 95 %, 98 %, 99 % or 100 % sequence identity with SEQ ID NO. 36 (TPP-34847). **In some highly preferred embodiments A of the first aspect** the first and second antigen binding domains of the isolated tetravalent bispecific antibody capable of binding/ binding LTβR comprise a heavy chain variable region having at least 90 %, 95 %, 98 %, 99 % or 100 % sequence identity with SEQ ID NO. 45 and in a light chain variable region having at least 90 %, 95 %, 98 %, 99 % or 100 % sequence identity with SEQ ID NO. 46 (TPP-34999). **In some highly preferred embodiments A of the first aspect** the first and second antigen binding domains of the isolated tetravalent bispecific antibody capable of binding/ binding LTβR comprise a heavy chain variable region having at least 90 %, 95 %, 98 %, 99 % or 100 % sequence identity with SEQ ID NO. 55 and in a light chain variable region having at least 90 %, 95 %, 98 %, 99 % or 100 % sequence identity with SEQ ID NO. 56 (TPP-35000).

It should be noted that with respect to the heavy chain of TPP-29168 (SEQ ID NO. 5) the heavy chain variable region and the light chain variable region of the antigen binding domain capable of binding/ binding LTβR are underlined in Table 1. As TPP-29168 comprises the scFv TPP-34999 the underlined parts correspond to SEQ ID NO. 45 and SEQ ID NO. 46 respectively. It should be noted that with respect to the heavy chain of TPP-23518 (SEQ ID NO. 7) and heavy chain of TPP-29448 (SEQ ID NO. 9) the heavy chain variable region and the light chain variable region of the antigen binding domain capable of binding/ binding LTβR are underlined in Table 1. As TPP-23518 and TPP-29448 comprises the scFv TPP-34847 the underlined parts correspond to SEQ ID NO. 35 and SEQ ID NO. 36 respectively. It should be noted that with respect to the heavy chain of TPP-29570 (SEQ ID NO. 11) the heavy chain variable region and the light chain variable region of the antigen binding domain capable of binding/ binding LTβR are underlined in Table 1. As TPP-29570 comprises the scFv TPP-34848 the underlined parts correspond to SEQ ID NO. 65 and SEQ ID NO. 66 respectively. It should be noted that with respect to the heavy chain of TPP-32970 (SEQ ID NO. 19) the heavy chain variable region and the light chain variable region of the antigen binding domain capable of binding/ binding LTβR are underlined in Table 1. As TPP-32970 comprises the scFv TPP-34849 the underlined parts correspond to SEQ ID NO. 75 and SEQ ID NO. 76 respectively.

**In some highly preferred embodiments A of the first aspect** the first and second antigen binding domains of the isolated tetravalent bispecific antibody capable of binding LTβR comprise
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and wherein at least one, two, three, four, five or six of these CDR sequences have one or two single amino acid exchanges, or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID. NO 64 (TPP-34848) wherein at least one, two, three, four, five or six of these CDR sequences have one or two single amino acid exchanges, or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), wherein at least one, two, three, four, five or six of these CDR sequences have one or two single amino acid exchanges, or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999) wherein at least one, two, three, four, five or six of these CDR sequences have one or two single amino acid exchanges, or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000) wherein at least one, two, three, four, five or six of these CDR sequences have one or two single amino acid exchanges.

**In some highly preferred embodiments A of the first aspect** the first and second antigen binding domains of the isolated tetravalent bispecific antibody capable of binding LTβR are identical antigen-binding scFv fragments each comprising
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 63, a light chain complementarity determining region (LCDR2) comprising the amino acid sequence of SEQ ID NO. 64 and a light chain complementarity determining region (LCDR3) comprising the amino acid sequence of SEQ ID NO. 65 (TPP-34848) or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847), or
d) d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

**In some highly preferred embodiments A of the first aspect** the first and second antigen binding domains of the isolated tetravalent bispecific antibody capable of binding LTβR are identical antigen-binding scFv fragments each comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849). **In some highly preferred embodiments A of the first aspect** the first and second antigen binding domains of the isolated tetravalent bispecific antibody capable of binding LTβR are identical antigen-binding scFv fragments each comprising a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848). **In some highly preferred embodiments A of the first aspect** the first and second antigen binding domains of the isolated tetravalent bispecific antibody capable of binding LTβR are identical antigen-binding scFv fragments each comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847). **In some highly preferred embodiments A of the first aspect** the first and second antigen binding domains of the isolated tetravalent bispecific antibody capable of binding LTβR are identical antigen-binding scFv fragments each comprising a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999). **In some highly preferred embodiments A of the first aspect** the first and second antigen binding domains of the isolated tetravalent bispecific antibody capable of binding LTβR are identical antigen-binding scFv fragments each comprising a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

**In some highly preferred embodiments A of the first aspect** the first and second antigen binding domains of the isolated tetravalent bispecific antibody capable of binding LTβR are identical antigen-binding scFv fragments each comprising
a) as heavy chain variable region SEQ ID NO. 75 comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and as light chain variable region SEQ ID NO. 76 comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) as heavy chain variable region SEQ ID NO. 65 comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and as light chain variable region SEQ ID NO. 66 comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848) or
c) as heavy chain variable region SEQ ID NO. 35 comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and as light chain variable region SEQ ID NO. 36 comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847), or
d) as heavy chain variable region SEQ ID NO. 45 comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and as light chain variable region SEQ ID NO. 46 comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999), or
e) as heavy chain variable region SEQ ID NO. 55 comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and as light chain variable region SEQ ID NO. 56 comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

**In some highly preferred embodiments A of the first aspect** the first and second antigen binding domains of the isolated tetravalent bispecific antibody capable of binding LTβR are identical antigen-binding scFv fragments each comprising as heavy chain variable region SEQ ID NO. 75 comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and as light chain variable region SEQ ID NO. 76 comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849). **In some highly preferred embodiments A of the first aspect** the first and second antigen binding domains of the isolated tetravalent bispecific antibody capable of binding LTβR are identical antigen-binding scFv fragments each comprising as heavy chain variable region SEQ ID NO. 65 comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and as light chain variable region SEQ ID NO. 66 comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848). **In some highly preferred embodiments A of the first aspect** the first and second antigen binding domains of the isolated tetravalent bispecific antibody capable of binding LTβR are identical antigen-binding scFv fragments each comprising as heavy chain variable region SEQ ID NO. 35 comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and as light chain variable region SEQ ID NO. 36 comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847). **In some highly preferred embodiments A of the first aspect** the first and second antigen binding domains of the isolated tetravalent bispecific antibody capable of binding LTβR are identical antigen-binding scFv fragments each comprising as heavy chain variable region SEQ ID NO. 45 comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and as light chain variable region SEQ ID NO. 46 comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999). **In some highly preferred embodiments A of the first aspect** the first and second antigen binding domains of the isolated tetravalent bispecific antibody capable of binding LTβR are identical antigen-binding scFv fragments each comprising as heavy chain variable region SEQ ID NO. 55 comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and as light chain variable region SEQ ID NO. 56 comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

**In some highly preferred embodiments B of the first aspect** the first and second antigen binding domains of the isolated tetravalent bispecific antibody capable of binding LTβR
a) have at least 90 %, 95 %, 98 %, 99 % or 100 % sequence identity with SEQ ID NO. 77 (TPP-34849),
b) have at least 90 %, 95 %, 98 %, 99 % or 100 % sequence identity with SEQ ID NO. 67 (TPP-34848),
c) have at least 90 %, 95 %, 98 %, 99 % or 100 % sequence identity with SEQ ID NO. 37 (TPP-34847),
d) have at least 90 %, 95 %, 98 %, 99 % or 100 % sequence identity with SEQ ID NO. 47 (TPP-34999),
e) have at least 90 %, 95 %, 98 %, 99 % or 100 % sequence identity with SEQ ID NO. 57 (TPP-35000).

**In some highly preferred embodiments B of the first aspect** the first and second antigen binding domains of the isolated tetravalent bispecific antibody capable of binding LTβR have at least 90 %, 95 %, 98 %, 99 % or 100 % sequence identity with SEQ ID NO. 77 (TPP-34849). It should be noted that in Table 1 in the line "TPP-34849 scFV PRT" the CDR regions of TPP-34849 are given in **bold** and the linker is given in *italics,* hence the other given amino acids denote the framework regions of TPP-34849.

In **some highly preferred embodiments B of the first aspect** the first and second antigen binding domains of the isolated tetravalent bispecific antibody capable of binding LTβR have at least 90 %, 95 %, 98 %, 99 % or 100 % sequence identity with SEQ ID NO. 67 (TPP-34848). It should be noted that in Table 1 in the line "TPP-34848 scFV PRT" the CDR regions of TPP-34848 are given in **bold** and the linker is given in *italics,* hence the other given amino acids denote the framework regions of TPP-34848.

**In some highly preferred embodiments B of the first aspect** the first and second antigen of the isolated tetravalent bispecific antibody binding domains capable of binding LTβR have at least 90 %, 95 %, 98 %, 99 % or 100 % sequence identity SEQ ID NO. 37 (TPP-34847). It should be noted that in Table 1 in the line "TPP-34847 scFV PRT" the CDR regions of TPP-34847 are given in bold and the linker is given in *italics,* hence the other given amino acids denote the framework regions of TPP-34847. **In some highly preferred embodiments B of the first aspect** the first and second antigen binding domains of the isolated tetravalent bispecific antibody capable of binding LTβR have at least 90 %, 95 %, 98 %, 99 % or 100 % sequence identity with SEQ ID NO. 47 (TPP-34999). It should be noted that in Table 1 in the line "TPP-34999 scFV PRT" the CDR regions of TPP-34999 are given in **bold** and the linker is given in *italics,* hence the other given amino acids denote the framework regions of TPP-34999. **In some highly preferred embodiments B of the first aspect** the first and second antigen binding domains of the isolated tetravalent bispecific antibody capable of binding LTβR have at least 90 %, 95 %, 98 %, 99 % or 100 % sequence identity with SEQ ID NO. 57 (TPP-35000). It should be noted that in Table 1 in the line "TPP-35000 scFV PRT" the CDR regions of TPP-35000are given in **bold** and the linker is given in *italics,* hence the other given amino acids denote the framework regions of TPP-35000.

**In some highly preferred embodiments B of the first aspect** the first and second antigen binding domains of the isolated tetravalent bispecific antibody capable of binding LTβR are identical antigen-binding scFv fragments
a) having at least 90 %, 95 %, 98 %, 99 % or 100 % sequence identity with SEQ ID NO. 77 (TPP-34849),
b) having at least 90 %, 95 %, 98 %, 99 % or 100 % sequence identity with SEQ ID NO. 67 (TPP-34848),
c) having at least 90 %, 95 %, 98 %, 99 % or 100 % sequence identity with SEQ ID NO. 37 (TPP-34847),
d) having at least 90 %, 95 %, 98 %, 99 % or 100 % sequence identity with SEQ ID NO. 47 (TPP-34999),
e) having at least 90 %, 95 %, 98 %, 99 % or 100 % sequence identity with SEQ ID NO. 57 (TPP-35000).

**In some highly preferred embodiments B of the first aspect** the first and second antigen binding domains of the isolated tetravalent bispecific antibody capable of binding LTβR are identical antigen-binding scFv fragments having at least 90 %, 95 %, 98 %, 99 % or 100 % sequence identity with SEQ ID NO. 77 (TPP-34849). **In some highly preferred embodiments B of the first aspect** the first and second antigen binding domains of the isolated tetravalent bispecific antibody capable of binding LTβR are identical antigen-binding scFv fragments having at least 90 %, 95 %, 98 %, 99 % or 100 % sequence identity with SEQ ID NO. 67 (TPP-34848). **In some highly preferred embodiments B of the first aspect** the first and second antigen binding domains of the isolated tetravalent bispecific antibody capable of binding LTβR are identical antigen-binding scFv fragments having at least 90 %, 95 %, 98 %, 99 % or 100 % sequence identity with SEQ ID NO. 37 (TPP-34847). **In some highly preferred embodiments B of the first aspect** the first and second antigen binding domains of the isolated tetravalent bispecific antibody capable of binding LTβR are identical antigen-binding scFv fragments having at least 90 %, 95 %, 98 %, 99 % or 100 % sequence identity with SEQ ID NO. 47 (TPP-34999). **In some highly preferred embodiments B of the first aspect** the first and second antigen binding domains of the isolated tetravalent bispecific antibody capable of binding LTβR are identical antigen-binding scFv fragments having at least 90 %, 95 %, 98 %, 99 % or 100 % sequence identity with SEQ ID NO. 57 (TPP-35000).

### LRRC15 part of the Tetravalent Bispecific Antibody

In some preferred embodiments of the first aspect the third and fourth antigen binding domains of the isolated tetravalent bispecific antibody described herein are capable of binding LRRC15. Hence this isolated tetravalent bispecific LTβR/LRRC15 antibody is an example of a combination of two "targeting moieties binding to (human) LRRC15" and two "targeting moieties binding to (human) LTβR". In some preferred embodiments of the first aspect the isolated tetravalent bispecific antibody described herein targets an LRRC15 expressing cancer cell via the third and fourth binding domains capable of binding to LRRC15 and the cancer cell is selected from the group consisting of lung cancer, non-small cell lung carcinoma, head and neck cancer, head and neck squamous cell carcinoma, sarcoma, glioblastoma, melanoma, breast cancer, HER2 negative breast cancer, HER2 positive breast cancer, triple negative breast cancer, HR+ breast cancer, pancreatic cancer, pancreatic ductal adenocarcinoma or colorectal cancer.In some embodiment the LRRC15 may be from any species e.g. human, monkey, macaca fascicularis (cynomolgus monkey), macaca mulatta (rhesus macaque), rodent, mouse, rat, horse, bovine, pig, dog, cat and camel LRRC15. Preferably, the LRRC15 is human LRRC15 and/or cynomolgus and/or murine LRRC15. While in principle the third and fourth antigen binding domains capable of binding LRRC15 may be selected without limitation from the group consisting of peptides, proteins, antibodies or nanoparticles, polynucleotides, DNA and RNA fragments, aptamers, or small molecules it is herein preferred that the third and fourth antigen binding domains capable of binding LRRC15 are part of an antibody or are fragments of an antibody comprising antigen binding domains binding to human LRRC15, e.g. as described elsewhere herein.

**In some preferred embodiments C of the first aspect** the third and fourth antigen binding domains of the isolated tetravalent bispecific antibody capable of binding LRRC15 comprise at least one, two, three, four, five and preferably six CDR sequences having at least 90 %, 95 %, 98 %, 99 % or 100 % sequence identity with at least one of
a) SEQ ID NO. 122, SEQ ID NO. 123 , SEQ ID NO. 124, SEQ ID NO. 126, SEQ ID NO. 127, and SEQ ID NO. 128 (TPP-17078), or
b) SEQ ID NO. 140, SEQ ID NO. 141, SEQ ID NO. 142, SEQ ID NO. 144, SEQ ID NO. 145, and SEQ ID NO. 146 (TPP-17421).

**In some preferred embodiments C of the first aspect** the third and fourth antigen binding domains of the isolated tetravalent bispecific antibody capable of binding LRRC15 comprise at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078). **In some preferred embodiments C of the first aspect** the third and fourth antigen binding domains of the isolated tetravalent bispecific antibody capable of binding LRRC15 comprise at least one, two, three, four, five and preferably six CDR sequences having at least 90 %, 95 %, 98 %, 99 % or 100 % sequence identity with at least one of SEQ ID NO. 140, SEQ ID NO. 141, SEQ ID NO. 142, SEQ ID NO. 144, SEQ ID NO. 145, and SEQ ID NO. 146 (TPP-17421). **In some preferred embodiments C of the first aspect** the third and fourth antigen binding domains of the isolated tetravalent bispecific antibody capable of binding LRRC15 comprise
a) a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) or
b) a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421).

**In some highly preferred embodiments D of the first aspect** the third and fourth antigen binding domains of the isolated tetravalent bispecific antibody capable of binding LRRC15 are comprised in
a) a heavy chain variable region having at least 90 %, 95 %, 98 %, 99 % or 100 % sequence identity with SEQ ID NO. 121 and/or
   a light chain variable region at least 90 %, 95 %, 98 %, 99 % or 100 % sequence identity with SEQ ID NO. 125 (TPP-17078), or
b) a heavy chain variable region having at least 90 %, 95 %, 98 %, 99 % or 100 % sequence identity with SEQ ID NO. 139 and/or
   a light chain variable having at least 90 %, 95 %, 98 %, 99 % or 100 % sequence identity with SEQ ID NO. 143 (TPP-17421)

**In some highly preferred embodiments D of the first aspect** the third and fourth antigen binding domains of the isolated tetravalent bispecific antibody capable of binding LRRC15 are comprised in a heavy chain variable region chain having at least 90 %, 95 %, 98 %, 99 % or 100 % sequence identity with SEQ ID NO. 121 and/or a light chain variable region having at least 90 %, 95 %, 98 %, 99 % or 100 % sequence identity with SEQ ID NO. 125 (TPP-17078). **In some highly preferred embodiments D of the first aspect** the third and fourth antigen binding domains of the isolated tetravalent bispecific antibody capable of binding LRRC15 are comprised in a variable heavy chain having at least 90 %, 95 %, 98 %, 99 % or 100 % sequence identity with SEQ ID NO. 139 and/or a variable light chain having at least 90 %, 95 %, 98 %, 99 % or 100 % sequence identity with SEQ ID NO. 143 (TPP-17421).

**In some highly preferred embodiments E of the first aspect,** the third and fourth antigen binding domains of the isolated tetravalent bispecific antibody capable of binding LRRC15 are comprised in
a) a heavy chain region having at least 90 %, 95 %, 98 %, 99 % or 100 % sequence identity with SEQ ID NO. 133 and/or
   a light chain region having at least 90 %, 95 %, 98 %, 99 % or 100 % sequence identity with SEQ ID NO. 134 (TPP-14389), or
b) a heavy chain region having at least 90 %, 95 %, 98 %, 99 % or 100 % sequence identity with SEQ ID NO. 135 and/or
   a light chain region having at least 90 %, 95 %, 98 %, 99 % or 100 % sequence identity with SEQ ID NO. 136 (TPP-14392), or
c) a heavy chain region having at least 90 %, 95 %, 98 %, 99 % or 100 % sequence identity with SEQ ID NO. 137 and/or
   a light chain region having at least 90 %, 95 %, 98 %, 99 % or 100 % sequence identity with SEQ ID NO. 138 (TPP-17074), or
d) a heavy chain region having at least 90 %, 95 %, 98 %, 99 % or 100 % sequence identity with SEQ ID NO. 131 and/or
   a light chain region having at least 90 %, 95 %, 98 %, 99 % or 100 % sequence identity with SEQ ID NO. 132 (TPP-17078), or
e) a heavy chain region having at least 90 %, 95 %, 98 %, 99 % or 100 % sequence identity with SEQ ID NO. 149 and/or
   a light chain region having at least 90 %, 95 %, 98 %, 99 % or 100 % sequence identity with SEQ ID NO. 150 (TPP-17421).

**In some highly preferred embodiments E of the first aspect,** the third and fourth antigen binding domains of the isolated tetravalent bispecific antibody capable of binding LRRC15 is comprised in a heavy chain region having at least 90 %, 95 %, 98 %, 99 % or 100 % sequence identity with SEQ ID NO. 133 and/or a light chain region having at least 90 %, 95 %, 98 %, 99 % or 100 % sequence identity with SEQ ID NO. 134 (TPP-14389). **In some highly preferred embodiments E of the first aspect,** the third and fourth antigen binding domains of the isolated tetravalent bispecific antibody capable of binding LRRC15 is comprise in a heavy chain region having at least 90 %, 95 %, 98 %, 99 % or 100 % sequence identity with SEQ ID NO. 135 and/or a light chain region having at least 90 %, 95 %, 98 %, 99 % or 100 % sequence identity with SEQ ID NO. 136 (TPP-14392).In **some highly preferred embodiments E of the first aspect,** the third and fourth antigen binding domains of the isolated tetravalent bispecific antibody capable of binding LRRC15 comprise a heavy chain region having at least 90 %, 95 %, 98 %, 99 % or 100 % sequence identity with SEQ ID NO. 137 and/or a light chain region having at least 90 %, 95 %, 98 %, 99 % or 100 % sequence identity with SEQ ID NO. 138 **(TPP-17074).In some highly preferred embodiments E of the first aspect,** the third and fourth antigen binding domains of the isolated tetravalent bispecific antibody capable of binding LRRC15 comprise a heavy chain region having at least 90 %, 95 %, 98 %, 99 % or 100 % sequence identity with SEQ ID NO. 131 and/or a light chain region having at least 90 %, 95 %, 98 %, 99 % or 100 % sequence identity with SEQ ID NO. 132 (TPP-17078). **In some highly preferred embodiments E of the first aspect,** the third and fourth antigen binding domains of the isolated tetravalent bispecific antibody capable of binding LRRC15 comprise a heavy chain region having at least 90 %, 95 %, 98 %, 99 % or 100 % sequence identity with SEQ ID NO. 149 and/or a light chain region having at least 90 %, 95 %, 98 %, 99 % or 100 % sequence identity with SEQ ID NO. 150 (TPP-17421).

### Tetravalent Bispecific LTβR Antibody targeted to LRRC15

Also provided are isolated tetravalent bispecific antibodies which are more potent agonistic LTβR antibodies in the presence of LRRC15 as **embodiments F of the first aspect.** As described in example 4 an isolated tetravalent bispecific antibody - comprising two antigen binding domains capable of binding to LTβR described herein and two antigen binding domains capable of binding to LRRC15 - showed more stimulation of the LTβR signaling pathway in the presence of LRRC15 expressing cells than in the absence of LRRC15 expressing cells. Moreover, in example 6 the isolated tetravalent bispecific antibodies - comprising two antigen binding domains capable of binding to LTβR described herein and two antigen binding domains capable of binding to LRRC15 - i.e. the targeted LTβR antibodies comprising a first and second antigen binding domain are binding to human LTβR and a third antigen binding domain and a fourth antigen binding domain binding to LRRC15 - showed better anti-tumor efficacy than the non-targeted LTβR or control antibodies.

**In some preferred embodiments F of the first aspect the** isolated tetravalent bispecific antibody comprises
**a)** a first and second antigen binding domain comprising at least one, two, three, four, five and preferably six CDR sequences having at least 90 %, 95 %, 98 %, 99 % or 100 % sequence identity with at least one of SEQ ID NO. 69, SEQ ID NO. 70, SEQ ID NO. 71, SEQ ID NO. 72, SEQ ID NO. 73 and SEQ ID NO. 74 (TPP-34849) and a third and fourth antigen binding domain comprising at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078), (TPP-32508, TPP-31142, TPP-32970) or
**b)** a first and second antigen binding domain comprising at least one, two, three, four, five and preferably six CDR sequences having at least 90 %, 95 %, 98 %, 99 % or 100 % sequence identity with at least one of SEQ ID NO. 69, SEQ ID NO. 70, SEQ ID NO. 71, SEQ ID NO. 72:, SEQ ID NO. 73 and SEQ ID NO. 74 (TPP-34849) and a third and fourth antigen binding domain comprising at least one, two, three, four, five and preferably six CDR sequences having at least 90 %, 95 %, 98 %, 99 % or 100 % sequence identity with at least one of SEQ ID NO. 140, SEQ ID NO. 141, SEQ ID NO. 142, SEQ ID NO. 144, SEQ ID NO. 145, and SEQ ID NO. 146 (TPP-17421) (TPP-32971) or
**c)** a first and second antigen binding domain comprising at least one, two, three, four, five and preferably six CDR sequences having at least 90 %, 95 %, 98 %, 99 % or 100 % sequence identity with at least one of SEQ ID NO. 29, SEQ ID NO. 30, SEQ ID NO. 31, SEQ ID NO. 32:, SEQ ID NO. 33 and SEQ ID NO. 34 (TPP-34847) and a third and fourth antigen binding domain comprising at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078), (TPP-23518) or
**d)** a first and second antigen binding domain comprising at least one, two, three, four, five and preferably six CDR sequences having at least 90 %, 95 %, 98 %, 99 % or 100 % sequence identity with at least one of SEQ ID NO. 59, SEQ ID NO. 60, SEQ ID NO. 61, SEQ ID NO. 62, SEQ ID NO. 63, and SEQ ID NO. 64 (TPP-34848) and a third and fourth antigen binding domain comprising at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078), (TPP-29570) or
**e)** a first and second antigen binding domain comprising at least one, two, three, four, five and preferably six CDR sequences having at least 90 %, 95 %, 98 %, 99 % or 100 % sequence identity with at least one of SEQ ID NO. 39, SEQ ID NO. 40, SEQ ID NO. 41, SEQ ID NO. 42, SEQ ID NO. 43 and SEQ ID NO. 44 (TPP-34999) and a third and fourth antigen binding domain comprising at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078), (TPP-29168) or
**f)** a first and second antigen binding domain comprising at least one, two, three, four, five and preferably six CDR sequences having at least 90 %, 95 %, 98 %, 99 % or 100 % sequence identity with at least one of SEQ ID NO. 49, SEQ ID NO. 50, SEQ ID NO. 51, SEQ ID NO. 52, SEQ ID NO. 53 and SEQ ID NO. 54 (TPP-35000) a third and fourth antigen binding domain comprising at least one, two, three, four, five and preferably six CDR sequences having at least 90 %, 95 %, 98 %, 99 % or 100 % sequence identity with at least one of SEQ ID NO. 122, SEQ ID NO. 123 , SEQ ID NO. 124, SEQ ID NO. 126, SEQ ID NO. 127, and SEQ ID NO. 128 (TPP-17078) (TPP-29701).

**In some highly preferred embodiments F of the first aspect the** isolated tetravalent bispecific antibody comprises
a) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-32508, TPP-31142, TPP-32970) or
b) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421) (TPP-32971) or
c) a first and second antigen binding domain comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-23518) or
d) a first and second antigen binding domain comprising a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29570) or
e) a first and second antigen binding domain comprising a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29168)
f) a first and second antigen binding domain comprising a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29701).

**In some highly preferred embodiments F of the first aspect the** isolated tetravalent bispecific antibody comprises a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-32508, TPP-31142, TPP-32970). **In some highly preferred embodiments F of the first aspect the** isolated tetravalent bispecific antibody comprises a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421) (TPP-32971). **In some highly preferred embodiments F of the first aspect the** isolated tetravalent bispecific antibody comprises a first and second antigen binding domain comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-23518)). **In some highly preferred embodiments F of the first aspect the** isolated tetravalent bispecific antibody comprises a first and second antigen binding domain comprising a first and second antigen binding domain comprising a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29570). **In some highly preferred embodiments F of the first aspect the** isolated tetravalent bispecific antibody comprises a first and second antigen binding domain comprising a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29168). **In some highly preferred embodiments F of the first aspect the** isolated tetravalent bispecific antibody comprises a first and second antigen binding domain comprising a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29701).

**In some further highly preferred embodiments F of the first aspect** the isolated tetravalent bispecific antibody comprises
a) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-32508, TPP-31142, TPP-32970) or
b) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421) (TPP-32971) or
c) a first and second antigen binding domain comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-23518) or
d) a first and second antigen binding domain comprising a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64(TPP-34848) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29570) or
e) a first and second antigen binding domain comprising a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29168)
f) a first and second antigen binding domain comprising a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29701) wherein each of the first and second antigen binding domains are an scFV fragments,

As the isolated bispecific antibodies described in the first aspect are tetravalent they comprise a total of 24 CDRS. In some highly preferred embodiments of the first aspect the isolated tetravalent bispecific antibody described herein the first antigen binding domain capable of binding LTβR and the second antigen binding domain capable of binding LTβR are identical. In a preferred embodiment of this embodiment such an isolated tetravalent bispecific antibody targets an LRRC15 expressing cancer cell via the third and fourth binding domains capable of binding to LRRC 15 wherein the third and fourth binding domains are also identical. Hence in such a setting an isolated bispecific antibodies described in the first aspect comprises 24 CDRs in total which equates to 12 differing CDRs as each CDR is present twice.

In a preferred embodiment of this embodiment such an isolated tetravalent bispecific antibody targets an LRRC15 expressing cancer cell via the third and fourth binding domains capable of binding to LRRC15, and wherein the cancer cell selected from the group consisting of lung cancer, non-small cell lung carcinoma, head and neck cancer, head and neck squamous cell carcinoma, sarcoma, glioblastoma, melanoma, breast cancer, HER2 negative breast cancer, HER2 positive breast cancer, triple negative breast cancer, HR+ breast cancer, pancreatic cancer, pancreatic ductal adenocarcinoma or colorectal cancer. In a preferred embodiment not only the first antigen binding domain capable of binding LTβR and the second antigen binding domain capable of binding LTβR are identical but also the third and fourth antigen binding domains capable of binding LRRC15 are identical. Again in a preferred embodiment of this embodiment such an isolated tetravalent bispecific antibody targets an LRRC15 expressing cancer cell via the third and fourth binding domains capable of binding to LRRC15 and wherein the cancer cell selected from the group consisting of lung cancer, non-small cell lung carcinoma, head and neck cancer, head and neck squamous cell carcinoma, sarcoma, glioblastoma, melanoma, breast cancer, HER2 negative breast cancer, HER2 positive breast cancer, triple negative breast cancer, HR+ breast cancer, pancreatic cancer, pancreatic ductal adenocarcinoma or colorectal cancer.

**In some highly preferred embodiments G of the first aspect** an isolated tetravalent bispecific antibody described herein comprises
a) a heavy chain region having at least 90 %, 95 %, 98 %, 99 % or 100 % sequence identity with SEQ ID NO. 19 and a light chain region having at least 90 %, 95 %, 98 %, 99 % or 100 % sequence identity with SEQ ID NO. 20 (TPP-32970) or
b) a heavy chain region having at least 90 %, 95 %, 98 %, 99 % or 100 % sequence identity with SEQ ID NO. 15 and a light chain region having at least 90 %, 95 %, 98 %, 99 % or 100 % sequence identity with SEQ ID NO. 16 (TPP-31142) or
c) a heavy chain region having at least 90 %, 95 %, 98 %, 99 % or 100 % sequence identity with SEQ ID NO. 21 and a light chain region having at least 90 %, 95 %, 98 %, 99 % or 100 % sequence identity with SEQ ID NO. 22 (TPP-32971) or
d) a heavy chain region having at least 90 %, 95 %, 98 %, 99 % or 100 % sequence identity with SEQ ID NO. 5 and a light chain region having at least 90 %, 95 %, 98 %, 99 % or 100 % sequence identity with SEQ ID NO. 6 (TPP-29168) or
e) a heavy chain region having at least 90 %, 95 %, 98 %, 99 % or 100 % sequence identity with SEQ ID NO. 25 and a light chain region having at least 90 %, 95 %, 98 %, 99 % or 100 % sequence identity with SEQ ID NO. 26 (TPP-29701) or
f) a heavy chain region having at least 90 %, 95 %, 98 %, 99 % or 100 % sequence identity with SEQ ID NO. 11 and a light chain region having at least 90 %, 95 %, 98 %, 99 % or 100 % sequence identity with SEQ ID NO. 12 (TPP-29570)

**In some highly preferred embodiments G of the first aspect** the isolated tetravalent bispecific antibody capable of binding LTβR and LRRC15 thus comprises a heavy chain region having at least 90 %, 95 %, 98 %, 99 % or 100 % sequence identity with SEQ ID NO. 19 and a light chain region having at least 90 %, 95 %, 98 %, 99 % or 100 % sequence identity with SEQ ID NO. 20 (TPP-32970). **In some highly preferred embodiments G of the first aspect** the isolated tetravalent bispecific antibody capable of binding LTβR and LRRC15 thus comprises a heavy chain region having at least 90 %, 95 %, 98 %, 99 % or 100 % sequence identity with SEQ ID NO. 15 and a light chain region having at least 90 %, 95 %, 98 %, 99 % or 100 % sequence identity with SEQ ID NO. 16 (TPP-31142). **In some highly preferred embodiments G of the first aspect** the isolated tetravalent bispecific antibody capable of binding LTβR and LRRC15 thus comprises a heavy chain region having at least 90 %, 95 %, 98 %, 99 % or 100 % sequence identity with SEQ ID NO. 21 and a light chain region having at least 90 %, 95 %, 98 %, 99 % or 100 % sequence identity with SEQ ID NO. 22 (TPP-32971). **In some highly preferred embodiments G of the first aspect** the isolated tetravalent bispecific antibody capable of binding LTβR and LRRC15 thus comprises a heavy chain region having at least 90 %, 95 %, 98 %, 99 % or 100 % sequence identity with SEQ ID NO. 5 and a light chain region having at least 90 %, 95 %, 98 %, 99 % or 100 % sequence identity with SEQ ID NO. 6 (TPP-29168). **In some highly preferred embodiments G of the first aspect** the isolated tetravalent bispecific antibody capable of binding LTβR and LRRC15 thus comprises a heavy chain region having at least 90 %, 95 %, 98 %, 99 % or 100 % sequence identity with SEQ ID NO. 25 and a light chain region having at least 90 %, 95 %, 98 %, 99 % or 100 % sequence identity with SEQ ID NO. 26 (TPP-29701). **In some highly preferred embodiments G of the first aspect** the isolated tetravalent bispecific antibody capable of binding LTβR and LRRC15 thus comprises a a heavy chain region having at least 90 %, 95 %, 98 %, 99 % or 100 % sequence identity with SEQ ID NO. 11 and a light chain region having at least 90 %, 95 %, 98 %, 99 % or 100 % sequence identity with SEQ ID NO. 12 (TPP-29570).

As demonstrated in example 3.1 TPP-31142, TPP-32970 and TPP-32971 showed reduced binding to human Fcy compared to TPP-29445 since these compounds comprised a silent Fc domain. TPP-32970 and TPP-32971 showed even more reduced binding than TPP-31142.

### LTβR binding domains (Second Aspect)

As demonstrated in example 5 the finding that the isolated tetravalent bispecific antibody of the first aspect induced stimulation of the LTβR signaling pathway if used as targeted LTβR antibody but did not lead to relevant LTβR signaling pathway stimulation if used as untargeted LTβR antibody was dependent on the first and second antigen binding domains capable of binding to LTβR. In detail the comparative isolated tetravalent bispecific antibody comprised a first and a second antigen binding domain capable of binding to LTβR but did not comprising the first and second antigen binding domain CDRs of the second aspect. Instead, the comparative isolated tetravalent bispecific antibody comprised first and a second antigen binding domain capable of binding to LTβR of the state of the art. Both the comparative isolated tetravalent bispecific antibody and the isolated tetravalent bispecific antibody comprising the first and second antigen binding domains CDRs of the second aspect comprised third and fourth binding domains capable of binding to mesothelin. Both the comparative isolated tetravalent bispecific antibody and the isolated tetravalent bispecific antibody comprising the first and second antigen binding domains CDRs of the second aspect were LTβR agonists in co-cultures. However, the comparative isolated tetravalent bispecific antibody was a stronger LTβR agonists in monoculture and also as non-targeted antibody. Hence the first and second antigen binding domains capable of binding to LTβR were agonistic stimulators of the LTβR signaling pathway only in a targeted LTβR antibody i.e. they were considerably more potent / specific agonists of the LTβR signaling pathway only if a targeting moiety was present.

Therefore, in a **second aspect** what is described herein relates to a first antigen binding domain capable of binding to LTβR and a second antigen binding domain capable of binding to LTβR wherein each of the first and second antigen binding domain comprises at least one, two, three, four, five and preferably six CDR sequences, wherein each of said CDR sequences has at least 90 %, 95 %, 98 %, 99 % or 100 % sequence identity with at least one of
a) SEQ ID NO. 69, SEQ ID NO. 70, SEQ ID NO. 71, SEQ ID NO. 72, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 40 (TPP-34999), or
e) SEQ ID NO. 49, SEQ ID NO. 50, SEQ ID NO. 51, SEQ ID NO. 52,, SEQ ID NO. 53 and SEQ ID NO. 54 (TPP-35000).

In a preferred embodiment of the **second aspect** the first and second antigen binding domains capable of binding to LTβR each comprise
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848), or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000).

In a preferred embodiment of the **second aspect** the first antigen binding domain capable of binding to LTβR and the second antigen binding domain capable of binding to LTβR wherein each of the first and second antigen binding domain comprises at least one, two, three, four, five and preferably six CDR sequences, wherein each of said CDR sequences has at least 90 %, 95 %, 98 %, 99 % or 100 % sequence identity with at least one of
a) SEQ ID NO. 69, SEQ ID NO. 70, SEQ ID NO. 71, SEQ ID NO. 72, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 40 (TPP-34999), or
e) SEQ ID NO. 49, SEQ ID NO. 50, SEQ ID NO. 51, SEQ ID NO. 52,, SEQ ID NO. 53 and SEQ ID NO. 54 (TPP-35000)
are binding human LTβR.

In a preferred embodiment of the **second aspect** the first and second antigen binding domains capable of binding to LTβR each comprise
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848), or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000)
and are binding human LTβR.

In a further preferred embodiment of the **second aspect** the first antigen binding domain capable of binding to LTβR and the second antigen binding domain capable of binding to LTβR are identical wherein each of the first and second antigen binding domain comprises at least one, two, three, four, five and preferably six CDR sequences, wherein each of said CDR sequences has at least 90 %, 95 %, 98 %, 99 % or 100 % sequence identity with at least one of
a) SEQ ID NO. 69, SEQ ID NO. 70, SEQ ID NO. 71, SEQ ID NO. 72, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 40 (TPP-34999), or
e) SEQ ID NO. 49, SEQ ID NO. 50, SEQ ID NO. 51, SEQ ID NO. 52,, SEQ ID NO. 53 and SEQ ID NO. 54 (TPP-35000).

In a further preferred embodiment of the **second aspect** the first antigen binding domain capable of binding to LTβR and the second antigen binding domain capable of binding to LTβR are identical and each comprise
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848), or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000).

In a further preferred embodiment of the **second aspect** the first antigen binding domain capable of binding to LTβR and the second antigen binding domain capable of binding to LTβR are identical and each comprise
a) as heavy chain variable region SEQ ID NO. 75 comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and as light chain variable region SEQ ID NO. 76 comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) as heavy chain variable region SEQ ID NO. 65 comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and as light chain variable region SEQ ID NO. 66 comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848) or
c) as heavy chain variable region SEQ ID NO. 35 comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and as light chain variable region SEQ ID NO. 36 comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847), or
d) as heavy chain variable region SEQ ID NO. 45 comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and as light chain variable region SEQ ID NO. 46 comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999), or
e) as heavy chain variable region SEQ ID NO. 55 comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and as light chain variable region SEQ ID NO. 56 comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

In some highly preferred embodiments of the **second aspect** the first and second antigen binding domains of the isolated tetravalent bispecific antibody capable of binding LTβR are identical antigen-binding scFv fragments each comprising as heavy chain variable region SEQ ID NO. 75 comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and as light chain variable region SEQ ID NO. 76 comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849). In some highly preferred embodiments of the **second aspect** the first and second antigen binding domains of the isolated tetravalent bispecific antibody capable of binding LTβR are identical antigen-binding scFv fragments each comprising as heavy chain variable region SEQ ID NO. 65 comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and as light chain variable region SEQ ID NO. 66 comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848). In some highly preferred embodiments of **the second aspect** the first and second antigen binding domains of the isolated tetravalent bispecific antibody capable of binding LTβR are identical antigen-binding scFv fragments each comprising as heavy chain variable region SEQ ID NO. 35 comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and as light chain variable region SEQ ID NO. 36 comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO. 34 (TPP-34847). In some highly preferred embodiments of the **second aspect** the first and second antigen binding domains of the isolated tetravalent bispecific antibody capable of binding LTβR are identical antigen-binding scFv fragments each comprising as heavy chain variable region SEQ ID NO. 45 comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and as light chain variable region SEQ ID NO. 46 comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO. 44 (TPP-34999). In some highly preferred embodiments of the **second aspect** the first and second antigen binding domains of the isolated tetravalent bispecific antibody capable of binding LTβR are identical antigen-binding scFv fragments each comprising as heavy chain variable region SEQ ID NO. 55 comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and as light chain variable region SEQ ID NO. 56 comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO. 54 (TPP-35000).

In some highly preferred embodiments of the **second aspect** the first and second antigen binding domains capable of binding LTβR are antigen-binding fragments binding to human LTβR
a) having at least 90 %, 95 %, 98 %, 99 % or 100 % sequence identity with SEQ ID NO. 77 (TPP-34849),
b) having at least 90 %, 95 %, 98 %, 99 % or 100 % sequence identity with SEQ ID NO. 67 (TPP-34848),
c) having at least 90 %, 95 %, 98 %, 99 % or 100 % sequence identity with SEQ ID NO. 37 (TPP-34847),
d) having at least 90 %, 95 %, 98 %, 99 % or 100 % sequence identity with SEQ ID NO. 47 (TPP-34999),
e) having at least 90 %, 95 %, 98 %, 99 % or 100 % sequence identity with SEQ ID NO. 57 (TPP-35000).

In some highly preferred embodiments of the **second aspect** the first and second antigen binding domains capable of binding LTβR are identical antigen-binding scFv fragments binding to human LTβR
a) having at least 90 %, 95 %, 98 %, 99 % or 100 % sequence identity with SEQ ID NO. 77 (TPP-34849),
b) having at least 90 %, 95 %, 98 %, 99 % or 100 % sequence identity with SEQ ID NO. 67 (TPP-34848),
c) having at least 90 %, 95 %, 98 %, 99 % or 100 % sequence identity with SEQ ID NO. 37 (TPP-34847),
d) having at least 90 %, 95 %, 98 %, 99 % or 100 % sequence identity with SEQ ID NO. 47 (TPP-34999),
e) having at least 90 %, 95 %, 98 %, 99 % or 100 % sequence identity with SEQ ID NO. 57 (TPP-35000).

In some highly preferred embodiments of the **second aspect** the first and second antigen binding domains capable of binding LTβR are identical antigen-binding scFv fragments binding to human LTβR having at least 90 %, 95 %, 98 %, 99 % or 100 % sequence identity with SEQ ID NO. 77 (TPP-34849). In some highly preferred embodiments of the **second aspect** the first and second antigen binding domains capable of binding LTβR are identical antigen-binding scFv fragments binding to human LTβR having at least 90 %, 95 %, 98 %, 99 % or 100 % sequence identity with SEQ ID NO. 67 (TPP-34848). In some highly preferred embodiments of the **second aspect** the first and second antigen binding domains capable of binding LTβR are identical antigen-binding scFv fragments binding to human LTβR having at least 90 %, 95 %, 98 %, 99 % or 100 % sequence identity with SEQ ID NO. 37 (TPP-34847). In some highly preferred embodiments of the **second aspect** the first and second antigen binding domains capable of binding LTβR are identical antigen-binding scFv fragments binding to human LTβR having at least 90 %, 95 %, 98 %, 99 % or 100 % sequence identity with SEQ ID NO. 47 (TPP-34999). In some highly preferred embodiments of the **second aspect** the first and second antigen binding domains capable of binding LTβR are identical antigen-binding scFv fragments binding to human LTβR having at least 90 %, 95 %, 98 %, 99 % or 100 % sequence identity with SEQ ID NO. 57 (TPP-35000).

The scFV fragments TPP-34847, TPP-34848, TPP-34849, TPP-34999, TPP-35000 were found during antibody screening as detailed in 1.2.3 to have acceptable affinity towards human and mouse LTβR. Moreover as described in 1.2.5. the scFV fragment TPP-34849 (SEQ ID NO. 77) carrying the single amino acid alteration VH:M108T compared to TPP-34848 (SEQ ID NO. 67) and the germlining alterations as compared to TPP-34847 (SEQ ID NO. 37) - comprised in TPP-23518 - showed reduced potency compared to TPP-34847. In an even more preferred embodiment of the second aspect the one first antigen binding domain specific for LTβR and the one second antigen binding domain specific for LTβR each are each a single-chain Fv antibody fragment and each comprise the single amino acid alteration VH:M108T compared to TPP-34848 and TPP-34847. This mutation VH:M108T unexpectedly showed the most favorable profile i.e. the EC-50 value (cf. Example 4.1.) of tetravalent bispecific antibodies comprising TPP-34849 (TPP-32970, TPP-32791 and TPP-31142) was higher than that of TPP-23518 comprising TPP-34847 when no LLRC15 expressing cells were present or if the LRRC15 antigen binding domain was missing.

### Pharmaceutical composition (Third Aspect)

**According to a third aspect,** there is provided a pharmaceutical composition comprising a isolated tetravalent bispecific antibody (BsAB) according to the first aspect or first antigen binding domain capable of binding to LTβR and a second antigen binding domain capable of binding to LTβR according to the second aspect or a combination of both.

For example, the pharmaceutical composition comprises a tetravalent bispecific antibody BsAB according to the first aspect in a therapeutically effective amount. Preferably, the pharmaceutical composition furthermore comprises a pharmaceutically acceptable carrier, excipient, or stabilizer.

Pharmaceutical compositions can be prepared by mixing the isolated tetravalent bispecific antibody (BsAB) according to the first aspect or first antigen binding domain capable of binding to LTβR and a second antigen binding domain capable of binding to LTβR according to the second aspect having the desired degree of purity with optional physiologically acceptable carriers, excipients or stabilizers (Remington 's Pharmaceutical Sciences (18th ed.; Mack Pub. Co.: Eaton, Pa., 1990). Pharmaceutical compositions may be for example in the form of lyophilized formulations or aqueous solutions.

### Carriers, excipients, stabilizers

Acceptable carriers, excipients, or stabilizers are nontoxic to recipients at the dosages and concentrations employed, and include buffers such as phosphate buffer (e.g. PBS), citrate buffer, and other organic acid buffer; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (e.g. less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as Tween^{®}, Pluronic^{®} or polyethylene glycol (PEG).

Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate, para-aminobenzoic acid (pABA)),
- colourants (for example inorganic pigments such as, for example, iron oxides, titanium dioxide),
- flavourings, sweeteners, flavour- and/or odour-masking agents.

In a highly preferred embodiment, the pharmaceutical composition comprises
a. a tetravalent bispecific antibody as described herein, and
b. citrate, e.g. 1 mM to 100 mM or 30 mM, and/or
c. sucrose, e.g. 1 mM to 100 mM or 50 mM, and/or
d. EDTA, e.g. 0.1 mM to 20 mM or 2 mM, and/or
e. pABA, e.g. 0.01 to 10 mg/ml or 0.5 mg/ml, and/or
f. polysorbate, e.g. 0.1 to 20 % or 7 %.

### Multiple therapeutically active compounds

The pharmaceutical composition may contain more than one active compound, e.g. as necessary or beneficial for the particular cancer type e.g. PanCa, HCC, lung ADC, Cervix, Endometrium, Ovarian, undefined breast Ca, ER/PR/ pos breat cancer, TNBCa, HER21 pos breast, lung LCC, Lung SCC, Lung ADC, CDC KRAS mut, CRC KRAS WT, Sarcoma being treated. **According to some preferred embodiments,** the pharmaceutical composition comprises one or more further therapeutically active compounds.

In some preferred of these embodiments, the one or more further therapeutically active compound(s) comprise(s)
a) an antibody or a small molecule targeting a checkpoint protein, such as PD1, PD-L1 or CTLA-4, and/or
b) a chemotherapeutic agent, such as a taxane, doxorubicin, cis-platin, carboplatin or oxaliplatin, and/or
c) a kinase inhibitor, a microtubule disruptor, an antimetabolite, a topoisomerase inhibitor, a DNA intercalator, an alkylating agent, a hormonal therapy, a receptor antagonist, an activator of tumor cell apoptosis, or an antiangiogenic agent, an immunomodulatory agent, an inhibitor of cell adhesion, a cytotoxic or cytostatic agent an activator of cell apoptosis, or an agent that increases the sensitivity of cells to apoptotic inducers.

### Use and method of treatment (Fourth Aspect)

**According to a fourth aspect,** herein is provided a tetravalent bispecific antibody according to the first aspect for use in a method of treating cancer, characterized in that the method of treating the cancer comprises administering at least one tetravalent bispecific antibody according to the first aspect comprising a first antigen binding domain capable of binding to LTβR and a second antigen binding domain capable of binding to LTβR and a third antigen binding domain capable of binding to an antigen specific for the cancer to be treated and a fourth antigen binding domain capable of binding to the same antigen other than LTβR to which the third antigen binding domain is capable of binding to, wherein the at least one isolated tetravalent bispecific antibody shows more stimulation of the LTβR signaling pathway in a targeted LTβR antibody.

**In one embodiment of the fourth aspect** herein is provided a tetravalent bispecific antibody according to the first aspect for use in a method of treating a cancer tumor, characterized in that the method of treating the cancer tumor comprises administering at least one tetravalent bispecific antibody according to the first aspect comprising a first antigen binding domain capable of binding to LTβR and a second antigen binding domain capable of binding to LTβR and a third antigen binding domain capable of binding to an antigen on the tumor cells or located in the tumor microenvironment and a fourth antigen binding domain capable of binding to the same antigen other than LTβR to which the third antigen binding domain is capable of binding to, wherein the at least one isolated tetravalent bispecific antibody shows more stimulation of the LTβR signaling pathway in a targeted LTβR antibody.

**In a preferred embodiment of the fourth aspect** herein is provided a tetravalent bispecific antibody according to the first aspect for use in a method of treating an LRRC15 positive cancer, characterized in that the method of treating the LRRC15 positive cancer comprises administering at least one tetravalent bispecific antibody according to the first aspect comprising a first antigen binding domain capable of binding to LTβR and a second antigen binding domain capable of binding to LTβR and a third antigen binding domain capable of binding to LRRC15 and a fourth antigen binding domain capable of binding to the same antigen other than LTβR to which the third antigen binding domain is capable of binding to, wherein the at least one isolated tetravalent bispecific antibody shows more stimulation of the LTβR signaling pathway in a targeted LTβR antibody.

Moreover there is provided the tetravalent bispecific antibody according to the first aspect or the antigen binding domains according to the 2nd aspect, or the pharmaceutical composition according to the 3rd aspect for use as a medicament.

Furthermore in example 6 it was demonstrated that the targeted LRRC15-LTβR tetravalent bispecific antibody comprising the first antigen binding domain capable of binding to LTβR and a second antigen binding domain capable of binding to LTβR of the second aspect did not show agonism in immune compromised NSG mice meaning that the anti-tumor effects were immune cell mediated. Hence it is preferred that the first antigen binding domain capable of binding to LTβR and a second antigen binding domain capable of binding to LTβR of the second aspect e.g. as part of a tetravalent bispecific antibody are used in immune cell dependent anti-tumor therapy.

Also, there is provided the use of the isolated tetravalent bispecific antibody according to the first aspect or the antigen binding domains capable of binding to LTβR according to the 2nd aspect, or the pharmaceutical composition according to the 3rd aspect for the manufacture of a medicament. Furthermore, there is provided a method of treating a disease, the method comprising administering an effective dose of the tetravalent bispecific antibody according to the first aspect or the antigen binding domains according to the 2nd aspect, or the pharmaceutical composition according to the 3rd aspect. For therapeutic applications, the isolated tetravalent bispecific, the antibody antigen binding domains capable of binding to LTβR according to the 2nd aspect or the pharmaceutical composition according to what is described herein, can be administered to a patient, e.g. to a human or non-human subject, in a pharmaceutically acceptable dosage form. For example, administration may occur intravenously as a bolus or by continuous infusion over a period of time, by intramuscular, intraperitoneal, intra-cerebrospinal, subcutaneous, intra-articular, intrasynovial, intrathecal, oral, topical, or inhalation routes. The antibodies, fragments, conjugates and pharmaceutical compositions according to the current description are particularly suitable to be administered by intra-tumoral, peri-tumoral, intra-lesional, or peri-lesional routes, to exert local as well as systemic therapeutic effects.

Possible administration routes include parenteral (e.g., intramuscular, intravenous, intra-arterial, intraperitoneal, or subcutaneous), intrapulmonary and intranasal. In addition, the antibodies, fragments, conjugates and pharmaceutical compositions might be administered by pulse infusion, with, e.g., declining doses of the antibody, fragment or conjugate. Preferably, the dosing is given by injections, most preferably intravenous or subcutaneous injections, depending in part on whether the administration is brief or chronic. The amount to be administered may depend on a variety of factors such as the clinical symptoms, weight of the patient or subject, and whether other drugs are administered. The skilled artisan will recognize that the route of administration will vary depending on the disorder or condition to be treated.

Dosing frequency of the isolated tetravalent bispecific antibody may range from once every 3-6 months to weekly or daily dosing. Similarly, dose levels range from a low mg fixed dose (daily, weekly, biweekly, or monthly, depending on antibody) up to approximately 1 g doses. Dosing frequency depends on a variety of factors including the concentration and turnover rate of the target, biodistribution of the target, half-life of the antibodies, fragments or conjugates and potential pharmacodynamic effects that may enhance the biological effects of the antibodies, fragments, conjugates and pharmaceutical compositions beyond its presence in pharmacologically relevant levels.

For the prevention or treatment of disease, the appropriate dosage of isolated tetravalent bispecific antibody according to the first aspect or antigen binding domains capable of binding to LTβR according to the 2nd aspect will depend on the type of disease to be treated, the severity and course of the disease, whether the antibody is administered for preventive or therapeutic purposes, previous therapy, the subject's clinical history and response to the antibody variant, and the discretion of the attending physician or health veterinary professional. The isolated tetravalent bispecific antibody according to the first aspect or antigen binding domains capable of binding to LTβR according to the 2nd aspect is suitably administered to the subject at one time or over a series of treatments.

When administered intravenously, the pharmaceutical composition comprising the antibodies can be administered by infusion over a period of about 0.5 to about 5 hours. In some embodiments, infusion may occur over a period of about 0.5 to about 2.5 hours, over a period of about 0.5 to about 2.0 hours, over a period of about 0.5 to about 1.5 hours, or over a period of about 1.5 hours, depending upon the antibodies, fragments, conjugates and pharmaceutical compositions being administered and the amount of antibody, fragment or conjugate being administered.

Preferably, the isolated tetravalent bispecific antibody described herein is administered at a total dose of ≥ 0.15 mg/kg, ≥ 1 mg/kg, ≥ 1.5 mg/kg, ≥ 3 mg/kg, ≥ 5 mg/kg, ≥ 10 mg/kg. It was surprisingly found that a dose ≥ about 1.5 mg/kg conjugate circumvented potential target mediated drug deposition. Target-mediated drug disposition (TMDD) corresponds to a special case wherein a significant proportion of a drug (relative to dose) is bound with high affinity to a pharmacological target, such that this interaction is reflected in the pharmacokinetic properties of the drug.

Preferably, the isolated tetravalent bispecific antibody is administered at an antibody dose of at least 1.5 mg/kg .

### Specific indications

As in general any targeting moiety can be combined with the LTβR antigen binding domains disclosed herein in the second aspect to form the isolated tetravalent bispecific antibody described herein in the first aspect, in theory all types of antigens especially cancer tumors can be targeted. Especially it has been demonstrated in examples 4, 5 and 6 that MSLN and LRRC15 positive cells/tumor environments can be targeted by the LTβR antigen binding domains disclosed herein in the second aspect and hence by the isolated tetravalent bispecific antibody described herein in the first aspect.

With relation to the LRRC 15 targeting isolated tetravalent bispecific antibody of the 1st aspect: According to the TCGA dataset, LRRC15 may be overexpressed in patients with glioma, thyroid cancer, lung cancer, colorectal cancer, head and neck cancer, stomach cancer, liver cancer, pancreatic cancer, renal cancer, urothelial cancer, prostate cancer, testis cancer, breast cancer, cervical cancer, endometrial cancer and melanoma.

In principle, the antibodies, fragments and pharmaceutical compositions described herein can be used in the treatment of any cancer involving LRRC15 expressing cells.

For example, the cancer may comprise cells expressing LRRC15, such as breast cancer, head and neck cancer, squamous cell cancer, (squamous) lung cancer, pancreatic cancer, diffuse large B-cell carcinoma, lung adenocarcinoma, colorectal cancer, gastric cancer, and sarcoma. In some examples, the cancer comprises stroma cells expressing LRRC15. In some other or the same examples, the cancer comprises tumor cells expressing LRRC15 or both.

In some preferred embodiments, the isolated tetravalent bispecific antibody described herein is used as a medicament in the treatment of sarcoma, breast cancer, lung cancer, colorectal cancer, non small cell lung carcinoma (NSCLC), testicular cancer or melanoma.

### Combination Treatment

**According to some preferred embodiments** the medical use of the fourth aspect is a use in simultaneous, separate, or sequential combination with one or more further therapeutically active compounds. The isolated tetravalent bispecific antibody as described herein in the first aspect or the antigen binding domains capable of binding to LTβR according to the second aspect may be used adjunctive to - or with - other agents or treatments having anti-cancer properties. When used adjunctively, the antibodies, fragments or conjugates and other agent(s) may be formulated together in a single, combination pharmaceutical composition or formulation, as described elsewhere herein, or may be formulated and administered separately, either on a single coordinated dosing regimen or on different dosing regimens. Agents administered adjunctively with isolated tetravalent bispecific antibody as described herein in the first aspect or the antigen binding domains capable of binding to LTβR according to the second aspect may have complementary activities to the antibodies, fragments or conjugates thereof, such that the isolated tetravalent bispecific antibody as described herein in the first aspect or the antigen binding domains capable of binding to LTβR according to the second aspect and other agents do not adversely affect each other.

Agents that may be used adjunctively with the isolated tetravalent bispecific antibody as described herein in the first aspect or the antigen binding domains capable of binding to LTβR according to the second aspect can be those described elsewhere herein as further therapeutically active compounds for pharmaceutical composition. For example, agents that may be used adjunctively with isolated tetravalent bispecific antibody as described herein in the first aspect or the antigen binding domains capable of binding to LTβR according to the second aspect can be alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal antiinflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, and the like, as well as combinations of one or more of these agents.

### Diagnostic uses (Fifth Aspect)

The isolated tetravalent bispecific antibody as described herein in the first aspect or the antigen binding domains capable of binding to LTβR according to the second aspect may be used for a variety of purposes, e.g. for in vitro, in vivo and ex vivo applications and/or in vitro, in vivo and ex vivo diagnostics. **According to a fifth aspect,** there is provided the isolated tetravalent bispecific antibody as described herein in the first aspect or antigen binding domains according to the 2nd aspect, or the pharmaceutical composition according to the 3th aspect for use as a diagnostic agent.

For example, LTβR or LRRC15 antibodies or antigen-binding fragments thereof can be used for detecting the presence of LTβR or LRRC15-expressing tumors respectively. The presence or level of LTβR or LRRC15-expressing cells within various biological samples, including serum, and tissue biopsy specimens, may be analyzed.

### DNA/RNA (Sixt Aspect)

**According to a 6^{th} aspect,** there is provided a polynucleotide encoding an isolated tetravalent bispecific antibody according to the first aspect or antigen-binding domains according 2^{nd} aspect. Sequences of exemplary polynucleotides are provided with the sequence listing.

### Vector (Seventh Aspect)

**According to an 7^{th} aspect,** there is provided a vector comprising a polynucleotide according to the 6^{th} aspect.

### Production cell (Eighth Aspect)

**According to an 8^{th} aspect,** there is provided an isolated cell arranged for production of an isolated tetravalent bispecific antibody according to the first aspect or antigen-binding domains according 2^{nd} aspect. Preferably the isolated cell is a mammalian host cell such as a CHO cell or a HEK cell.

### Production method (Ninth Aspect)

**According to a 9t^{h} aspect,** there is provided a method of producing an isolated tetravalent bispecific antibody according to the first aspect or antigen-binding domains according 2^{nd} aspect.

In some preferred embodiments, the method comprises culturing a cell according to the 9^{th} aspect to obtain an isolated tetravalent bispecific antibody according to the first aspect or antigen-binding domains according 2^{nd} and optionally comprises purification of the antibody or antigen-binding domains.

In some preferred embodiments, the method comprises making a polynucleotide according to the 7^{th} aspect.

### Kit of parts (Tenth Aspect)

**According to an 10^{th} aspect**, there is provided a kit comprising the isolated tetravalent bispecific antibody according to the first aspect or antigen-binding domains according 2^{nd} or the pharmaceutical composition according to the 3^{rd} aspect, with instructions for use.

The antibodies, antigen-binding domains or pharmaceutical compositions described herein can be provided in a kit, i.e., a packaged combination of reagents in predetermined amounts in one or more containers with instructions. For example, where the antibody, fragment or conjugate is a therapeutic antibody, fragment or conjugate, the instructions for use may comprise the package insert.

For example, where the antibody is labeled with an enzyme, the kit may include substrates and cofactors required by the enzyme (e.g., a substrate precursor which provides the detectable chromophore or fluorophore). In addition, other additives may be included such as stabilizers, buffers (e.g., a block buffer or lysis buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

### Further uses

The antibodies and antigen-binding domains described herein may be used for a variety of purposes, e.g. for in vitro and ex vivo applications and/or in vitro and ex vivo diagnostics.

In a specific example, the antibodies or antigen binding domains described herein can be used for purification or immobilization of LTβR or LRRC15 expressing cells. In another specific example, the antibodies or antigen binding domains described herein can be used for qualitatively and/or quantitatively measuring levels of LT(3R or LRRC15 expressing cells in biological samples, e.g. in immunoassays, see, e.g., Harlow et al., Antibodies: A Laboratory Manual, Second Edition (Cold Spring Harbor Laboratory Press, 1988).

### Examples

While what has been described herein has been illustrated and described in detail in the drawing and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawing, the disclosure, and the appended claims. Any reference signs should not be construed as limiting the scope. All amino acid sequences disclosed herein are shown from N-terminus to C-terminus; all nucleic acid sequences disclosed herein are shown 5'->3'.

All examples were carried out using standard techniques, except where described otherwise herein. Routine molecular biology techniques of the following examples can be carried out as described in standard laboratory manuals, such as Sambrook et al., Molecular Cloning: A Laboratory Manual, 2nd Ed.; Cold Spring Harbor Laboratory Press, Cold Spring Harbor, N.Y., 1989.

As to general expression of antibodies, antibody fragments and antigen binding domains preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (NY). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resistance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection. Suitable mammalian host cells for expressing the antibodies, antigen binding domains thereof or variants thereof provided herein include Chinese Hamster Ovary (CHO cells) such as CHO-K1, CHO-S, CHO-KISV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4): 1007-15], NS0 myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2):E9).

The expression vector can be designed such that the expressed protein is secreted into the culture medium in which the host cells are grown. The antibodies, antigen-binding fragments thereof or variants thereof can be recovered from the culture medium using standard protein purification methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference depending upon the host employed in a recombinant production procedure, the antibody can be glycosylated or can be non-glycosylated. Such methods are described in many standard laboratory manuals, such as Sambrook, supra, Sections 17.37-17.42; Ausubel, supra, Chapters 10, 12, 13, 16, 18 and 20.

### Example 1. Generation of antibodies, antigens and reference compounds

### Example 1.1 Generation of the LRRC15 antigen binding domain

The generation of LRRC15 antigen binding domains is described in WO 2022/157094 A2 and incorporated herein in its entirety. In brief it was carried out as follows: all antibodies were expressed in HEK293 cells using standard transient transfection procedures and purified from the cell culture supernatant via Protein-A and size exclusion chromatography. In order to generated suitable LRRC15 antigen binding domains various approaches were tried. Prior art antibody sequences such as those of TPP-12942 (huM25) were randomly altered. Only few of the resulting antibodies showed improved behavior. Only examples with improved characteristics were included herein. In other examples, a fully human antibody phage display library was used to isolate LRRC15-specific, human monoclonal antibodies such as TPP-1633 by protein panning (see Hoogenboom H. R., Nat Biotechnol 2005; 23(3):1105-16) with extracellular domains of human LRRC15 and murine LRRC15 as immobilized targets. Protein sequences for LRRC15 were retrieved from the UniProtKB/TrEMBL database using the following identifier: Q8TF66 for human LRRC15, Q80X72 for murine LRRC15 and G7NYR2 for cynomolgus (Macaca fascicularis) LRRC15. To obtain recombinant extracellular domains of LRRC15 protein, extracellular domains were C-terminally appended with a His Tag and expressed in HEK293 cells using standard transient transfection procedures. Proteins were purified from the cell culture supernatant via Ni-IMAC and size exclusion chromatography. Different Fab-phages were identified using phage display, and the corresponding antibodies were re-cloned into a mammalian IgG1 expression vector which provides the missing CH2-CH3 domains not present in the soluble Fab. The resulting IgGs were transiently expressed in mammalian cells, purified by Protein A chromatography and further characterized by their binding abilities to human and murine LRRC15. The antibody TPP-1633 was found to be cross-reactive to both human and mouse LRRC15 with monovalent affinities (KD) in the 200 nM range. Antibodies TPP-1633 and TPP-12942 were furthermore subjected to sequence germlining and further alterations were introduced. The resulting antibodies e.g. TPP-17078 were characterized with regard to monovalent binding affinity (KD) and off-rate (kd) as assessed by surface plasmon resonance (SPR). Variants - especially TPP-14389, TPP-14392, TPP-17074, TPP-17078, or TPP-17421 - of both TPP-1633 and TPP-12942 families were found to be superior with regard to affinity.

### Example 1.2. Generation of the LTβR antigen binding domains

### Example 1.2.1 General description of recombinant DNA constructs and expression, purification and quantification of scFvs and full-length antibodies

Heavy and light chains as well as scFvs were cloned into pTT5 vector system (National Research Council Canada, NRC file 11266) and expressed in HEK293 cells using standard transient transfection procedures. In brief, for small-scale transfection, the HEK293 cells were diluted to 0.5x106 cells/mL with F17 medium (Invitrogen, #A13835-01) supplemented with 10 mL/L of 10% Pluronic F68 (Invitrogen; #24040-32) solution to a total volume of 22.5 mL in 125 mL polycarbonate Erlenmeyer shaker flasks (Corning, #CLS431143), two days before transfection. The cell density at the day of transfection was at least 1.7x10E6 cells/mL. At the day of transfection, 25 µg DNA were thawed in 1.25 mL transfection medium (F17 medium + Pluoronic as above) and 50 µl Polyethylenimine (Polysciences, #23966) at 1 mg/mL, i.e., 50 µg, were added to another 1.25 ml transfection medium. Both solutions were vortexed and subsequently, the Polyethylenimine (PEI) solution transferred to the DNA solution. The mixture was vortexed again and incubated for 15 min at room temperature. After the DNA-PEI mixture was added to the cells and the flask immediately swirled, the cells were incubated at 37° at 5% CO2 in a humidified incubator for five days. Supernatants were collected and antibodies were purified by Protein A chromatography followed by size-exclusion chromatography. After harvest, the supernatant was concentrated and filtered (pore size 0.2 µm) and loaded onto a HiTrap MabSelect SuRe column (Cytiva).

The column was equilibrated in DPBS, pH 7.4. coupled to an Akta Pure 25 system (Cytiva). The column was washed with 10 CV and elution was performed with 6 CV elution buffer (50 mM acetic acid + 50 mM NaCl, pH 3.0). Peak fractions were pooled and neutralized using 3 M Tris pH 9.0. The pool was concentrated (Vivaflow 200 30 kDa membrane [Hydrosart 30kDa]) and filtered through a 0.2 µm filter. The capture step pool was analyzed by analytical SEC (SEC sample was diluted with DPBS pH 7.4 to 2 mg/ml). Next, a preparative SEC run was performed using a Superdex 200 SEC column (Cytiva) coupled to an Akta Pure 25 system. The SEC column was equilibrated using 2 CV DPBS, pH 7.4, sample was loaded, and the column was eluted with 1.5 CV DPBS, pH 7.4. Peak fractions were pooled. The pool was concentrated and sterile filtered (pore size 0.2 µm). The final concentration of the antibody solution was determined using absorbance at 280 nm using a Nanodrop UV spectrophotometer (Thermo). The antibodies were aliquoted, snap-frozen in liquid nitrogen and stored at - 80 °C.

### Example 1.2.2 Antibody Generation from BioInvent antibody libraries

A fully human antibody phage display library (BioInvent n-CoDeR scFv library) was used to isolate human monoclonal LTβR antibodies by panning in alternating fashion against biotinylated recombinant human Lymphotoxin betalTNFRSF3 Fc Chimera (huLTβR, R&D Systems, 629-LR) and biotinylated recombinant mouse Lymphotoxin betalTNFRSF3 Fc Chimera (muLTβR, R&D Systems, 1008-LR) using the following protocol. Briefly, aliquots of streptavidin coated magnetic beads (Dynabeads, Invitrogen) were pre-blocked with 300 ul of a 3% Milk powder solution in PBST (Phosphate buffered saline + 0.05% Tween20) for one hour at room temperature (RT) with end-over-end rotation. Beads were washed (3x with PBST) and subsequently coated with 200 nM of the biotinylated huLTβR target or 400 nM biotinylated off-target antigen here hROR1-Fc (TPP-3188) for 1 hour at RT with end-over-end rotation. A pre-blocked phage library aliquot was incubated firstly with the off-target coated beads to deplete for non-specific binders and the depleted phage (harvested as supernatant following magnetic capture of the beads) were then incubated with the biotinylated target antigen (huLTβR in the first round) coated beads for 1 hour at RT with end-over-end rotation. Non-binding phage were removed by stringent washing (up to 11 wash cycles per selection round) using PBST. Single-chain Fv-expressing phages binding specifically to the bead-bound target following the wash steps were resuspended in 1 ml of PBS and 500 µl of this solution was used to infect 10 ml of Escherichia coli strain HB101 (which had reached an OD₆₀₀ of 0.05) for 30 minutes at 37 °C. The culture was centrifuged, and the cell pellet resuspended in 1 ml of LB (Luria Bertani) growth medium followed by plating on solid LB agar containing Ampicillin to select for phagemid-infected cells. Following overnight growth at 37 °C, the enriched clone pool was harvested by scraping the surface of the agar plate and preparing glycerol stocks. Subsequently the phages from this clone pool were amplified in Escherichia coli strain HB101 using M13KO7 Helper Phage (Invitrogen^{™}). In two subsequent selection rounds performed as described above the target was switched to 200 nM muLTbR for round 2 and back to 100 nM huLTβR for round 3 in order to drive selection of phage expressing scFvs which are crossreactive. For a first qualitative assessment, 88 randomly picked scFv-expressing phage clones from each selection round were inoculated into a flatbottom 96-well plate filled with 100µl LB media supplemented with 100µg/ml Ampicillin and 1% Glucose. After overnight cultivation at 37°C, a new flatbottom 96-well plate filled with 100 µl LB media supplemented with 100µl Ampicillin was inoculated with 5 µl of the overnight culture. The inoculated plate was incubated for 3 hours at 37°C. After the 3 hours 100µl LB media supplemented with 100µl/ml Ampicillin, 200 µM Isopropyl-β**-**D-thiogalactoside (IPTG) and M13KO7 helper phage (6x109 plaque forming units/ml) was added, and plates further incubated at 37°C for 30 minutes followed by incubation at 30°C overnight. Next day the supernatant, including the expressed Fab-phage molecules, was tested for binding to the respective target used before for panning (huLTβR and muLTβR) compared to an off-target antigen (hROR1-Fc TPP-3188) with ELISA, similar to the ELISA protocol as described below in 1.2.3, and underwent sequencing analysis to evaluate diversity. One clone pool was found to contain a unique crossreactive hit rate of ~40% and was chosen for further screening.

### Example 1.2.3 Antibody Screening

From the clone pool generated as described in example 1.2.2 above 10000 single clones were picked and sequenced via Next Generation Sequencing to identify antibodies different in the sequence of their CDRs and screened via ELISA for binding to the recombinant proteins described above (huLTβR, R&D Systems, 629-LR and muLTβR, R&D Systems, 1008-LR). Briefly for HTS-ELISA screening, the proteins were immobilized on streptavidin coated plates (Greiner bio-one 781997) with a concentration of 1 µg/ml at 4°C in coating buffer (Carbonat-Basis, Candor 121125). After washing the plates 3 times with 60 µl PBS 0.05% Tween and blocking with 50 µl Smart Block^{®} (Candor 113500) for 1h at 20°C, 10 µl scFv samples were added to the plates and incubated at 20°C for 1h. After subsequent washing with 60 µl PBS 0.05% Tween for 3 times, 20 µl of an anti-c-Myc HRP antibody were added and incubated for 1h at 20°C followed by subsequent washing 3 times with 60 µl PBS 0.05% Tween and addition of 20 µl Amplex Red solution (Invitrogen A12222, 1: 1000 in NaP-buffer 50 mM pH7.6 with 1: 10000 of 30% H2O2). After final incubation for 20 min at 20°C the signal was determined using an emission wavelength of 595 nm and an excitation wavelength of 530 nm. For analysis, the signal to background ratio of the individual scFv clones was used whereas the background is defined by the average value of 48 wells on each plate, not containing any scFv culture but the respective sample medium. Significant binding to either human or mouse recombinant LTβR was defined as given for a certain clone, if the signal to background ratio was greater than 5, while no specific binding (signal to background ratio smaller 3) could be detected on an irrelevant off-target hROR1-Fc (TPP-3188). This analysis resulted in over 60 distinct scFv clones which demonstrated specific binding to both human and mouse recombinant LTβR and were hence, considered to be cross-reactive. These selected scFv clones were reformatted into bispecific antibodies in the 2+2 IgG-scFv format as described in e.g. in Sambrook et al., Molecular Cloning: A Laboratory Manual, Cold Spring Harbor Laboratory Press, Cold Spring Harbor, New York, 2001. The resulting bispecific antibodies hence contained two anti-LTβR scFvs as well as the anti-LRRC15 antibody comprising two antigen binding domains i.e. the third and fourth antigen binding domains e.g. of the 1^{st} aspect (derived as described above in 1.1 and WO2022157094 A2) and were expressed in HEK293 cells using standard transient transfection procedures (cf. e.g. Sambrook et al., Molecular Cloning: A Laboratory Manual, Cold Spring Harbor Laboratory Press, Cold Spring Harbor, New York, 2001). Briefly, per well, 380 µl HEK293 cells of a solution containing 2×10⁶ cells/ ml in Freestyle F17 medium containing 2 mM Glutamax and 0.1% Pluronic, were seeded in 96-deep well plates. Cells were transfected using 293-fectin (Invitrogen, 12347-019) and 0,375 µg of each plasmid coding for the heavy and light chain of the antibody. Cells were incubated for 5 days at 37°C and 5% CO2 and supernatant harvested via centrifugation for 5 min at 600 rpm. Cell culture supernatants from transient transfections were applied to HT29-LRRC15 monoculture LTβR agonist assay (with the set-up as described in Example 4,1 below) to further characterize the abilities of the antibodies to mediate LRRC15-LTβR specific cross-linking and LTβR agonism *in vitro.* Among the resulting antibodies was TPP-23518 comprising TPP-34847. The antibody TPP-29168 comprising TPP-34999 was derived using a related approach aiming at human reactivity only.

### Example 1.2.4 Reduction of sequence-based immunogenicity

For the germlining and sequence optimization process of TPP-34847 i.e. the LTβR part of TPP-23518 and TPP-34999 i.e. the LTβR part of TPP-29168 the closest germline families for light and heavy chain were selected and scrutinized for potential CMC (Chemistry, Manufacturing and Controls) relevant residues. A lower number of germline deviations of an antibody in comparison to its closest human germline sequence, reduces - without affecting antigen binding - the risk of immunogenicity caused by those molecules (Hwang et al.; Methods, Volume 36, Issue 1, 2005, Pages 35-42, ISSN 1046-2023). Deviations from closest human germlines in complementarity-determining regions (CDRs) and framework regions (FW) of the scFv fragments and potential CMC relevant residues in CDR regions were backmutated by site directed mutagenesis following standard molecular protocols (cf. e.g. Sambrook et al., Molecular Cloning: A Laboratory Manual, Cold Spring Harbor Laboratory Press, Cold Spring Harbor, New York, 2001). The generated DNA constructs were expressed by transient transfection of mammalian cells and purified as described in 1.2.1. The resulting antibodies were then tested in human and mouse co-culture assays to test for LTβR agonism as described in Example 4.1 as well as unspecific binding and affinity towards human and mouse LTβR (via SPR assay with the setup as described in example 2). Single reversions were then combined on DNA level, expressed purified and again tested for unspecific binding and affinity towards human and mouse LTβR by SPR (with the set-up as described in example 2) as well as in human and mouse co-culture assays to test for LTβR agonism with the set-up as described in example 4. This led to the sequence optimized ("germlined") molecules TPP-34848 and TPP-35000. Details of the revisions are given in Table 2.

**Table 2 Alterations introduced during germlining**

| **TPP-ID** | **# of light and heavy chain deviations to closest human germline (excluding CDR-H3)** | **Revision** |
|---|---|---|
| TPP-34847 | 18 | E1Q, L5V, L11V, G16R, D31S, H32Y, Y33G, D35H, L116T |
| | | G166S, V170I, P174Y, N176Y, G192R, T194N, N195Q, A235D, W240V |
| TPP-34848 | 13 | L11V, G16R, D31S, H32Y, Y33G, D35H, L116T G166S, P174Y, N176Y, G192R, T194N, , W240V |
| TPP-34999 | 16 | E01Q, L05V, L11V, G16R, I34M, P52S, L115T |
| | | G165S,A166S, E170G, T171S, S175Y, D191R, R194Q, N237S, I240V |
| TPP-35000 | 6 | G16R, P52S |
| | | G165S, A166S, S175Y, D191R, |

### Example 1.2.5 Optimization of target dependent clustering

In order to identify variants that showed an optimized profile with regards to target dependent clustering, i.e. that activate/stimulate the LTβR signaling pathway to a considerably greater extend upon parallel binding to a targeting moiety such as LRRC15 - the CDRs of the LTβR part of TPP-23518 - i.e. the CDRs of the LTβR scFv of TPP-23518 which was TPP-34847- were subjected by a first round of single mutation gathering. For mutation gathering NNK (N = A or G or C or T, K = G or T) randomizations individual amino acid positions were generated by site directed mutagenesis using synthetic oligonucleotides including NNK for codon-diversification. Generation of recombination libraries was performed using sequential rounds of standard overlap extension PCR (Sambrook J, Russell DW (2001) Molecular cloning: a laboratory manual, 3rd edn. Sambrook).The resulting single NNK libraries were sequenced, and all identified single amino acid exchange variants were expressed by transient transfection of HEK293 cells similar to what was described in 1.2.1 and resulting expression supernatants were screened in functional assays (here LTβR agonist assay on human HT29-LRRC15 and HT29 cells as described in Example 4.1 with CXCL10 expression as readout). sNNK variants that demonstrated increased or equal stimulation of the LTβR signaling pathway in comparison to TPP-23518 in the HT29-LRRC15 cell co-culture assay, while demonstrating less stimulation of the LTβR signaling pathway in comparison to its parental antibody TPP-23518 in the HT29 cell monoculture assay - were selected as antibodies with improved profile. After a first screening round results for the top variants were confirmed in co-culture assays with HT29 and SAOS-2 cells as well as NIH-3T3 with SAOS-2 cells with the setup as described in Example 4.1 (cf. Table 3 below).

**Table 3 Comparison of different variants**

| | Mono-culture assay human | Co-culture assay mouse |
|---|---|---|
| Mutation given in comparison to VL (SEQ ID NO. 36) or VH (SEQ ID NO. 35) of TPP-34847 | HT-29-LRRC15: EC50 | NIH-3T3 + SAOS-2: EC50 |
| TPP-23518 | 1.9 to 4.4 | 4.54 to 14.8 |
| VH: M108T | 4.1 | 19.6 |
| VL: V29D | 1.3 | 63.7 |
| VH: R101W | 4 | 8.7 |
| VH: N57Q | 3.2 | 3.9 |
| VH: A61H | 0.6 | 2.1 |
| VH: K58L | 0.4 | 4.8 |
| VH: G55L | 0.2 | 0.6 |
| VL: V100N | 1 | 4.7 |

Hence the variants VH:M108T and VL:V29D showed reduced potency compared to TPP-23518 (the VL and VH regions respectively are also marked in Table 1 in SEQ ID NO. 7) and the variants VH:A61H, VH:K58L, VH:G55L and VL:V100N showed improved potency compared to TPP-23518 (the VL and VH regions respectively are also marked in Table 1 in SEQ ID NO. 7) in this setting.

Moreover, the affinity towards human and mouse LTβR was determined via SPR with the setting detailed in example 2 but employing human LTβR+ as an analyte (cf. Table 4 below)

**Table 4 Affinity towards human and mouse LTβR of selected single mutations variants as determined using SPR.**

| | SPR human LTβR | | | SPR mouse LTβR | | |
|---|---|---|---|---|---|---|
| Mutation given in comparison to VL (SEQ ID NO. 36) or VH (SEQ ID NO. 35) of TPP-34847 | ***kₐ* [1/Ms]** | ***k_{d}* [1/s]** | ***K*_{D} [M]** | ***kₐ* [1/Ms]** | ***k_{d}* [1/s]** | ***K*_{D} [M]** |
| TPP-23518 | 1.2E+05 | 2.3E-03 | 2.0E-08 | 1.2E+05 | 7.6E-04 | 6.6E-09 |
| VH: M108T | 1.2E+05 | 1.3E-03 | 1.1E-08 | 5.0E+05 | 9.8E-04 | 2.0E-09 |
| VL: V29D | 1.3E+05 | 3.9E-03 | 2.9E-08 | 1.6E+05 | 6.3E-03 | 4.0E-08 |
| VH: R101W | 6.8E+04 | 1.1E-03 | 1.6E-08 | 1.1E+05 | 1.6E-03 | 1.5E-08 |
| VH: N57Q | 1.1E+05 | 2.0E-03 | 1.8E-08 | 2.1E+05 | 2.8E-03 | 1.4E-08 |
| VH: A61H | 1.7E+05 | 3.9E-03 | 2.3E-08 | 1.9E+05 | 1.4E-03 | 7.2E-09 |
| VH: K58L | 5.5E+04 | 6.7E-04 | 1.2E-08 | 1.0E+05 | 3.9E-04 | 3.9E-09 |
| VH: G55L | 1.2E+05 | 1.7E-03 | 1.4E-08 | 2.1E+05 | 7.5E-04 | 3.7E-09 |
| VL: V100N | 1.7E+05 | 2.8E-03 | 1.7E-08 | 1.7E+05 | 9.5E-04 | 5.7E-09 |

In addition the temperature stability of the single exchange variants was assessed using Differential Scanning Calorimetry (DSC) using a VP-Capillary DSC system (Malvern Instruments GmbH, former MicroCal Inc.) with a cell volume of 0.137 mL. All samples were diluted in PBS buffer to a final concentration of 0.5 mg/mL, and a buffer control without protein was used as a reference. The samples were scanned from 20 °C to 95 °C at a scan rate of 120 °C/h. The data were analyzed using Origin 7.0 Data analysis (OriginLab Corp.). Furthermore, the generated single exchange variants were characterized with regard to unspecific binding, which should be as minimal as possible using three different ELISA assays (Table 5). For that purpose, baculovirus particles (#25690, Curia, USA), dsDNA (# D1626, Sigma Aldrich) and Insulin (# I9278, Sigma Aldrich) were incubated on ELISA plates (#781077, Geiner) by adding 20µL of the following coating solutions per 384well, sealed with foil and placed at 4°C over night: BVP and Insulin at 5µg/ml and dsDNA at 10µg/mL in sodium carbonate (pH 9.6) coating buffer (#121-125, Candor).The next day, unbound BVPs, dsDNA and Insulin were aspirated from the wells followed by 3 washes with 60µl wash buffer (PBS) per well. All remaining steps were performed at room temperature (22°C). For each incubation step plates were covered with foil or a lid. Next, 50µL of blocking buffer (PBS with 1% BSA (#A4503, Sigma Aldrich)) were added and incubated for 30 minutes prior to 3 washes each with 60 µL wash buffer. Then, 20 µl of 100nM primary antibody (i.e., test antibodies and the positive control antibody Lenzilumab (cf. Lenzilumab (CSF2) - Research Grade Biosimilar Catalog Number: orb1237584/ biorbyt) were added in PBS with 1% BSA (#A4503, Sigma Aldrich) to the wells in quadruplicates and incubated for 1 h followed by 6 washes with 60 µl of wash buffer. Then, 20µL of HRP goat anti-human IgG Fc specific detection antibody (#A0170, Sigma Aldrich) diluted 1/10000 in PBS with 1% BSA were added to the wells and incubated for 1 h followed by six washes as before. Finally, 20µL developing solution, prepared by adding 10 µl 5mM Amplex Red solution (#A1222, Invitrogen) and 1 µl 30% H2O2 to 10 ml PBS, were added to each well and incubated for 10 min in dark. Fluorescence was measured at excitation: 535nm and emission: 565nm on a Tecan infinite M1000 plate reader. Unspecific binding rank was determined for each reagent by first, determining the average background signal over the plate from 'sample buffer only' wells and next determining the signal to noise ratio (S/B) b dividing each test well (sample and control wells) by the average background. Next the median from the quadruplicates was calculated and the unspecific binding rank determined according to: (100/ (average S/B positive CTRL Lenzilumab)*(average S/B sample). A score below 10% was considered to reflect low polyreactivity.

**Table 5: Temperature stability and unspecific binding to baculoviral particle (BVP), Insulin and DNA**

| Mutation given in comparison to VL (SEQ ID NO. 36) or VH (SEQ ID NO. 35) of TPP-34847 | Temperature (Tm of Fab/Tm of scFv+CH2+CH3) | Unspecific Binding ELISA (rank) in % | | |
|---|---|---|---|---|
| | | BVP | Insulin | DNA |
| TPP-23518 | 77.67/70.69 | 4.4 | 4.8 | 3.9 |
| VH: M108T | 78/63 | 3.6 | 3.5 | 3.0 |
| VL: V29D | 78/65 | 3.5 | 3.6 | 3.1 |
| VH: R101W | 78/68 | 3.3 | 3.1 | 2.9 |
| VH: N57Q | 78/72 | 3.7 | 3.4 | 3.5 |
| VH: A61H | 78/69 | 4.1 | 3.7 | 3.9 |
| VH: K58L | 78/67 | 3.5 | 3.1 | 3.1 |
| VH: G55L | 78/63 | 3.6 | 3.4 | 3.5 |
| VL: V100N | 78/68 | 3.4 | 2.8 | 2.9 |

To generate further improved variants single mutations VH M108T VH I29D, VH: R101W VH:N57Q VH:A61H VH:K58L VH:G55L and VL:V100N were individually or together combined with the final germline TPP-29570, which comprises the scFV fragment TPP-34848 ("TPP-20767-gl46") by standard molecular biology protocols or DNA synthesis (Sambrook J, Russell DW (2001) Molecular cloning: a laboratory manual, 3rd edn. Sambrook). The combination variants were reformatted into hIgG1-LALA and tested in human and mouse co-culture assays to test for LTβR agonism with the set-up as described in Example 4. Selected results are shown in Table 6 below.

**Table 6 Comparison of different variants**

| Mutation given in comparison to VL (SEQ ID NO. 66) or VH (SEQ ID NO. 65) of TPP-34848 | Co-culture assay human SAOS-2 + HT-29 (CXCL10) EC50 higher (+) or lower (-) than TPP-23518 | Co-culture assay mouse NIH-3T3 + SAOS-2 (CXCL9) EC50 higher (+) or lower (-) than TPP-23518 |
|---|---|---|
| TPP-23518 | | |
| VH: (M108T) | - | - |
| VL: (I29D) | - | + |
| VL: (I29D); VH: (M108T) | - | + |
| VH: (R101W) | - | + |
| VH: (N57Q) | - | + |
| VH: (A61H) | - | + |
| VH: (K58L) | - | + |
| VH: (G55L) | - | + |
| VL: (V100N) | - | + |

Moreover the generated recombination variants were tested for affinity towards human and mouse LTβR using an SPR assay with the conditions described in example 2 but employing human LTβR as an analyte. Results are shown in the Table 7 below.

**Table 7**

| | SPR human LTBR | | | SPR mouse LTBR | | |
|---|---|---|---|---|---|---|
| Mutation given in comparison to VL (SEQ ID NO. 66) or VH (SEQ ID NO. 65) of TPP-34848 | ***kₐ* [1/Ms]** | ***k_{d}* [1/s]** | ***K*_{D} [M]** | ***kₐ* [1/Ms]** | ***k_{d}* [1/s]** | ***K*_{D} [M]** |
| Antibody with TPP-34848 | 3.91E+05 | 1.74E-03 | 4.46E-09 | 3.67E+05 | 5.94E-04 | 1.62E-09 |
| VH: (M108T) | 1.93E+05 | 2.51E-04 | 1.30E-09 | 2.62E+05 | 3.90E-04 | 1.49E-09 |
| VL: (I29D) | 1.50E+05 | 3.55E-04 | 2.36E-09 | 1.91E+05 | 5.27E-04 | 2.77E-09 |
| VL: (I29D); VH: (M108T) | 1.54E+05 | 3.39E-04 | 2.20E-09 | 1.96E+05 | 6.08E-04 | 3.10E-09 |
| VH: (R101W) | 1.12E+05 | 3.23E-04 | 2.89E-09 | 2.35E+05 | 2.85E-03 | 1.21E-08 |
| VH: (N57Q) | 1.89E+05 | 3.43E-04 | 1.81E-09 | 3.05E+05 | 1.43E-03 | 4.69E-09 |
| VH: (A61H) | 1.88E+05 | 4.27E-04 | 2.27E-09 | 2.65E+05 | 5.45E-04 | 2.06E-09 |
| VH: (K58L) | 1.83E+05 | 1.64E-04 | 8.97E-10 | 2.53E+05 | 2.42E-04 | 9.56E-10 |
| VH: (G55L) | 1.84E+05 | 3.01E-04 | 1.63E-09 | 2.52E+05 | 3.86E-04 | 1.53E-09 |
| VL: (V100N) | 1.83E+05 | 3.80E-04 | 2.08E-09 | 2.62E+05 | 4.18E-04 | 1.59E-09 |

The scFV TPP-34849 ("20767-gl76"/SEQ ID NO. 77) which in comparison to TPP-34848 carries the alteration VH M108T was chosen and integrated into the bispecific tetravalent antibodies TPP-32508, TPP-31142, TPP-32970 and TPP-32971. For illustration purposes only the mutation is depicted for TPP-32970 (TPP-32970 "HC" SEQ ID NO. 19) in Table 1 in underlined and bold.

### Example 2. Relationship of LTβR antibodies with LTβR ligands LIGHT and LTa1b2

It was investigated whether the BsAB competed with LTβR ligands LIGHT and LTa1b2 via surface plasmon resonance (SPR) assay. In the SPR assay changes in the optical phenomenon surface plasmon resonance (SPR) can be used as an indication of real-time interactions between biological molecules. The SPR experiments for competition analyses were performed using a Biacore T200 instrument (Cytiva) equipped with Series S Sensor Chips CMS (Cytiva). Binding assays were carried out at 25 °C with assay buffer HBS-EP+ (Cytiva) supplemented with 1 mg/ml BSA and 500 mM NaCl. Human LTβR was covalently immobilized to the chip surface via amine coupling chemistry according to the manufacturer's instructions using 1-ethyl-3-(3-dimethylaminopropyl) carbodiimide hydrochloride (EDC), N-hydroxysuccinimide (NHS) and ethanolamine HCl, pH 8.5 ("Amine Coupling Kit" BR-1000-50, Cytiva.). A tandem binding approach was performed by first injecting the BsAB and followed by human LTβR ligand LIGHT or human LTalb2 at 1.000 nM or vice versa i.e. first the ligands and then the BsAB. The sensor surface was regenerated with glycine pH 2.0 after each cycle Results of competition are given in Table 8.

**Table 8 Relationship of LTβR antibodies with LTβR ligands LIGHT and LTalb**

| TPP-No | Competition with LIGHT | Competition with LTalb2 | Competition with TPP-15972 |
|---|---|---|---|
| TPP-23518 | No | No | Yes |

Overall the results demonstrated that there is no competition of TPP-34847 - the LTβR scFV of TPP-23518 - with LTβR ligands LIGHT and LTa1b2 using the described SPR set-up. As the order in which the molecules were injected did not have any effect on the outcome both ligands can bind to the complex of BsAB and LTβR. The mouse cross-reactive surrogate BsAb TPP-15972 and the human BsAB TPP-23518 on the other hand seem to bind a close, adjacent or shared epitope, since no substantial additional response in the SPR assay after addition of the second antibody could be detected using covalently immobilized mouse LTβR and the same tandem approach.

### Example 3. Assessment of FcγR engagement and effector function (ADCC, ADCP)

### Example 3.1 Assessment of Fcγ receptor mediated binding

To determine the ability of the antibodies to bind to Fcy receptors, binding assays were conducted using surface plasmon resonance (SPR). Assays were performed on a Biacore T200 instrument (Cytiva) at 25°C using assay buffer HBS EP+, 500 mM NaCl. His-tagged Fcy receptors were captured via anti-his-tag IgGs ("His capture kit", Order No. 28995056, Cytiva) covalently amine coupled to a Series S CM5 sensor chip (Cytiva). The amine coupling was carried out according to the manufacturer's instructions using 1-ethyl - 3-(3-dimethylaminopropyl) carbodiimide hydrochloride (EDC), N-hydroxysuccinimide (NHS) and ethanolamine HCl, pH 8.5 ("Amine Coupling Kit" BR-1000-50, Cytiva.). Antibodies were used as analyte at 25 µM concentration in a single concentration binding assay, to determine their binding response behaviour. The sensor surface was regenerated with glycine pH 1.5 after each cycle. Obtained sensorgrams were double referenced (subtraction of reference flow cell signal and buffer injection) and were fitted to a 1:1 Langmuir binding model to derive kinetic/affinity data if applicable using the Biacore T200 Evaluation software. Results are shown in Table 9 with TPP-29446 and TPP-29445 acting as positive controls. This assay was also used to determine whether one of the alterations in IgG1 domains known to reduce Fc domain mediated receptor functions - such as LALA (L234A and L235A) and N297A (Strohl, W., 2009, Curr. Opin. Biotechnol. Vol. 20 (6): 685- 691); and E233P/L234V/L235A/DG236/D265G/A327Q/A330S ("HS") or N297A, N297Q, and N297G ("aglycosylation"; Bolt et al., Eur J Immunol. 1993 Feb;23(2):403-11) - would prove beneficial in a targeted antibody. Results are shown in Table 9.

**Table 9 SPR analysis of binding responses of antibodies generated as described in Example 1 for different Fcγ receptors. "Strongly reduced Binding response" is defined as a response of only a third or less based on RMax value.**

| **TPP** | **Format** | **FcγRI (CD64)** | **FcγRIIa (CD32a)** | **FcgRIIIb (CD16b)** | **FcγRIIb (CD32b)** | **FcγRIIIa V176** | **FcγRIIIa F176** |
|---|---|---|---|---|---|---|---|
| TPP-29445 | IgG1 | Binding | Binding | Binding | Binding | Binding | Binding |
| TPP-29446 | IgG1 | Binding | Binding | Binding | Binding | Binding | Binding |
| TPP-29416 | hIgG1-HS | None | None | None | None | None | None |
| TPP-29419 | hIgG1-HS | None | None | None | None | None | None |
| TPP-23518 | hIgG4-aglyco | Binding | Binding | Strongly reduced Binding | Binding | NA | NA |
| TPP-31142 | hIgG1-LALA | Strongly reduced Binding | Strongly reduced Binding | Strongly reduced Binding | Strongly reduced Binding | Strongly reduced Binding | Strongly reduced Binding |
| TPP-32970 | hIgG1-HS | None | None | None | None | None | None |
| TPP-32971 | hIgG1-HS | None | None | None | None | None | None |

Overall no significant binding of Fcγ receptors was observed. Antibodies comprising a LALA sequence show residual binding to FcγRs.

### Example 3.2 Assessment of effector function (ADCC, ADCP)

The effector functions were assessed using a reporter bioassays for Fey receptor engagement.

The day prior to the assay, target cells were seeded in triplicates, at a cell density of 18,750 cells/200µL medium per well, in a flat 96-well plate and incubated overnight. The next day, 190µL of medium was removed from the well and then added with 37.5µL of the following test antibodies (prepared as 2x concentrate). The plates were incubated for 30 minutes at 37°C. Meanwhile, effector cells from the ADCC Reporter Bioassay kit (Promega G7018) were thawed according to manufacturer's recommendation. The effector cells are Jurkat T cell line engineered with overexpression of Fcy receptor IIIa (with V158 polymorphism, which is a receptor with high affinity for Fc binding). The effector cells were added to the plate at a cell density of 75,000 cells/37.5µL assay buffer, and further incubated for 5 hours. Using this assay setup, the E:T ratio was 4 and the final assay volume was 75µL. At the end of the incubation period, 75µL of Bio-Gio^{™} Luciferase Assay Reagent was added to the plate, to detect the engagement of Fcy receptor IIIa, with the luminescence readout measured by SpectraMax iD5 Multi-Mode Microplate Readers. Results were analyzed using GraphPad Prism 9.

For the engagement of Fcγ receptor IIa (with H131 polymorphism, which is a receptor with high affinity for Fc binding), effector cells from FcyRIIa-H ADCP Reporter Bioassay were used (Promega G9901). The assay was set up the same way at E:T ratio 4, and the final luminescence readout was detected using the same plate reader. The extent of engagement was expressed as fold change of luminescence, calculated based on the background luminescence of a well containing only target cells and effector cells without any antibody. Results are given in Table 10.

**Table 10 ADCC and ADCP assay**

| | **ADCC (FcγRIIIa-V158)** | **ADCP Reporter (FcγRIIa-H131)** |
|---|---|---|
| | *Induction of more than 2 fold at a Log10 concentration (µg*/*mL) between 0.01 and 0.1* = + | *Induction of more than 2 fold at a Log10 concentration (µg*/*mL) between 0.01 and 0.1* = + |
| Positive control | + | + |
| TPP-31120 | - | - |
| TPP-31142 | - | - |
| TPP-32970 | - | - |
| TPP-32971 | - | - |
| TPP-32972 | - | - |

Table 10 shows the engagement of Fcy receptors IIIa and IIa, to confirm Fc-binding that would lead to ADCC and ADCP, respectively. The assays used engineered receptors with polymorphism of high affinity for Fc, hence the engagement of receptors (expressed as fold change compared to negative control) is well suited for magnifying detection of Fc-binding. In both cases, only the positive control antibody (HLA-ABC Monoclonal Antibody (W6/32) cf. ThermoFisher 14-9983-82 and US20130288387A1) showed binding to the Fcy receptors, whereas all the other test antibodies were silent, a trend that is consistent across all the concentrations tested.

### Example 4. Agonistic activity of bispecific antibodies in vitro

### Example 4.1 Chemokine induction

Stimulation of LTβR for example leads to more expression of the chemokines CXCL9 and CXCL10. Thus in order to assess the potential of the generated LTβR - LRRC15 tetravalent bispecific antibodies to stimulate the LTβR+ signaling pathway a novel co-culture assays employing a cell line expressing LTβR and a cell line expresses LRRC15 was established. In detail the following cell lines were used:
- human osteosarcoma cell line Saos-2 (Fogh J et al. (1977). Journal of the National Cancer Institute 58: 209-214) which endogenously expressed LRRC15,
- human HT29 cell line which expressed LTβR
- mouse NIH3T3 cell line, which expressed LTβR
- human HT29 cells that were stably transfected to express LRRC15 (HT29-LRRC15), and hence expressed both LTBR and LRRC15.

For the experiments the LTβR + and LRRC15 + cells were either seeded in Poly-D-Lysine 384 well white/clear flat bottom TC-treated microplate (BD, 356660) in co-culture at a 1:1 ratio with 5000 cells/well each (Saos-2: NIH3T3 in 25 µl medium containing 45% DMEM-Glu [ThermoFisher, 61965026], 45% McCoy's 5A medium [ThermoFisher, 16600082] and 10% FBS ([Invitrogen 10082-147]) or 1:5 ratio (Saos-2 : HT29 in 25 µl medium containing 90% McCoy's 5A medium ([ThermoFisher, 16600082] and 10% FBS [Invitrogen 10082-147]), respectively, or in mono-culture with 5000 cells /well (HT29-LRRC15 in McCoy's 5Amedium (ThermoFisher, 16600082) +10% FBS (Invitrogen 10082-147)). Incubation was carried out for 3h in the presence of 200 ng/ml human (Biolegend, 570208) or mouse IFNy (Biolegend, 575308), respectively. Thereafter the respective LTβR - LRRC15 bispecific and control antibodies were added at different concentrations in the same culture medium, without IFNy, resulting in a final concentration of 100 ng/ml IFNgamma. The tetravalent bispecific isotype was also termed "bivalent non-targeted anti-LTBR agonist", "non targeted" or "untargeted". The cells were incubated over night at 37°C and subsequently the culture supernatants applied to ELISA to determine the concentration of the LTβR responsive chemokines mouse CXCL9 or human CXCL10, respectively. ELISA sets for CXCL9 (Cat # DY492) and CXCL10 (Cat # DY266) were purchased from R&D Systems (Minneapolis, USA)and the assays were performed as recommended by the manufacturer. Moreover, each of the cell lines Saos-2, human HT29, mouse NIH3T3 and HT29-LRRC15 was cultured alone under the same conditions, treated with the LTβR - LRRC15 bispecific antibodies as well as the bispecific isotype control and culture supernatants were also applied to ELISA to determine the concentration of the LTβR responsive chemokines. Signals were used to calculate the concentrations of secreted CXCL9 in case of Saos-2: NIH3T3 co-culture and NIH3T3 monoculture and CXCL10 in case of Saos-2:HT29 coculture as well as HT29-LRRC15 and HT29 monoculture with Microsoft Excel. Induction of LTβR signaling was calculated as EC50 values using non-linear regression and three parameter agonist response calculation by GraphPad Prism (GraphPad Software, San Diego). Results are shown in Table 11a and Table 11b.

**Table 11a Results of in vitro experiments**

| Values denote multiple compared to value at "0" | | 4.4E-11 [M] | 3.0E-12 [M] | 0 |
|---|---|---|---|---|
| Cell type(s) | Antibody | | | |
| HT29(LTβR⁺) mono-culture CXLX10 | TPP-23518 | 1,5 | 0,9 | 1 |
| | TPP-20759 | 2,7 | 0,9 | 1 |
| | TPP-32970 | 0,7 | 0,8 | 1 |
| | TPP-32971 | 1,5 | 0,7 | 1 |
| | TPP-32972 | 1 | 0,9 | 1 |
| | TPP-31142 | 0,5 | 0,9 | 1 |
| | TPP-31120 | 1 | 0,7 | 1 |
| HT29(LTβR⁺)-LRRC15⁺ mono-culture (trans-assay) CXLX10 | TPP-23518 | 5,8 | 2,8 | 1 |
| | TPP-20759 | 1 | 1 | 1 |
| | TPP-32970 | 4,3 | 2,3 | 1 |
| | TPP-32971 | 4,1 | 2,1 | 1 |
| | TPP-32972 | 1 | 0,8 | 1 |
| | TPP-31142 | 4,7 | 2,5 | 1 |
| | TPP-31120 | 0,3 | 0,5 | 1 |
| Saos-2 (LRRC15⁺) HT29 (LTβR⁺) Co-culture CXLX10 | TPP-23518 | 3,8 | 0,9 | 1 |
| | TPP-20759 | 1,4 | 0,9 | 1 |
| | TPP-32970 | 4,3 | 1,6 | 1 |
| | TPP-32971 | 4,5 | 1,7 | 1 |
| | TPP-32972 | 1 | 0,8 | 1 |
| | TPP-31142 | 1,7 | 3,5 | 1 |
| | TPP-31120 | 1 | 0,8 | 1 |

**Table 11b Results of in vitro experiments**

| Values denote multiple compared to value at "0" | | 6,67E-10 [M] | 4.4E-11 [M] | 0 |
|---|---|---|---|---|
| Cell type(s) | Antibody | | | |
| NIH3T3 (LTβR⁺) mono-culture CXCL9 | TPP-23518 | 2 | 1,2 | 1 |
| | TPP-20759 | 2 | 1,1 | 1 |
| | TPP-32970 | 1,1 | 1 | 1 |
| | TPP-32971 | 1,6 | 1 | 1 |
| | TPP-32972 | 1,4 | 1 | 1 |
| | TPP-31142 | 1,3 | 1 | 1 |
| | TPP-31120 | 1,2 | 1 | 1 |
| Saos-2 (LRRC15⁺) NIH3T3(LTβR⁺) CXCL9 | TPP-23518 | 2,8 | 2,5 | 1 |
| | TPP-20759 | 1,7 | 1,1 | 1 |
| | TPP-32970 | 3,6 | 2,7 | 1 |
| | TPP-32971 | 3,2 | 2,5 | 1 |
| | TPP-32972 | 1,5 | 1 | 1 |
| | TPP-31142 | 3,2 | 2,1 | 1 |
| | TPP-31120 | 1 | 1 | 1 |

The results show that the tetravalent bispecific targeted LTβR antibodies comprising two LRRC15 binding moieties /antigen binding domains and two LTβR binding moieties /antigen binding domains described herein induced stronger stimulation of mouse CXCL9 or human CXCL10 chemokines and hence relevant stimulation of the LTβR signaling pathway in the co-cultures but not in the monocultures compared to the non-targeted isolated tetravalent control antibodies. If this effect was mediated by FcyR binding the antibodies containing only the LTBR binding moieties in combination with an isotype control should have demonstrated a similar effect, which was not the case. Moreover, the results show that different LRRC15 targeting moieties can be employed in combination with the LTβR scFV fragments described herein to form the isolated tetravalent bispecific targeted LTβR antibodies described herein.

**Table 12 Results of in vitro experiments (n.d. = not defined)**

| | Antibody | EC-50 [M] |
|---|---|---|
| HT29(LTβR⁺) mono-culture | TPP-23518 | 1,74E-09 |
| | TPP-20759 (control TPP-23518) | 3,87E-10 |
| | TPP-32970 | 9,62E-10 |
| | TPP-32971 | 6,50E-10 |
| | TPP-31142 | 5,26E-10 |
| | TPP-32972 (control TPP-32970 and TPP-32971) | 5,06E-10 |
| | TPP-31120 (control TPP-31142) | 1,64E-09 |
| NIH3T3 (LTβR⁺) mono-culture | TPP-23518 | 1.0E-09 |
| | TPP-20759 (control TPP-23518) | 1.1E-09 |
| | TPP-32970 | 8.3E-09 |
| | TPP-32971 | 1.6E-09 |
| | TPP-31142 | 1.8E-09 |
| | TPP-32972 (control TPP-32970 and TPP-32971) | 2.7E-09 |
| | TPP-31120 (control TPP-31142) | 3.5E-09 |
| HT29(LTβR⁺)-LRRC15⁺ mono-culture (trans-assay) | TPP-23518 | 5.0E-10 |
| | TPP-20759 (control TPP-23518) | 9.3E-10 |
| | TPP-32970 | 2.9E-11 |
| | TPP-32971 | 1.4E-11 |
| | TPP-31142 | 4.8E-11 |
| | TPP-32972 (control TPP-32970 and TPP-32971) | 1.6E-09 |
| | TPP-31120 (control TPP-31142) | 2.3E-09 |
| Saos-2 (LRRC15⁺): HT29 (LTβR**⁺**) Co-culture | TPP-23518 | n.d. |
| | TPP-20759 (control TPP-23518) | n.d. |
| | TPP-32970 | 5.8E-11 |
| | TPP-32971 | 5.3E-11 |
| | TPP-31142 | 7.9E-11 |
| | TPP-32972 (control TPP-32970 and TPP-32971) | 1.5E-09 |
| | TPP-31120 (control TPP-31142) | 2.4E-09 |
| Saos-2 (LRRC15⁺): NIH3T3(LTβR⁺) | TPP-23518 | 4.5E-11 |
| | TPP-20759 (control TPP-23518) | 1.5E-09 |
| | TPP-32970 | 2.1E-11 |
| | TPP-32971 | 4.4E-11 |
| | TPP-31142 | 6.4E-11 |
| | TPP-32972 (control TPP-32970 and TPP-32971) | 1.9E-09 |
| | TPP-31120 (control TPP-31142) | 4.5E-09 |

The results in Table 12 showed that relevant stimulation of mouse CXCL9 chemokines and hence relevant stimulation of the LTβR signaling pathway in setting where LRRC15⁺ and LTβR⁺ cells were present by the targeted tetravalent bispecific targeted LTβR antibodies comprising two LRRC15 binding moieties /antigen binding domains and two LTβR binding moieties /antigen binding domains described herein TPP-23518, TPP-32970, TPP-32971, TPP-31142) occurred at a lower EC-50 value as compared to non-targeted isolated tetravalent antibodies (TPP-20759, TPP-32972 and TPP-31120). In mono-cultures where no LRRC15⁺ expression was present this effect was not seen. Hence the targeted tetravalent bispecific targeted LTβR antibodies showed higher agonistic potency in settings where LRRC15⁺ and LTβR⁺ cells were present than the non-targeted control antibodies, but this was not the case if no LRRC15⁺ cells were present.

### Example 4.2 Induction of NF-κβ signaling

Stimulation of LTβR for example leads to more activity of nuclear factor κβ (NF-κβ). Activation/stimulation of NF-κβ through tumor necrosis factor receptors (TNFR) or the TNFR superfamily members such as LTβR occurs upon engagement with their respective ligands and "clustering"/"hyperclustering" of LTPR receptors. Hence also the induction of NF-κβ signaling was tested. Briefly, the used co-culture assay used NIH-3T3 NF-κβ cells (BPS, 79469), known to express LTBR, and SAOS-2 cells (ACC 243, DSMZ) known to express LRRC15. The NIH-3T3 had a NF-κβ luciferase reporter construct stably integrated into the genome. Following activation/stimulation endogenous NF-κβ transcription factors bind to the DNA response elements to induce transcription of the luciferase gene. For the experiment the LTβR+ and LRRC15+ cells were either seeded in Poly-D-Lysine 384 well white/clear flat bottom TC-treated microplate (BD, 356660) in co-culture at a 1:1 ratio with 5000 NIH-3T3 cells and 5000 SAOS-2 cells or in mono-culture with 5000 cells/well, respectively. Cells were seeded in 25 µl culture medium per well containing 45% Growth Medium 5A (BPS, BPS-79534), 45% McCoy's 5A medium (ThermoFisher, 16600082) and 10% FCS (ThermoFisher, 10082147). After 3h incubation at 37°C and 5% CO2, the respective antibodies were added in the same culture medium. The cells were incubated over night at 37°C, 5% CO2 and subsequently 25 µl/well of Bright Glo (Promega, E2620) was added and the plate incubated for 10 min protected from light. Readout was luminescence on a Tecan plate reader.

**Table 13 Results of in vitro experiments**

| Values denote multiple compared to value at "0" | | 4,E-10 [M] | 8,E-11 [M] | 0 |
|---|---|---|---|---|
| Cell type(s) | Antibody | | | |
| NIH3T3 (LTβR**⁺**) NF-κβ Luminescense | TPP-23518 (targeted) | 29 | 13 | 1 |
| | TPP-20759 (non-targeted) | 15 | 5 | 1 |
| | TPP-32970 (targeted) | 2 | 1 | 1 |
| | TPP-32971 (targeted) | 5 | 1 | 1 |
| | TPP-32972(non-targeted) | 3 | 1 | 1 |
| | TPP-31142(targeted) | 2 | 1 | 1 |
| | TPP-31120(non-targeted) | 2 | 1 | 1 |
| Saos-2 (LRRC15+): NIH3T3 (LTβR⁺)NF-κβ Luminescense | TPP-23518 (targeted) | 36 | 29 | 1 |
| | TPP-20759 (non-targeted) | 16 | 6 | 1 |
| | TPP-32970 (targeted) | 28 | 27 | 1 |
| | TPP-32971 (targeted) | 27 | 23 | 1 |
| | TPP-32972(non-targeted) | 3 | 2 | 1 |
| | TPP-31142(targeted) | 28 | 25 | 1 |
| | TPP-31120(non-targeted) | 2 | 1 | 1 |

The results in Table 13 show that the tetravalent bispecific targeted LTβR antibodies comprising two LRRC15 binding moieties /antigen binding domains and two LTβR binding moieties /antigen binding domains described herein (TPP-23518, TPP-32970, TPP-32971, TPP-31142) stimulated more CXCL9 expression and hence showed more agonistic potency of LTβR signaling pathway than the non-targeted isolated tetravalent antibodies lacking the two LRRC15 binding moieties (TPP-20759, TPP-32972 and TPP-31120) in the co-culture setting. Moreover, the results again show that different LRRC15 targeting moieties can be employed in combination with the LTβR scFV fragments described herein to form the isolated tetravalent bispecific targeted LTβR antibodies described herein. Moreover, the tetravalent bispecific targeted LTβR antibodies comprising two LRRC15 binding moieties /antigen binding domains and two LTβR binding moieties /antigen binding domains described herein should considerably less stimulation of mouse CXCL9 if used on monoculture.

**Table 14 Results of in vitro experiments**

| | Antibody | EC-50 [M] |
|---|---|---|
| NIH3T3 (LTβR**⁺**) NF-κβ | TPP-23518 | 1,82E-10 |
| | TPP-20759 | 6,60E-10 |
| | TPP-32970 | 7,52E-09 |
| | TPP-32971 | 1,69E-09 |
| | TPP-31142 | 2,45E-06 |
| | TPP-32972 | 3,98E-09 |
| | TPP-31120 | 2,08E-08 |
| Saos-2 (LRRC15+): NIH3T3 (LTβR⁺)NF-κβ | TPP-23518 | 3,91E-11 |
| | TPP-20759 | 7,43E-10 |
| | TPP-32970 | 1,84E-11 |
| | TPP-32971 | 4,16E-11 |
| | TPP-31142 | 2,58E-11 |
| | TPP-32972 | 4,44E-09 |
| | TPP-31120 | 3,29E-03 |

The results in Table 14 showed that relevant stimulation of mouse CXCL9 chemokines and hence relevant stimulation of the LTβR signaling pathway in setting where LRRC15⁺ and LTβR⁺ cells were present by the targeted tetravalent bispecific targeted LTβR antibodies comprising two LRRC15 binding moieties /antigen binding domains and two LTβR binding moieties /antigen binding domains described herein (TPP-23518, TPP-32970, TPP-32971, TPP-31142) occurred at a lower EC-50 value compared to non-targeted isolated tetravalent antibodies (TPP-20759, TPP-32972 and TPP-31120). In monocultures where no LRRC15⁺ expression was present this effect was not seen.

### Example 5. Comparative Example

In example 4 it was demonstrated that the isolated tetravalent bispecific antibodies comprising the LTβR binding domains generated herein stimulated the LTβR signaling pathway in the co-cultures very efficiently if used as targeted LTβR antibody but were only low level LTβR agonist if used as non-targeted LTβR antibody. In this example the isolated tetravalent bispecific variant of the bispecific antibody "COVA14146" of WO 2021/116337 A1 (TPP-32244) was used. It was generated as described in WO 2021/116337 A1. TPP-32244 was compared to an isolated tetravalent bispecific antibody comprising the LTβR binding domain TPP-34848 and the Mesothelin antibody used in the COVA14146 antibody of WO 2021/116337 resulting in TPP-32248. In other words both isolated tetravalent bispecific antibodies were targeted LTβR antibodies via the two Mesothelin antigen binding domains, but comprised different LTβR binding domains.

The co-culture assays used A549 Dual NF-kB-SEAP & IRF-Luc reporter cells (Invivogen, a549d-nfis) known to express LTBR and NCI-H226 cells (ATCC CRL-5826) known to express Mesothelin. Briefly, for the experiment the LTβR + and MSLN + cells were either seeded in Poly-D-Lysine 96 well white/clear flat bottom TC-treated microplate (ThermoFisher, 356651) in co-culture at a 1:1 ratio with 25000 cells of each cell line per well or in mono-culture with 25000 cells/well, respectively, in 50 µl culture medium containing 45% RPMI (ThermoFisher, A1049101) and 45% DMEM-Glu (100µg/ml PenStrep (ThermoFisher, 15140122), 100µg/ml Normocin (Invivogen, ant-nr-1), 10µg/ml Blasticidin (Invivogen, ant-bl-1) and 100µg/ml Zeozin (Invivogen, ant-zn-1) and 10% FCS (Invitrogen 10082-147). After 3h incubation at 37°C and 5% CO2, the respective LTβR - MSLN bispecific antibodies were added at different concentrations in 50 µl of the same culture medium. The cells were incubated over night at 37°C, 5% CO₂. For the readout 50 µl of freshly prepared QUANTI-Blue^{™} Solution (Invitrogen rep-qbs) was added per well of a transparent 96-well plate. 50 µl of supernatant of the culture plates was added to the QUANTI-Blue reagent and incubated at 37°C for 30 min. After that, measurement of optical density (OD) at 620 nm using a Tecan microplate reader was performed. Results are given below.

**Table 15 Comparative example**

| Values denote multiple compared to value at "0" | | 1,E-09 [M] | 1,E-10 [M] | 0 |
|---|---|---|---|---|
| Cell type(s) | Antibody | | | |
| A549-Dual cells (LTβR+) /NCI-H226 (MSLN+) cells | TPP-32248 (MSLN/LTβR): | 2,1 | 1,7 | 1 |
| | TPP-32247(Isotype/LTβR) | 1 | 1,1 | 1 |
| | TPP-32244 (MSLN/BHA10): | 2 | 2 | 1 |
| | TPP-32246 (isotype/BHA10) | 1,7 | 1,5 | 1 |
| Mono-culture A549-Dual cells (LTβR+) | TPP-32248 (MSLN/LTβR): | 1,7 | 1,3 | 1 |
| | TPP-32247(Isotype/LTβR) | 1 | 1 | 1 |
| | TPP-32244 (MSLN/BHA10): | 2 | 1,8 | 1 |
| | TPP-32246 (isotype/BHA10) | 1 | 1 | 1 |

Table 15 shows that in a setting where MSNL⁺ and LTβR⁺ cells were present the targeted tetravalent bispecific LTβR antibody comprising two MSNL binding moieties /antigen binding domains and two LTβR binding moieties /antigen binding domains described herein (TPP-32248) showed a response compared to baseline whereas the control non-targeted tetravalent bispecific targeted antibody comprising two LTβR binding moieties described herein (TPP-32247) showed almost no response compared to baseline. In contrast the targeted (TPP-32244) and the non-targeted (TPP-32246 ) antibodies comprising two scFv BHA10 (TPP-32246) LTβR domains i.e. the variant of the bispecific antibody "COVA14146" of WO 2021/116337 both showed response in the co-culture assay. In the mono-culture assay TPP-32248 was a low level agonist compared to TPP-32244 which also showed agonistic activity in the mono-culture assay. Hence the LTβR domains/ scFv fragments described herein showed decreased non-targeted agonism.

**Table 16**

| | | 1, EC-50 [M] |
|---|---|---|
| Cell type(s) | Antibody | |
| A549-Dual cells (LTβR+) /NCI-H226 (MSLN+) cells | TPP-32248 (MSLN/LTβR+): | 1,263E-10 |
| | TPP-32247(Isotype/LTβR+) | No activity |
| | TPP-32244 (MSLN/BHA10): | 1,258E-11 |
| | TPP-32246 (isotype/BHA10) | 5,915E-11 |
| Monoculture A549-Dual cells (LTβR+) | TPP-32248 (MSLN/LTβR+): | 2,114E-09 |
| | TPP-32247(Isotype/LTβR+) | No activity |
| | TPP-32244 (MSLN/BHA10): | 3,661E-11 |
| | TPP-32246 (isotype/BHA10) | 1,474E-10 |

| | | |
|---|---|---|
| Reporter assay (absorbance at 620 nm) | | |

Table 16 shows that in a setting where MSNL⁺ and LTβR⁺ cells were present the targeted tetravalent bispecific LTβR antibody comprising two MSNL binding moieties /antigen binding domains and two LTβR binding moieties /antigen binding domains described herein (TPP-32248 ) showed activity whereas the control non-targeted tetravalent bispecific targeted antibody comprising two LTβR binding moieties described herein (TPP-32247) did not show activity. In contrast the targeted (TPP-32244) and the untargeted (TPP-32246) antibodies comprising two scFv BHA10 (TPP-32246) i.e. the variant of the bispecific antibody "COVA14146" of WO 2021/116337 both showed activity in the co-culture assay. Moreover, TPP-32246 and TPP-32247 showed higher potency in the mono-culture assay. Hence, the tetravalent bispecific antibody described herein, exhibited decreased non-targeted agonism suggesting higher specificity and superiority necessary to minimize systemic toxicity in vivo while maintaining efficacy.

### Example 6. In vivo effect of LRRC15-LTβR bispecific antibodies

The evaluate the anti-tumor effect of LRRC15-LTβR bispecific antibodies the murine fibrosarcoma model Sa1/N (ATCC, CRL-2544) was chosen. For this purpose, female A/JOlaHsd mice (Envigo) were implanted subcutaneously with 1.0 × 10⁷ Sa1/N cancer cells (in 50% Matrigel / 50% Medium; 0.1 ml per animal s.c.). At a mean tumor size of 35-40 mm² animals were randomized into treatment and control groups (n=12 animals/group) and treatment started with respective antibodies at a dose of 2.5 mg/ml in PBS (application volume was 5 ml/kg), applied via intraperitoneal (i.p.) route on day 1 and day 4 each week for overall three weeks. A mouse IgG1 antibody TPP-19175 was included as IgG1 isotype control and a PBS vehicle control that did not contain any antibody was included as overall control.

The tumor size and body weight were determined three times a week. Changes in body weight were a measure of treatment-related toxicity (> 10% = critical, stop of treatment until recovery, > 20% = toxic, termination). The tumor area was detected by means of an electronic caliper gauge [length (mm) x width (mm)]. *In vivo* anti-tumor effect was determined as T/C ratio (Treatment/Control) calculated with relative tumor areas at study end by the formula [(tumor area of treatment group at day x) - (tumor area of treatment group at day before first treatment)] / [(tumor area control group at day x) - (tumor area of control group at day before first treatment)]. Treatments having a T/C below 0.5 were defined as active (effective). In more detail, a T/C of or below 0.3 indicates "good" anti-tumor activity, T/C between 0.3 and 0.7 indicates "moderate" anti-tumor activity and T/C of more than 0.7 is indicative for "weak/low" or "no" (1.0) anti-tumor activity. Statistical analysis was performed using Sigma Stat software. A one-way analysis of variance on ranks was performed and differences to the control were assessed by a pair-wise comparison procedure (Dunn's method) or multiple comparisons versus control group (Holm-Sidak method).

Animals were sacrificed individually when tumor area reached a size of 200 mm². The entire study was terminated at day 40 after tumor inoculation. For evaluation of anti-tumor effect treatment vs control ratio ("T/C") was calculated based on relative tumor areas at day 28 after tumor inoculation, when controls started to be sacrificed due to reaching maximum tumor size. At study end (day 40) we further assessed "response" according to RECIST and "survival" (surviving portion with tumor area < 200 mm², n of 12 = 100%)

**Table 17: Summarized is the anti-tumor efficacy of the use of the respective antibody presented as treatment vs control ratio (T/C) based on relative tumor area at day 28 after tumor cell inoculation (24 days after the first treatment) when controls started to be sacrificed due to reaching maximum tumor size. Further, "response" according to RECIST (response evaluation criteria in solid tumors), and "survival" were assessed at study end at day 40 after tumor cell inoculation (36 days after first treatment).**

| Treatment group | T/C _{rel TA} [day 28] | Response [day 40] | Survival [day 40] [n of 12 = 100%] |
|---|---|---|---|
| 1 - Control PBS ("vehicle") | 1,00 | 12 PD | 9% |
| 2 - IgG control TPP-19175 | 0,76 | 1 PR, 11 PD | 25% |
| 5 - TPP-32508 (targeted) | 0,11 ^{∗∗}#§ | 3 CR, 2 PR, 7 PD | 83% |
| 6 - TPP-32510 (non-targeted) | 0,75 | 12 PD | 25% |
| 7 - TPP-32511 (targeted) LC variant | 0,34 * | 1 PR, 11 PD | 75% |
| 8 - TPP-32506 (non-targeted) LC variant | 0,74 | 12 PD | 50% |
| 9 - TPP-29448 (targeted) | 0,16 **# | 2 CR, 1 PR, 9 PD | 75% |
| 10 - TPP-29449 (non-targeted) | 0,51 * | 2 PR, 10 PD | 50% |
| ** p<0.001 vs vehicle, * p<0.05 vs vehicle, # p<0.05 vs isotype, § p < 0.001 targeted vs non-targeted. | | | |
| Response (determined at day 40 after tumor inoculation) according to RECIST: *CR: complete response, no visible tumor; PR: partial response, >30% smaller than initial tumor size; SD: stable disease, <30% smaller and <20% bigger than initial tumor size; PD: progressive disease, >20% bigger than initial tumor size. | | | |
| Survival: animals were sacrificed individually when tumors reached a size of 200 mm²; study was terminated at day 40 after tumor inoculation; survival is defined % of animals per group still alive (tumor area < 200 mm²) at day 40 (n of 12 = 100%) T/C rel TA: treatment vs control (the term control is used synonymous with vehicle) ratio based on relative tumor area at day 28 after tumor cell inoculation | | | |

Overall, Table 17 showed good anti-tumor efficacy (T/C=0.11) of the use of the tetravalent bispecific targeted LTβR antibody TPP-32508 vs low activity (T/C=0.75) of the non-targeted LTβR antibody TPP-32510 variant was observed. Also, the use of the antibody TPP-32511 showed anti-tumor efficacy (T/C=0.34) whereas the control TPP-32506 showed low activity (T/C=0.74). Good anti-tumor efficacy of the use of the antibody (T/C=0.16) was also achieved by treatment with TPP-29448, while the respective non-targeted variant TPP-29449 showed moderate activity (T/C=0.51). All treatments were well tolerated without any detectable critical body weight change (loss) throughout the study.

In a subsequent *in vivo* study different doses of TPP-32508 or TPP-32510 were tested for anti-tumor efficacy of the use of an antibody vs controls (IgG1 isotype and PBS vehicle) in the SA1/N sarcoma model in A/JOlaHsd mice (methods as described above). TPP-32508 or TPP-32510 were dosed twice per week (day 1 and day 4) at 0.1 mg/kg, 0.5 mg/kg, 2.5 mg/kg and 10 mg/kg. Treatments were applied via intravenous (i.v.) route. The study was terminated at day 26 after tumor inoculation and anti-tumor efficacy was determined as "T/C" ratio and "response" (Table 18).

**Table 18**

| Treatment group | T/C _{rel TA} [day 26] | Response [day 26] |
|---|---|---|
| 1 - Control PBS ("vehicle") | 1,00 | 2 PR 10 PD |
| 2 - IgG control TPP-19175 | 0,69 | 5 PR 7 PD |
| 3 - TPP-32508 (targeted) 0.1 mg/kg | 0,78 | 3 PR 1 SD 8 PD |
| 4 - TPP-32510 (non-targeted) 0.1 mg/kg | 0,91 | 2 PR 1 SD 9 PD |
| 5 - TPP-32508 (targeted) 0.5 mg/kg | 0.21 | 4 PR 3 SD 6 PD |
| 6 - TPP-32510 (non-targeted) 0.5 mg/kg | 0.66 | 3 PR 9 PD |
| 7 - TPP-32508 (targeted) 2.5 mg/kg | -0.17 ^{∗} § | 6 PR 5 SD 1 PD |
| 8 - TPP-32510 (non-targeted) 2.5 mg/kg | 0.39 | 3 PR 2 SD 7 PD |
| 9 - TPP-32508 (targeted) 10 mg/kg | -0.07 ^{∗} § | 4 PR 4 SD 4 PD |
| 10 - TPP-32510 (non-targeted) 10 mg/kg | 0.56 | 1 PR 4 SD 7 PD |
| ^{∗} p<0.05 vs vehicle, # p<0.05 vs isotype, § p < 0.001 targeted vs non-targeted. | | |
| Response (determined at day 26 after tumor inoculation) according to RECIST: *CR: complete response, no visible tumor; PR: partial response, >30% smaller than initial tumor size; SD: stable disease, <30% smaller and <20% bigger than initial tumor size; PD: progressive disease, >20% bigger than initial tumor size | | |
| T/C rel TA: treatment vs control (the term control is used synonymous with vehicle) ratio based on relative tumor area at day 26 after tumor inoculation | | |

Dose-dependent anti-tumor efficacy of the use of the tetravalent bispecific targeted LTβR antibody TPP-32508 was observed (cf. Table 18) with a maximum achieved upon 2.5 mg/kg (T/C=-0.17) and 10 mg/kg (T/C=-0.07) vs moderate to weak activity in all dose groups of the non-targeted LTβR antibody TPP-32510 variant with overall good tolerability of the treatments. Despite high variability of SA1/N tumor growth in this study especially due to weak engraftment in the TPP-19175 group a statistically significant improvement of targeted vs non-targeted anti-tumor efficacy of the use the tetravalent bispecific targeted LTβR antibody TPP-32508 could be demonstrated in the highest dosing group.

To elucidate whether the anti-tumor activity of LRRC15-LTβR tetravalent bispecific antibodies detected in the SA1/N sarcoma model in immunocompetent mice was mediated via immune system activity , activity was evaluated in the SA1/N model when growing on immuno-compromised mice. For that purpose, female 6-8-week-old NSG (NOD/SCID/*IL2rg^{null}* mice (Charles River Deutschland)) were implanted subcutaneously with 5 × 10⁶ Sa1/N cancer cells. At a mean tumor size of 36 mm² animals were randomized into treatment and control groups (n=12 animals/group) and 24 hours later treatment started. TPP-32508 vs TPP-32510, TPP-29448 vs TPP- 29449, vs controls (IgG1 isotype and PBS vehicle) were dosed twice per week (day 1 and day 4) at 2.5 mg/kg intravenously (i.v.) (methods as described above). The study was terminated at day 21 after tumor inoculation, 17 days after treatment start. Overall, there was no detectable anti-tumor efficacy of the use of the tetravalent bispecific targeted LTβR antibodies TPP-32508 and TPP-29448 in immune compromised NSG mice. This indicated that anti-tumor efficacy of the use of an antibody observed in SA1/N model in immunocompetent mice effects was mediated by immune system activity i.e., LRRC15-targeted LTBR agonism induced immune cell dependent anti-tumor activity.

Moreover, qualitative assessment of marker genes e.g., those that could be used as pharmacodynamic biomarkers, was started in tumors derived from experiment in the SA1N tumor model summarized in Table 19. Six animals (each) from 3 different treatment groups, Isotype control TPP-19175, targeted TPP-32508, non-targeted TPP-32510, were harvested individually at study end or earlier when tumors reached maximal size before (between day 24 and 40 after inoculation). Per group six tumors were selected randomly for RNA extraction with the Qiagen RNeasy Mini Kit. RNA was converted to cDNA and only samples with a RIN (RNA integrity number) value above 9.5 were selected for library preparation with TruSeq Stranded mRNA HT kit. Samples were sequenced on a NextSeq 2000 single-end at 50bp and a depth of 50 million reads per sample. FASTQ files were aligned through STAR with the pseudo-autosomal regions masked, the corresponding annotation used was GENCODE M29, counted with RSEM and differential expression of the count matrix was performed with DESeq2.

**Table 19**

| Marker (z-score of log2tpm of more than 1 = +) | | | | | |
|---|---|---|---|---|---|
| | | CCL21 | CXCL13 | CCL 19 | CD8a |
| Antibody | Tumor | | | | |
| TPP-19175 | 1 | - | - | - | - |
| | 2 | - | - | - | - |
| | 3 | - | - | - | - |
| | 4 | - | - | - | - |
| | 5 | - | - | - | - |
| | 6 | - | - | - | - |
| TPP-32510 | 1 | - | - | - | - |
| | 2 | - | - | - | - |
| | 3 | - | - | - | - |
| | 4 | - | - | - | - |
| | 5 | - | - | - | - |
| | 6* | + | + | - | - |
| TPP-32508 | 1# | + | + | + | + |
| | 2 | + | + | + | + |
| | 3 | + | + | + | + |
| | 4 | + | + | + | + |
| | 5# | - | + | + | - |
| | 6 | - | + | + | + |
| partial responder # non responder | | | | | |

Overall, as shown in Table 19 there was as more pronounced induction of genes involved in LTβR signaling upon treatment with the LRRC15-targeted LTβR tetravalent bispecific antibody TPP-32508 compared to the IgG control mAb TPP-19175 and the non-targeted control TPP-32510 as indicated by upregulation of lymphocyte attracting chemokines expression that correlated with treatment response.

## Claims

1. Isolated tetravalent bispecific antibody comprising four antigen binding domains wherein a first and second antigen binding domain are binding to human LTβR and wherein a third antigen binding domain and a fourth antigen binding domain are binding to the same antigen other than LTβR wherein each of the first and second antigen binding domains are an scFV fragment comprising
a) a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849), or
b) a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848), or
c) a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847), or
d) a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999), or
e) a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000).

2. The isolated tetravalent bispecific antibody according to claim 1 wherein the isolated tetravalent bispecific antibody has at least one of the following properties
a) it is an agonistic LTβR antibody, or
b) is a more potent agonistic LTβR antibody if the antigen to which the third antigen binding domain and a fourth antigen binding domains are binding to is present
c) is a more potent agonistic LTβR antibody if the antigen to which the third antigen binding domain and a fourth antigen binding domains are binding to is present thus the isolated tetravalent bispecific antibody according to claim 1 stimulates the LTβR pathway more if it is a targeted antibody than if it is a non-targeted antibody.

3. The isolated tetravalent bispecific antibody according to claim 1 or claim 2 wherein each of the first and second antigen binding domains are identical scFV fragments
a) having at least 90 %, 95 %, 98 %, 99 % or 100 % sequence identity with SEQ ID NO. 77 (TPP-34849),
b) having at least 90 %, 95 %, 98 %, 99 % or 100 % sequence identity with SEQ ID NO. 67 (TPP-34848),
c) having at least 90 %, 95 %, 98 %, 99 % or 100 % sequence identity with SEQ ID NO. 37 (TPP-34847),
d) having at least 90 %, 95 %, 98 %, 99 % or 100 % sequence identity with SEQ ID NO. 47 (TPP-34999),
e) having at least 90 %, 95 %, 98 %, 99 % or 100 % sequence identity with SEQ ID NO. 57 (TPP-35000).

4. The isolated tetravalent bispecific antibody according to anyone of claims 1 to 3 wherein the third and fourth antigen binding domains are binding LRRC15 and comprise
a) a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) or
b) a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421).

5. The isolated tetravalent bispecific antibody according to anyone of claims 1 to 4 wherein the isolated tetravalent bispecific antibody comprises
a) a first and second antigen binding domains as scFV fragments according to claim 3 and
b) third and fourth antigen binding domains according to claim 4 and
c) an Fc domain of human IgG1 or IgG4 subclass comprising one or more amino acid substitutions that reduce the binding affinity of the antigen binding molecule to an FC receptor and/or effector function.

6. The isolated tetravalent bispecific antibody according to anyone of claims 1 to 5 which in the presence of LRRC15 is an agonists of LTPR signaling and comprises
a) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-32508, TPP-31142, TPP-32970) or
b) a first and second antigen binding domain comprising a heavy chain variable region comprising a heavy chain complementary determining region (HCDR1) comprising the amino acid sequence of SEQ ID NO. 69 a complementary determining region (HCDR2) comprising the amino acid sequence of SEQ ID NO. 70 and a complementary determining region (HCDR3) comprising the amino acid sequence of SEQ ID NO. 71 and a light chain variable region comprising a light chain complementarity determining region (LCDR1) comprising the amino acid sequence of SEQ ID NO. 72, a light chain complementarity determining region (LCDR2) comprising the amino acid sequence of SEQ ID NO. 73 and a light chain complementarity determining region (LCDR3) comprising the amino acid sequence of SEQ ID NO. 74 (TPP-34849) and a third and fourth antigen binding domain comprising a heavy chain variable region comprising a heavy chain complementary determining region (HCDR1) comprising the amino acid sequence of SEQ ID NO. 140 a complementary determining region (HCDR2) comprising the amino acid sequence of SEQ ID NO. 141 and a complementary determining region (HCDR3) comprising the amino acid sequence of SEQ ID NO. 142 and a light chain variable region comprising a light chain complementarity determining region (LCDR1) comprising the amino acid sequence of SEQ ID NO. 144, a light chain complementarity determining region (LCDR2) comprising the amino acid sequence of SEQ ID NO. 145 and a light chain complementarity determining region (LCDR3) comprising the amino acid sequence of SEQ ID NO. 146 (TPP-17421) (TPP-32971) or
c) a first and second antigen binding domain comprising a heavy chain variable region comprising a heavy chain complementary determining region (HCDR1) comprising the amino acid sequence of SEQ ID NO. 29 a complementary determining region (HCDR2) comprising the amino acid sequence of SEQ ID NO. 30 and a complementary determining region (HCDR3) comprising the amino acid sequence of SEQ ID NO. 31 and a light chain variable region comprising a light chain complementarity determining region (LCDR1) comprising the amino acid sequence of SEQ ID NO. 32, a light chain complementarity determining region (LCDR2) comprising the amino acid sequence of SEQ ID NO. 33 and a light chain complementarity determining region (LCDR3) comprising the amino acid sequence of SEQ ID NO 34 (TPP-34847) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-23518) or
d) a first and second antigen binding domain comprising a heavy chain variable region comprising a heavy chain complementary determining region (HCDR1) comprising the amino acid sequence of SEQ ID NO. 59 a complementary determining region (HCDR2) comprising the amino acid sequence of SEQ ID NO. 60 and a complementary determining region (HCDR3) comprising the amino acid sequence of SEQ ID NO. 61 and a light chain variable region comprising a light chain complementarity determining region (LCDR1) comprising the amino acid sequence of SEQ ID NO. 62, a light chain complementarity determining region (LCDR2) comprising the amino acid sequence of SEQ ID NO. 63 and a light chain complementarity determining region (LCDR3) comprising the amino acid sequence of SEQ ID NO. 64 (TPP-34848) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29570) or
e) a first and second antigen binding domain comprising a heavy chain variable region comprising a heavy chain complementary determining region (HCDR1) comprising the amino acid sequence of SEQ ID NO. 39 a complementary determining region (HCDR2) comprising the amino acid sequence of SEQ ID NO. 40 and a complementary determining region (HCDR3) comprising the amino acid sequence of SEQ ID NO. 41 and a light chain variable region comprising a light chain complementarity determining region (LCDR1) comprising the amino acid sequence of SEQ ID NO. 42, a light chain complementarity determining region (LCDR2) comprising the amino acid sequence of SEQ ID NO. 43 and a light chain complementarity determining region (LCDR3) comprising the amino acid sequence of SEQ ID NO 44 (TPP-34999) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29168)
f) a first and second antigen binding domain comprising a heavy chain variable region comprising a heavy chain complementary determining region (HCDR1) comprising the amino acid sequence of SEQ ID NO. 49 a complementary determining region (HCDR2) comprising the amino acid sequence of SEQ ID NO. 50 and a complementary determining region (HCDR3) comprising the amino acid sequence of SEQ ID NO. 51 and a light chain variable region comprising a light chain complementarity determining region (LCDR1) comprising the amino acid sequence of SEQ ID NO. 52, a light chain complementarity determining region (LCDR2) comprising the amino acid sequence of SEQ ID NO. 53 and a light chain complementarity determining region (LCDR3) comprising the amino acid sequence of SEQ ID NO 54 (TPP-35000) and a third and fourth antigen binding domain comprising a heavy chain variable region comprising a heavy chain complementary determining region (HCDR1) comprising the amino acid sequence of SEQ ID NO. 122 a complementary determining region (HCDR2) comprising the amino acid sequence of SEQ ID NO. 123 and a complementary determining region (HCDR3) comprising the amino acid sequence of SEQ ID NO. 124 and a light chain variable region comprising a light chain complementarity determining region (LCDR1) comprising the amino acid sequence of SEQ ID NO. 126, a light chain complementarity determining region (LCDR2) comprising the amino acid sequence of SEQ ID NO. 127 and a light chain complementarity determining region (LCDR3) comprising the amino acid sequence of SEQ ID NO. 128 (TPP-17078) (TPP-29701).

7. The isolated tetravalent bispecific antibody according to anyone of claims 1 to 6 comprising
a) a heavy chain region having at least 90 %, 95 %, 98 %, 99 % or 100 % sequence identity with SEQ ID NO. 19 and a light chain region having at least 90 %, 95 %, 98 %, 99 % or 100 % sequence identity with SEQ ID NO. 20 (TPP-32970) or
b) a heavy chain region having at least 90 %, 95 %, 98 %, 99 % or 100 % sequence identity with SEQ ID NO. 15 and a light chain region having at least 90 %, 95 %, 98 %, 99 % or 100 % sequence identity with SEQ ID NO. 16 (TPP-31142) or
c) a heavy chain region having at least 90 %, 95 %, 98 %, 99 % or 100 % sequence identity with SEQ ID NO. 21 and a light chain region having at least 90 %, 95 %, 98 %, 99 % or 100 % sequence identity with SEQ ID NO. 22 (TPP-32971).

8. A first antigen binding domain capable of binding to LTβR and a second antigen binding domain capable of binding to LTβR wherein each of the first and second antigen binding domain comprises at least one, two, three, four, five and preferably six CDR sequences, wherein each of said CDR sequences has at least 90 %, 95 %, 98 %, 99 % or 100 % sequence identity with at least one of
a) SEQ ID NO. 69, SEQ ID NO. 70, SEQ ID NO. 71, SEQ ID NO. 72, SEQ ID NO. 73 and SEQ ID NO. 74 (TPP-34849), or
b) SEQ ID NO. 59, SEQ ID NO. 60, SEQ ID NO. 61, SEQ ID NO. 62, SEQ ID NO. 63, and SEQ ID NO. 64 (TPP-34848), or
c) SEQ ID NO. 29, SEQ ID NO. 30, SEQ ID NO. 31, SEQ ID NO. 32, SEQ ID NO. 33 and SEQ ID NO 34 (TPP-34847), or
d) SEQ ID NO. 39, SEQ ID NO. 40, SEQ ID NO. 41, SEQ ID NO. 42, SEQ ID NO. 43 and SEQ ID NO. 40 (TPP-34999), or
e) SEQ ID NO. 49, SEQ ID NO. 50, SEQ ID NO. 51, SEQ ID NO. 52,, SEQ ID NO. 53 and SEQ ID NO. 54 (TPP-35000).

9. A pharmaceutical composition comprising an isolated tetravalent bispecific antibody according to claims 1-7 or a first antigen binding domain capable of binding to LTβR and a second antigen binding domain capable of binding to LTβR according to claim 8.

10. An isolated tetravalent bispecific antibody according to claims 1-7 or a first antigen binding domain capable of binding to LTβR and a second antigen binding domain capable of binding to LTβR according to claim 8 for use as a medicament.

11. An isolated tetravalent bispecific antibody according to claims 1-7 or a first antigen binding domain capable of binding to LTβR and a second antigen binding domain capable of binding to LTβR according to claim 8 for use in the treatment of cancer.

12. An isolated tetravalent bispecific antibody according to claims 1-7 or a first antigen binding domain capable of binding to LTβR and a second antigen binding domain capable of binding to LTβR according to claim 8 for use according to claim 11, wherein the cancer is selected from the list consisting of lung cancer, non-small cell lung carcinoma, head and neck cancer, head and neck squamous cell carcinoma, sarcoma, glioblastoma, melanoma, breast cancer, HER2 negative breast cancer, HER2 positive breast cancer, triple negative breast cancer, HR+ breast cancer, pancreatic cancer, pancreatic ductal adenocarcinoma or colorectal cancer.

13. An isolated tetravalent bispecific antibody according to claims 1-7 or a first antigen binding domain capable of binding to LTβR and a second antigen binding domain capable of binding to LTβR according to claim 8 for use as a diagnostic agent.

14. A polynucleotide encoding an isolated tetravalent bispecific antibody according to claims 1-7 or a first antigen binding domain capable of binding to LTβR and a second antigen binding domain capable of binding to LTβR according to claim 8.

15. A kit of parts comprising an isolated tetravalent bispecific antibody according to claims 1-7 or a first antigen binding domain capable of binding to LTβR and a second antigen binding domain capable of binding to LTβR according to claim 8 thereof with instructions for use.
